# EUROPEAN PATENT SPECIFICATION

(11) **EP 4 077 310 B1**
(45) Date of publication and mention of the grant of the patent: **01.05.2024**
(21) Application number: 20838436.2
(22) Date of filing: 18.12.2020
(51) Int. Cl.: C07D 403/04, C07D 403/06, A61P 25/28, A61K 31/435

(54) **SIGMA-1 RECEPTOR LIGANDS AND USES THEREOF**
SIGMA-1-REZEPTORLIGANDEN UND VERWENDUNGEN DAVON
LIGANDS DU RÉCEPTEUR SIGMA-1 ET LEURS UTILISATIONS

(30) Priority: 19.12.2019 EP 19306705
(43) Date of publication of application: 26.10.2022
(73) Proprietor: Université de Strasbourg, 67000 Strasbourg (FR); Centre national de la recherche scientifique, 75016 Paris (FR); Institut National de la Santé et de la Recherche Médicale, 75013 Paris (FR); UNIVERSITE DE MONTPELLIER, 34090 Montpellier (FR); Ecole Pratique des Hautes Etudes, 75014 Paris (FR)
(72) Inventor: BOURGUIGNON, Jean-Jacques, 67400 Illkirch (FR); SCHMITT, Martine, 67000 Strasbourg (FR); BRICARD, Jacques, 67400 Illkirch-Graffenstaden (FR); MAURICE, Tanguy, 34980 Saint Gely-du-Fesc (FR)
(74) Representative: Cabinet Becker et Associés
(86) International application number: PCT/EP2020/086996
(87) International publication number: WO 2021/123145

(56) References cited:
- WO-A1-2017/222952
- WO-A1-2019/068771
- US-A1- 2012 010 222
- US-A1- 2015 119 412

## Description

### Field of the Invention

The present invention relates to the field of medicine. More specifically, the present invention relates to compounds that are sigma-1 receptor agonists and their use for the treatment of central nervous system disorders, including cognitive or neurodegenerative disorders, such as Alzheimer's disease, Parkinson's disease, Huntington's disease, Amyotrophic lateral sclerosis, and multiple sclerosis.

Any references to methods of treatment refer to the compounds, pharmaceutical compositions and medicaments of the present invention for use in a method for treatment of the human (or animal) body by therapy (or for diagnosis).

### Background of the Invention

Two sigma receptor subtypes have been identified based on their pharmacological profile.

The sigma-1 receptor is a membrane-associated protein, found throughout the body and widely expressed in the central nervous system, neurons, astrocytes, oligodendrocytes and microglia. The sigma-1 receptor is a single polypeptide transmembrane protein comprising 223 amino acids. As a single 25 kD polypeptide and chaperone protein, it is highly expressed at the mitochondria-associated endoplasmic reticulum (ER) membranes and plasma membranes. Among its identified partner proteins are the glucose-related protein 78/binding immunoglobulin protein (BiP) and the inositol-1,4,5 trisphosphate (IP3) receptor. The sigma-1 receptor is a ligand-operated chaperone protein, that binds and is activated/inactivated by diverse classes of pharmacological compounds, including for instance the dextrogyre isomers of bezomorphans, such as (+)-pentazocine and (+)-SKF-10,047. Although certain endogenous ligands have been identified interacting with the sigma-1 receptor, such as neuro(active)steroids, neuropeptides, choline or trace amines, the existence of a high-affinity endogenous sigma-1 receptor ligand is still unclear. Activation of the sigma-1 receptor by agonist has several cellular consequences but mainly results in an amplification of calcium exchanges between the ER and mitochondria, by interacting with the IP3 receptor on ER membranes, and in the induction of ER stress pathways, by interacting with ER stress sensors such as BiP or IRE-1.

Sigma-1 receptor agonists have been reported as having antidepressant effects in several animal models. For example, the selective σ₁ receptor agonists (+)-pentazocine, (+)-SKF-10,047, igmesine, OPC14523, DTG or SA4503 reduce freezing in the conditioned fear stress test or immobility time in the forced swim test or are active in the tail suspension test (Matsuno et al., Eur J Pharmacol 312:267-71, 1996; Tottori et al. Neuropharmacology 2001 41:976-88; Urani et al., J Pharmacol Exp Ther 298:1269-79, 2001).

It has also been reported to play a role in cell survival (Wang et al., Exp Cell Res 312:1439-46, 2006; Hayashi & Su, Cell 131:596-610, 2007; Jiang et al., Invest Ophthalmol Vis Sci 47:5576-82, 2006). Sigma-1 receptor ligands have been reported to be neuroprotective. The sigma-1 receptor ligand opipramol was reported as protected against ischemia in gerbils. In addition, other sigma ligands, including BMY-14802, caramiphen and haloperidol, exhibited properties in *in vivo* models that are consistent with protective effects (Pontecorvo et al., Brain Res Bull 26:461-5, 1991). Several sigma ligands were reported to inhibit ischemia-induced glutamate release from hippocampal slice preparations *in vitro* (Lobner et al., Neurosci Lett 1 17:169-74, 1990). It has also been reported that the sigma-1 receptor agonist (+)-pentazocine can protect the retinal cells against stress (Dun et al., Invest Ophthalmol Vis Sci 48:4785-94, 2007; Smith et al., Invest Ophthalmol Vis Sci 49:4154-61, 2008).

Particular note is made of the utility of a sigma-l receptor agonist with low sigma-2 receptor affinity in treating ischemic brain/neuronal injury such as from focal ischemia. Sigma-l receptor agonists are also believed useful in improving cognitive impairment such as exhibited with impaired neurotransmitter function (*e.g.,* acetylcholine) as well as age associated cognitive impairment, and anxiety associated impairment (including pregnancy stress resulting in learning deficits of offspring).

Agonists of the sigma-1 receptor are effective anti-amnesic compounds. This has been demonstrated in a number of pharmacological and pathological models of learning and memory impairment in rodents. In particular, sigma-1 receptor agonists are routinely validated *in vivo* against scopolamine-induced learning deficits, a model of muscarinic acetylcholine receptor (mAChR) blockade. For instance, the sigma-1 receptor agonists LS-1-137 (Malik et al., Br J Pharmacol 172:2519-31, 2015), the sigma-1 positive modulators E1R (Zvejniece et al., Br J Pharmacol 171:761-71, 2014) or OZP002 (Maurice et al., Pharmacol Res 144:315-30, 2019) or the mixed mAChR/σ₁ agonists ANAVEX1-41 or ANAVEX2-73 (blarcamesine), two diphenyl-3-furanmethanamine derivatives (Espallergues et al., Br J Pharmacol 152:267-79, 2017; Villard et al. Neuropsychopharmacology 34:1552-66, 2009), have recently been characterized as anti-amnesic drugs against scopolamine-induced learning impairments. The efficacy of sigma-1 receptor agonists as symptomatic drugs in cognition has been described not only in cholinergic amnesia models (e.g., scopolamine, mecamylamine, *p*-chloroamphetamine, forebrain lesions) but also in glutamatergic models of learning deficit. Learning impairment induced by the non-competitive NMDA receptor antagonist dizocilpine (MK-081) has been used to demonstrate that the positive modulation exerted by the sigma-1 receptor on NMDA neurotransmission, suggested *in vitro* or *in vivo* using extracellular recordings of the NMDA-induced firing of pyramidal neurons in the CA3 hippocampal area has behavioral consequences. The efficacy of sigma-1 receptor agonists in alleviating dizocilpine-induced learning impairment also points to the potential utility of these drugs in treating schizophrenia-related cognitive deficits, in particular since hypoglutamatergy models have been considered as highly pertinent for mimicking the negative symptoms of schizophrenia (Meltzer et al., Int J Neuropsychopharmacol 16:2181-94, 2013). Interestingly, sigma-1 receptor ligands tested in both the scopolamine and dizocilpine models showed a similar active dose-range *in vivo.* Activation of the sigma-1 receptor therefore appeared to similarly modulate the activity of the two neurotransmission systems involved in memory processes in the limbic and cortical structures, namely cholinergic and particularly glutamatergic systems. The sigma-1 receptor agonists enhance NMDA-induced firing in the hippocampus at very low doses. (+)-SKF-10,047 (also known as Alazocine), PRE084, and (+)-pentazocine increased the expression of the NR2A and NR2B subunits of NMDA receptor, as well as PSD95 (also known as SAP-90), in the rat hippocampus. The sigma-1 receptor agonist treatment leads to increased interaction between NR2 subunits and sigma-1 receptors and promotes trafficking of NMDA receptors to the cell surface. The sigma-1 receptor interacts with NMDA receptors through the regulation of a small conductance Ca²⁺-activated K⁺ current (SK channels). At the behavioral level, young male sigma-1 KO mice, at 2 months of age, showed signs of anxiety in procedures including the open-field, passive avoidance and elevated plus-maze, and an enhanced response to stress in the forced swim test. In male animals, the σ₁ receptor ablation therefore increased stress and anxiety responses but memory responses were unchanged. However, female sigma-1 KO mice showed memory alterations in spontaneous alternation and water-maze learning paradigms, and this phenotype increased with age. Of note, both 2- and 14-month old female sigma-1 KO mice showed decreased plasma levels of 17β-estradiol and a supplementation treatment with the hormone reversed the memory deficits in young and aged mice (Chevallier et al., J Psychopharmacol 25:960-75, 2011). This suggested that sigma-1 receptor ablation has a developmental impact on the steroidal tonus.

The sigma-1 receptor agonists are therefore promising symptomatic drugs in rodent models of cognitive alterations related to pathological ageing and neurodegenerative diseases. First, igmesine and PRE-084, in the low mg/kg dose-range, improved learning ability in the senescence-accelerated mouse SAMP/8 (Maurice et al. Brain Res 733:219-30, 1996). Second, these compounds also alleviated the memory deficits induced by amyloid toxicity in pharmacological models of Alzheimer's disease (AD). (+)-pentazocine, PRE-084, cutamesine, dimemorphan, ANAVEX1-41, blarcamesine, OZP002 and the sigma-1 receptor binding neuroactive steroids attenuated the learning deficits in mice that received a direct intracerebroventricular injection of oligomerized Aβ₂₅₋₃₅ peptide, which produces neurotoxicity closely related to AD pathology (Meunier et al., Br J Pharmacol. 149: 998-1012, 2006; Villard et al., J Psychopharmacol 25: 1101-17, 2011; Maurice et al., Pharmacol Res 144:315-30, 2019). All sigma-1 receptor agonists or positive modulators alleviated the Aβ₂₅₋₃₅-induced learning impairments in spatial or nonspatial tasks involving short-term as well as long-term memory. These effects were blocked by BD1047, haloperidol, BMY-14,802, and progesterone, all putative sigma-1 receptor antagonists. Of note, whereas they blocked sigma-1 receptor agonist effects, the antagonists alone did not alter behavior (positively or negatively) in these models. The sigma-1 receptor agonists are thus promising agents to treat AD symptoms, with active doses similar to or lower than the reference drugs donepezil, rivastigmine, galantamine, and memantine (Meunier et al., Br J Pharmacol 149: 998-1012, 2006).

Finally, blarcamesine, which is a mixed muscarinic and sigma-1 drug, has successfully completed a phase 2 clinical trial in AD. The drug stabilized ADAS-ADL scores and limited MMSE score decrease in patients after a 3-year treatment in patients showing the highest drug bioavailability (Hample et al., CTAD Abstracts, 2018).

WO2019/068771 discloses compounds as sigma-1 receptor ligands.

Thus, there is nowadays a strong need for the development of new sigma-1 receptor agonists for the treatment of cognitive disorders related to psychiatric pathologies, neurodegenerative diseases, such as Alzheimer, Huntington and Parkinson diseases, amyotrophic lateral sclerosis (ALS) or multiple sclerosis, and genetic diseases associated with MAM (Mitochondria Associated Membranes) dysfunctions. The present invention seeks to meet this and other needs. Compounds according to the invention have been found to have affinity for sigma-1 receptors.

### Summary of the invention

In this respect, the present invention relates to a compound having the following formula (I): 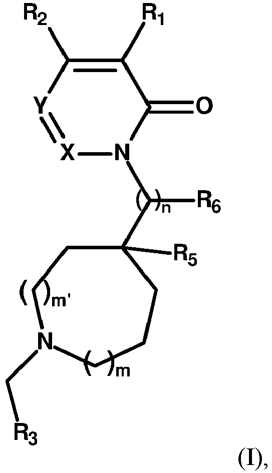 wherein:
R₁ and R₂ represent independently:
   - H;
   - an aryl(C₁-C₆)alkyl group;
   - an aryl group;
   - a cycloalkyl group;
   - a heterocyclic group; or
   - SR, R being alkyl, aryl or aralkyl;
   wherein one of R₁ and R₂ is H, and the other one of R₁ and R₂ is different from H;
X and Y are either, respectively:
   - CH and N, or
   - N and CR₄, or
   - CH and CR₄,
   wherein R₄ represents H or a (C₁-C₄)alkyl;
n is 0, 1, or 2; m is 0 or 1; m' is 0 or 1;
R₃ represents:
   - a radical selected from the group consisting of H, (C₁-C₆)alkyl, (C₂-C₆)alkenyl, aryl, heteroaryl, cycloalkyl, heterocycloalkyl, (C₁-C₆)alkoxy, and aryl(C₁-C₆)alkyl, said radical being optionally substituted by at least one group chosen among (C₁-C₆)alkyl, OH, halogen, aryl, heteroaryl, cycloalkyl, heterocycloalkyl, (C₁-C₆)alkoxy, - C(O)R, -CHOHR, C(O)₂R, C(O)NRR', -CONHOR, -CONHSO₂R, -NRR', - N(R)C(O)R', -N(R)NR'R", -N(R)C(O)₂R', -N(R)C(O)NR'R", -N(R)S(O)₂R', -SR, - S(O)R, -S(O)₂R, -S(O)NRR', and -S(O)₂NRR'; R, R', and R" being independently H, (C₁-C₆)alkyl, cycloalkyl, aryl, heterocycloalkyl, heteroaryl, (C₁-C₆)alkylcycloalkyl, (C₁-C₆)alkylaryl, (C₁-C₆)alkylheterocycloalkyl), or (C₁-C₆)alkylheteroaryl; or
   - a radical selected from the group consisting of OH, -C(O)R, -CHOHR, C(O)₂R, C(O)NRR', -CONHOR, -CONHSO₂R, -NRR', -N(R)C(O)R', -N(R)NR'R", - N(R)C(O)₂R', -N(R)C(O)NR'R", -N(R)S(O)₂R', -SR, -S(O)R, -S(O)₂R, -S(O)NRR', and -S(O)₂NRR'; R, R', and R" being independently H, (C₁-C₆)alkyl, cycloalkyl, aryl, heterocycloalkyl, heteroaryl, (C₁-C₆)alkylcycloalkyl, (C₁-C₆)alkylaryl, (C₁-C₆)alkylheterocycloalkyl), or (C₁-C₆)alkylheteroaryl;
R₅ represents H or OH; and
each of R₆ represents independently H or a (C₁-C₆)alkyl group;
a stereoisomer, a solvate or any pharmaceutical salt thereof.

It also relates to a compound as defined above for use as a medicine.

It further relates to a pharmaceutical composition comprising a compound as defined above, and a pharmaceutically acceptable support.

Another object of the invention is a compound or pharmaceutical composition as defined above, for use in the treatment of disorder modulated by sigma-1 receptor, including cognitive or neurodegenerative disorders. In a particular embodiment, said disorder is selected from the group consisting of (1) neurodegenerative diseases such as Alzheimer disease, Parkinson's disease, Huntington's disease, Amyotrophic lateral sclerosis, multiple sclerosis; (2) cognitive and memory alterations such as pathological ageing, ischemic amnesia, schizophrenia-related cognitive deficits and depression; (3) developmental cognitive disorders such as autism-related disorders and mental retardation-related disorders; and (4) genetic diseases associated with MAM dysfunctions.

### Detailed description

### Definitions

According to the present invention, the terms below have the following meanings:
The terms mentioned herein with prefixes such as for example C₁-C₃, C₁-C₆ or C₂-C₆ can also be used with lower numbers of carbon atoms such as C₁-C₂, C₁-C₅, or C₂-C₅. If, for example, the term C₁-C₃ is used, it means that the corresponding hydrocarbon chain may comprise from 1 to 3 carbon atoms, especially 1, 2 or 3 carbon atoms. If, for example, the term C₁-C₆ is used, it means that the corresponding hydrocarbon chain may comprise from 1 to 6 carbon atoms, especially 1, 2, 3, 4, 5 or 6 carbon atoms. If, for example, the term C₂-C₆ is used, it means that the corresponding hydrocarbon chain may comprise from 2 to 6 carbon atoms, especially 2, 3, 4, 5 or 6 carbon atoms.

The term "alkyl" refers to a saturated, linear or branched aliphatic group. The term "(C₁-C₃)alkyl" more specifically means methyl, ethyl, propyl, or isopropyl. The term "(C₁-C₆)alkyl" more specifically means methyl, ethyl, propyl, isopropyl, butyl, isobutyl, *tert*-butyl*,* pentyl or hexyl. In a preferred embodiment, the "alkyl" is a methyl, an ethyl, or a propyl, more preferably a methyl or an ethyl. The alkyl group includes halogenated alkyl group, such as perhalogenated alkyl (e.g. -CF₃)

The term "alkenyl" refers to an unsaturated, linear or branched aliphatic group comprising at least one carbon-carbon double bound. The term "(C₂-C₆)alkenyl" more specifically means ethenyl, propenyl, isopropenyl, butenyl, isobutenyl, pentenyl, prenyl or hexenyl. In a preferred embodiment, the "alkenyl" is an isobutenyl. "Isobutenyl" refers to a 2-methylprop-1-en-1-yl group.

The term "alkoxy" or "alkyloxy" corresponds to the alkyl group as above defined bonded to the molecule by an -O- (ether) bond. (C₁-C₃)alkoxy includes methoxy, ethoxy, propyloxy, and isopropyloxy. (C₁-C₆)alkoxy includes methoxy, ethoxy, propyloxy, isopropyloxy, butyloxy, isobutyloxy, tert-butyloxy, pentyloxy and hexyloxy. In a preferred embodiment, the "alkoxy" or "alkyloxy" is a methoxy.

The term "cycloalkyl" corresponds to a saturated or unsaturated (preferably at least one double carbon-carbon bond) mono-, bi- or tri-cyclic alkyl group having between 3 and 20 atoms of carbons (also named (C₃-C₂₀)cycloalkyl). It also includes fused, bridged, or spiro-connected cycloalkyl groups. The term "cycloalkyl" includes for instance cyclopropyl, cyclobutyl, cyclopentyl, and cyclohexyl. The term "cycloalkyl" may also refer to a 5-10 membered bridged carbocyclyl such as bicyclo[2,2,1]heptanyl, bicyclo[2,2,2]octanyl, or adamantyl, preferably bicyclo[2,2,2]octanyl. In a preferred embodiment, the "cycloalkyl" is a cyclopropyl, cyclopentyl or a cyclohexyl.

The term "heterocycloalkyl" corresponds to a saturated or unsaturated (preferably at least one double carbon-carbon bond) cycloalkyl group as above defined further comprising at least one heteroatom or heteroatomic group such as nitrogen, oxygen, or sulphur atom. It also includes fused, bridged, or spiro-connected heterocycloalkyl groups. Representative heterocycloalkyl groups include, but are not limited to 3-dioxolane, benzo [1,3] dioxolyl, pyranyl, tetrahydropyranyl, thiomorpholinyl, pyrazolidinyl, piperidyl, piperazinyl, azepanyl, 1,4-dioxanyl, imidazolinyl, pyrrolinyl, pyrrolidinyl, piperidinyl, imidazolidinyl, morpholinyl, 1,4-dithianyl, pyrrolidinyl, quinolizinyl, oxozolinyl, oxazolidinyl, isoxazolinyl, isoxazolidinyl, thiazolinyl, thiazolidinyl, isothiazolinyl, isothiazolidinyl, dihydropyranyl, tetrahydro-2H-pyranyl, tetrahydrofuranyl, and tetrahydrothiophenyl. The term "heterocycloalkyl" may also refer to a 5-10 membered bridged heterocyclyl such as 7-oxabicyclo[2,2,1]heptanyl. In a preferred embodiment, the heterocycloalkyl is azepanyl, piperidinyl, pyrrolidinyl or tetrahydropyranyl.

The term "aryl" corresponds to a mono- or bi-cyclic aromatic hydrocarbons having from 6 to 14 carbon atoms (also named (C₆-C₁₄)aryl). For instance, the term "aryl" includes phenyl, or naphthyl. In a preferred embodiment, the aryl is a phenyl.

The term "heteroaryl" as used herein corresponds to an aromatic, mono- or poly-cyclic group comprising between 5 and 14 atoms and comprising one or more heteroatoms, such as nitrogen (N), oxygen (O) or sulphur (S) atom, or heteroatomic groups. Examples of such mono- and poly-cyclic heteroaryl group may be: pyridinyl, thiazolyl, thiophenyl, furanyl, pyrrolyl, imidazolyl, triazolyl, tetrazolyl, benzofuranyl, thianaphthalenyl, indolyl, indolinyl, quinolinyl, isoquinolinyl, benzimidazolyl, triazinyl, thianthrenyl, isobenzofuranyl, phenoxanthinyl, isothiazolyl, isoxazolyl, pyrazinyl, pyridazinyl, indolizinyl, isoindolyl, indazolyl, purinyl, phtalazinyl, naphthyridinyl, quinoxalinyl, quinazolinyl, cinnolinyl, pteridinyl, carbazolyl, β-carbolinyl, phenanthridinyl, acridinyl, pyrimidinyl, phenanthrolinyl, phenazinyl, phenothiazinyl, furazanyl, phenoxazinyl, benzotriazolyl, benzoxazolyl, benzisoxazolyl, oxindolyl, benzothienyl, benzothiazolyl, s-triazinyl, oxazolyl, or thiofuranyl. In a preferred embodiment, the heteroaryl is pyridyl or imidazolyl.

The term "heterocycle" or "heterocyclic group" corresponds to a saturated or unsaturated, alicyclic or aromatic, mono- or poly-cyclic, hydrocarbon which contains one or more heteroatoms, such as oxygen (O), nitrogen (N) or sulphur atom (S), and optionally comprising one or more oxo groups. Heterocycles include, but are not limited to, heteroaryl, heterocycloalkyl, and other heterocyclic derivatives such as isoindolinyl, indolinyl, chromanyl, isochromanyl, phthalidyl, pyrrolidinonyl, imidazolidinonyl, chromenyl, tetrahydroquinolinyl, tetrahydroisoquinolinyl, xanthenyl, benzoxazolinyl, isatinyl, or dihydropyridyl. Preferably, the heterocycle is heteroaryl or heterocycloalkyl, and more preferably piperidinyl, pyrrolidinyl, azepanyl, pyrrolyl, imidazolyl or thiophenyl.

The term "nitrogen-containing heterocycle" or "nitrogen-containing heterocyclic group" refers to a heterocycle as defined above, containing at least one nitrogen atom. Examples of nitrogen-containing heterocycle include, but are not limited to, piperidinyl, pyrrolidinyl, azepanyl, pyrrolyl, or imidazolyl, preferably piperidinyl.

The term "arylalkyl" or "aralkyl" as used herein corresponds to an alkyl as defined above, substituted by at least one aryl group as defined above. More specifically, "aryl(C₁-C₆)alkyl" refers to a (C₁-C₆)alkyl as defined above, substituted by at least one aryl group as defined above. Examples of arylalkyl may be benzyl or phenylethyl (also named, phenethyl).

The term "alkylcycloalkyl" as used herein corresponds to a cycloalkyl as defined above, substituted by at least one alkyl group as defined above. More specifically, "(C₁-C₆)alkylcycloalkyl" refers to a cycloalkyl as defined above, substituted by at least one (C₁-C₆)alkyl group as defined above.

The term "alkylheterocycloalkyl" as used herein corresponds to a heterocycloalkyl as defined above, substituted by at least one alkyl group as defined above. More specifically, "(C₁-C₆)alkylheterocycloalkyl" refers to a heterocycloalkyl as defined above, substituted by at least one (C₁-C₆)alkyl group as defined above.

The term "alkylaryl" as used herein corresponds to an aryl as defined above, substituted by at least one alkyl group as defined above. More specifically, "(C₁-C₆)alkylaryl" refers to an aryl as defined above, substituted by at least one (C₁-C₆)alkyl group as defined above.

The term "alkylheteroaryl" as used herein corresponds to a heteroaryl as defined above, substituted by at least one alkyl group as defined above. More specifically, "(C₁-C₆)alkylheteroaryl" refers to a heteroaryl as defined above, substituted by at least one (C₁-C₆)alkyl group as defined above.

The term "halogen" corresponds to a fluorine, chlorine, bromine, or iodine atom, preferably a fluorine, chlorine or bromine, and more preferably a chlorine or a fluorine.

The expression "substituted by at least" means that the radical is substituted by one or several groups of the list.

It is understood that the substitution by two or more substituents on a hydrocarbon chain, such as an alkyl or cycloalkyl chain, may occur on the same carbon and/or on different carbons. For instance, the structures of an ethyl chain substituted by two given radicals "A" and "B", include, but is not limited to, structures of formulae (II) or (III):

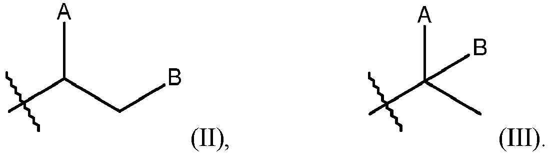

Unless specified otherwise, the alkyl, alkenyl, cycloalkyl, heterocycloalkyl, aryl, and heteroaryl groups as defined above may be unsubstituted or substituted by at least one substituent, said at least one substituent being selected from the group consisting of halogen, preferably fluorine and chlorine, CN, NO₂, SO₃H, alkyl, cycloalkyl, heterocycloalkyl, aryl, heteroaryl, -C(O)R, - C(O)₂R, -OH, -OR, -CHOHR, -C(O)NRR', -CONHOR, -CONHSO₂R, -NRR', -N(R)C(O)R', -N(R)NR'R", -N(R)C(O)₂R', -N(R)C(O)NR'R", -N(R)S(O)₂R', -SR, -S(O)R, -S(O₂)R, - S(O)NRR', -S(O)₂NRR'; R, R', and R" being independently H, (C₁-C₆)alkyl, cycloalkyl, aryl, heterocycloalkyl, heteroaryl, (C₁-C₆)alkylcycloalkyl, (C₁-C₆)alkylaryl, (C₁-C₆)alkylheterocycloalkyl), or (C₁-C₆)alkylheteroaryl.

As used herein, the terms "treatment", "treat" or "treating" refer to any act intended to ameliorate the health status of patients such as therapy, prevention, prophylaxis and retardation of a disease. In certain embodiments, such terms refer to the amelioration or eradication of the disease, or symptoms associated with it. In other embodiments, this term refers to minimizing the spread or worsening of the disease, resulting from the administration of one or more therapeutic agents to a subject with such a disease.

As used herein, the terms "subject", "individual" or "patient" are interchangeable and refer to an animal, preferably to a mammal, even more preferably to a human, including adult, child, newborn and human at the prenatal stage. However, the term "subject" can also refer to non-human animals, in particular mammals such as dogs, cats, horses, cows, pigs, sheep and non-human primates, among others.

The terms "quantity," "amount," and "dose" are used interchangeably herein and may refer to an absolute quantification of a molecule.

As used herein, the terms "active principle", "active ingredient" and "active pharmaceutical ingredient" are equivalent and refer to a component of a pharmaceutical composition having a therapeutic effect.

As used herein, the term "therapeutic effect" refers to an effect induced by an active ingredient, or a pharmaceutical composition according to the invention, capable to prevent or to delay the appearance of a disease, such as a neurodegenerative or cognitive disease, or to cure or attenuate the effects of a disease.

As used herein, the term "effective amount" refers to a quantity of an active ingredient or of a pharmaceutical composition which prevents, removes or reduces the deleterious effects of the disease. It is obvious that the quantity to be administered can be adapted by the man skilled in the art according to the subject to be treated, to the nature of the disease, etc. In particular, doses and regimen of administration may be function of the nature, of the stage and of the severity of the disease to be treated, as well as of the weight, the age and the global health of the subject to be treated, as well as of the judgment of the doctor.

As used herein, the term "excipient or pharmaceutically acceptable carrier" refers to any ingredient except active ingredients that is present in a pharmaceutical composition. Its addition may be aimed to confer a particular consistency or other physical or gustative properties to the final product. An excipient or pharmaceutically acceptable carrier must be devoid of any interaction, in particular chemical, with the active ingredients.

### Compounds

It is the purpose of the present invention to provide compounds, or pharmaceutically acceptable salts thereof, especially for use in the treatment of disorder modulated by σ₁ receptor (such as cognitive or neurodegenerative disorders), said compounds having the formula (I) as shown below, 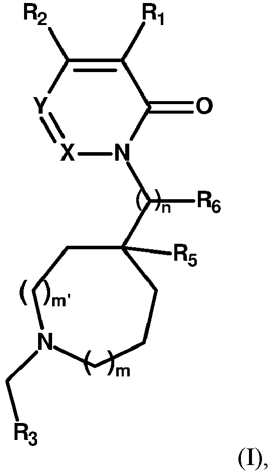
R₁ and R₂ represent independently:
   - H;
   - an aryl(C₁-C₆)alkyl group;
   - an aryl group;
   - a cycloalkyl group;
   - a heterocyclic group, preferably a nitrogen-containing heterocyclic group; or
   - SR, R being alkyl, aryl or aralkyl group;
   wherein one of R₁ and R₂ is H, and the other one of R₁ and R₂ is different from H;
X and Y are either, respectively:
   - CH and N, or
   - N and CR₄, or
   - CH and CR₄,
   wherein R₄ represents H or a (C₁-C₄)alkyl;
n is 0, 1, or 2; m is 0 or 1; m' is 0 or 1;
R₃ represents:
   - a radical selected from the group consisting of H, (C₁-C₆)alkyl, (C₂-C₆)alkenyl, aryl, heteroaryl, cycloalkyl, heterocycloalkyl, (C₁-C₆)alkoxy, and aryl(C₁-C₆)alkyl, said radical being optionally substituted by one or more groups chosen among (C₁-C₆)alkyl, OH, halogen, aryl, heteroaryl, cycloalkyl, heterocycloalkyl, (C₁-C₆)alkoxy, - C(O)R, -CHOHR, C(O)₂R, C(O)NRR', -CONHOR, -CONHSO₂R, -NRR', - N(R)C(O)R', -N(R)NR'R", -N(R)C(O)₂R', -N(R)C(O)NR'R", -N(R)S(O)₂R', -SR, - S(O)R, -S(O)₂R, -S(O)NRR', and -S(O)₂NRR'; R, R', and R" being independently H, (C₁-C₆)alkyl, cycloalkyl, aryl, heterocycloalkyl, heteroaryl, (C₁-C₆)alkylcycloalkyl, (C₁-C₆)alkylaryl, (C₁-C₆)alkylheterocycloalkyl), or (C₁-C₆)alkylheteroaryl; or
   - a radical selected from the group consisting of OH, -C(O)R, -CHOHR, C(O)₂R, C(O)NRR', -CONHOR, -CONHSO₂R, -NRR', -N(R)C(O)R', -N(R)NR'R", - N(R)C(O)₂R', -N(R)C(O)NR'R", -N(R)S(O)₂R', -SR, -S(O)R, -S(O)₂R, -S(O)NRR', and -S(O)₂NRR'; R, R', and R" being independently H, (C₁-C₆)alkyl, cycloalkyl, aryl, heterocycloalkyl, heteroaryl, (C₁-C₆)alkylcycloalkyl, (C₁-C₆)alkylaryl, (C₁-C₆)alkylheterocycloalkyl), or (C₁-C₆)alkylheteroaryl;
R₅ represents H or OH; and
each of R₆ represents independently H or a (C₁-C₆)alkyl group;
a stereoisomer, a solvate or any pharmaceutical salt thereof.

In a particular embodiment, the compounds of the invention are of formula (I) where R₁ and R₂ represent independently:
- H;
- an aryl(C₁-C₆)alkyl group;
- an aryl group;
- a cycloalkyl group;
- a heterocyclic group, preferably nitrogen-containing heterocyclic group; or
- SR, R being alkyl, aryl or aralkyl group;

wherein one of R₁ and R₂ is H, and the other one of R₁ and R₂ is different from H;
said group is optionally substituted by at least one -OH, halogen, (C₁-C₆)alkyl, or (C₁-C₆)alkyloxy;

In a particular embodiment, the compounds of the invention are of formula (I) where R₁ represents:
- an aryl(C₁-C₆)alkyl group;
- an aryl group;
- a cycloalkyl group;
- a heterocyclic group, preferably nitrogen-containing heterocyclic group; or
- SR, R being alkyl, aryl or aralkyl;
and R₂ is H.

In another particular embodiment, the compounds of the invention are of formula (I) where R₁ and R₂ represent independently:
- H;
- an aryl(C₁-C₆)alkyl group, such as a benzyl or a phenethyl;
- an aryl group, such as a phenyl; or
- a heterocyclic group, preferably nitrogen-containing heterocyclic group, such as a piperidinyl, pyrrolidinyl, azepanyl, pyrrolyl, or imidazolyl;

wherein one of R₁ and R₂ is H, and the other one of R₁ and R₂ is different from H,
said group being optionally substituted by at least one -OH, halogen, (C₁-C₆)alkyl, or (C₁-C₆)alkyloxy group.

In a preferred embodiment, the compounds of the invention are of formula (I) where R₁ represents:
- an aryl(C₁-C₆)alkyl group, such as a benzyl or a phenethyl, preferably a phenethyl;
- an aryl group, such as a phenyl; or
- a heterocyclic group, preferably nitrogen-containing heterocyclic group, such as a piperidinyl, pyrrolidinyl, azepanyl, pyrrolyl, or imidazolyl; and
R₂ represents H.

In a more preferred embodiment, the compounds of the invention are of formula (I) where R₁ represents:
- an aryl(C₁-C₆)alkyl group, such as a benzyl or a phenethyl, preferably a phenethyl; or
- an aryl group, such as a phenyl; and
R₂ represents H.

According to a particular embodiment, when R₁ or R₂ represents a nitrogen-containing heterocycle, said group is preferably attached to the rest of the molecule by a nitrogen atom of said nitrogen-containing heterocycle.

Preferably, R₁ is an aryl, such as a phenyl.

Preferably, R₂ is H.

In a more particularly embodiment, R₂ is H and R₁ is a phenyl, optionally substituted by one or more groups selected from the group consisting of halogen, preferably fluorine or chlorine, alkyl, including alkyl group substituted by one or more halogen atoms (such as CF₃), cycloalkyl, -OH, or a (C₁-C₆)alkoxy, such as methoxy, as defined above.

In another particular embodiment, R₁ represents H;
and R₂ represents:
- an aryl, such as a phenyl;
- an aryl(C₁-C₆)alkyl group, such as a benzyl or a phenethyl; or
- a heterocyclic group, preferably nitrogen-containing heterocyclic group, such as a piperidinyl;
preferably, R₂ is an aryl, such as a phenyl.

In another particular embodiment, when R₁ or R₂ represents an aryl, such as a phenyl, said aryl may be unsubstituted or substituted by at least one substituent selected from:
- a halogen, preferably a fluorine or a chlorine;
- an alkyl optionally substituted by at least one halogen, preferably a fluorine, more preferably the alkyl group is -CF₃;
- -OH; and
- -OR, R being an alkyl, cycloalkyl, heterocycloalkyl, aryl or heteroaryl, preferably an alkyl, more preferably a methyl or CF₃.

In a particular embodiment, R₃ represents:
- H;
- a (C₁-C₆)alkyl group, such as a methyl or an ethyl;
- a (C₂-C₆)alkenyl group, such as an isobutenyl;
- a cycloalkyl group, such as a cyclopropyl, a cyclopentyl, a cyclohexyl or a cyclohexenyl;
- an aryl group, such as a phenyl;
- a heterocycloalkyl group, such as a tetrahydropyranyl;
- a heteroaryl group, such as a pyridyl or a imidazolyl; said group being optionally substituted by at least one (C₁-C₆)alkyl, OH, halogen (e.g. fluorine, chlorine), aryl (e.g. phenyl), heteroaryl, cycloalkyl, heterocycloalkyl, (C₁-C₆)alkoxy, -C(O)₂R, -N(R)C(O)R' ; R and R' being independently H, (C₁-C₆)alkyl, cycloalkyl, aryl (such as a phenyl optionally substituted by a halogen (preferably a fluorine)), heterocycloalkyl, heteroaryl, (C₁-C₆)alkylcycloalkyl, (C₁-C₆)alkylaryl, (C₁-C₆)alkylheterocycloalkyl), or (C₁-C₆)alkylheteroaryl; and preferably said group being optionally substituted by at least one OH, halogen (e.g. chlorine), alkoxy (e.g. methoxy), - N(R)C(O)R' with R and R' being H or (C₁-C₆)alkyl (e.g. methyl); or
- a radical selected from -CHOHR, -C(O)R, -C(O)₂R ; R being H, (C₁-C₆)alkyl, cycloalkyl, aryl, heterocycloalkyl, heteroaryl, (C₁-C₆)alkylcycloalkyl, (C₁-C₆)alkylaryl, (C₁-C₆)alkylheterocycloalkyl), or (C₁-C₆)alkylheteroaryl, R being preferably aryl (e.g. phenyl) optionally substituted by a halogen (e.g. fluorine).

In another particular embodiment, R₃ is selected from the group consisting of:
- an aryl, such as phenyl;
- a heterocycloalkyl, such as a tetrahydropyranyl; and
- a (C₁-C₆)alkyl, such as a methyl, optionally substituted by a OH.

In a preferred embodiment, R₃ is selected from the group consisting of:
- a cyclopentyl, a cyclohexyl, a cyclopropyl,
- a pyridyl, an imidazolyl,
- a phenyl, said phenyl being optionally substituted by a halogen such as a chlorine or fluorine, or by OH,
- a benzyl,
- a tetrahydropyranyl,
- a radical -C(O)zR, R being a (C₁-C₆)alkyl, preferably a methyl,
- a radical -CHOHR, R being an aryl, preferably a phenyl optionally substituted by a fluorine,
- a radical -C(O)R, R being an aryl, preferably a phenyl optionally substituted by a fluorine;
- a methyl, optionally substituted by one or two substituents, each substituent being independently selected from the group consisting of:
   ▪ a phenyl,
   ▪ OH,
   ▪ a methoxy,
   ▪ a radical -NHC(O)R', R' being a (C₁-C₆)alkyl, preferably a methyl;
- an ethyl; and
- an isobutenyl.

In a more preferred embodiment, R₃ is a phenyl.

In a particular embodiment, X and Y are either, respectively:
- CH and N, or
- N and CR₄, with R₄ being H or (C₁-C₄)alkyl, preferably H.

In a preferred embodiment, X and Y are N and CR₄, respectively, wherein R₄ is H or a (C₁-C₄)alkyl, preferably R₄ is H or methyl, more preferably R₄ is H.

In a preferred embodiment, R₅ is H.

In a particular embodiment, (each) R₆ is independently H or methyl.

It is understood that:
- when n is 0, then R₆ is not present;
- when n is 1, then one R₆ is present and the compound of formula (I) is represented by the following structure: 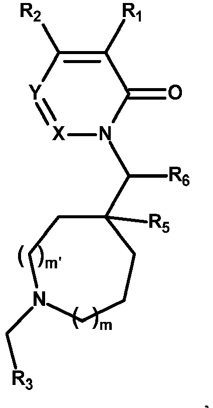 wherein R₁, R₂, R₃, R₅, X, Y, m, and m' are as defined above, and R₆ is H or a (C₁-C₆)alkyl group;
- when n is 2, then two R₆'s are present and the compound of formula (I) is represented by the following structure: 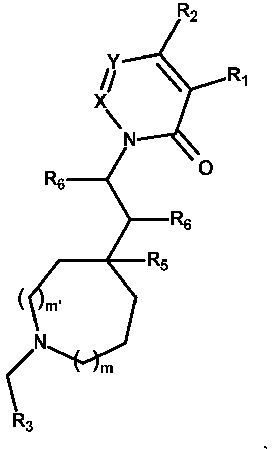
wherein R₁, R₂, R₃, R₅, X, Y, m, and m' are as defined above, and each R₆ is independently H or a (C₁-C₆)alkyl group.

In a preferred embodiment, R₆ is H (each R₆ when n is 2).

In a preferred embodiment, R₅ and (each) R₆ are H.

In a particular embodiment, m is 0.

In another particular embodiment, m' is 1.

In another particular embodiment, m is 0 and m' is 1.

Preferably, n is 0 or 1, and more preferably n is 1.

In a particular embodiment, the compound of the invention is a compound of formula (I) wherein at least one of, and preferably all, the following features is (are) fulfilled:
n is 1; and/or
m is 0; and/or
m' is 1; and/or
R₁ represents an aryl group; and/or
R₂ is H; and/or
R₃ represents:
   - a radical selected from H, an aryl (e.g. phenyl), a (C₁-C₆)alkyl (e.g. methyl, ethyl), a (C₂-C₆)alkenyl (e.g. isobutenyl), a cycloalkyl (e.g. cyclopropyl, cyclopentyl, cyclohexyl), a heterocycloalkyl (e.g. tetrahydropyranyl), a heteroaryl (e.g. pyridyl, imidazolyl), (C₁-C₆)alkoxy (e.g. methoxy), aryl(C₁-C₆)alkyl (e.g. benzyl),
      said radical being optionally substituted by one or two (C₁-C₆)alkyl, OH, halogen (e.g. fluorine, chlorine), aryl (e.g. phenyl), heteroaryl, cycloalkyl, heterocycloalkyl, (C₁-C₆)alkoxy, -C(O)₂R, -N(R)C(O)R'; R and R' being independently H, (C₁-C₆)alkyl, cycloalkyl, aryl (such as a phenyl optionally substituted by a halogen (preferably a fluorine)), heterocycloalkyl, heteroaryl, (C₁-C₆)alkylcycloalkyl, (C₁-C₆)alkylaryl, (C₁-C₆)alkylheterocycloalkyl), or (C₁-C₆)alkylheteroaryl; or
   - a radical selected from the group consisting of -CHOHR, -C(O)R, -C(O)₂R ; R being H, (C₁-C₆)alkyl, cycloalkyl, aryl, heterocycloalkyl, heteroaryl, (C₁-C₆)alkylcycloalkyl, (C₁-C₆)alkylaryl, (C₁-C₆)alkylheterocycloalkyl), or (C₁-C₆)alkylheteroaryl;and/or
      R₅ is H; and/or
      each R₆ is H.

In a particular embodiment,

R₁ is selected from the group consisting of:
- aryl(C₁-C₆)alkyl group, such as phenethyl,
- an aryl group, such a phenyl, optionally substituted by a halogen (e.g. a chlorine), and
- a heterocyclic group (preferably a nitrogen-containing heterocyclic group), such as a piperidinyl;

R₂ is H;
R₃ is selected from the group consisting of:
   - an aryl, such as phenyl;
   - a heterocycloalkyl, such as a tetrahydropyranyl; and
   - a (C₁-C₆)alkyl, such as a methyl, optionally substituted by a OH.
R₅ is H or OH, preferably H; and
R₆ is H or a (C₁-C₆)alkyl, such a methyl, preferably H;
X and Y are either, respectively:
   - CH and N, or
   - N and CR₄, with R₄ being H or (C₁-C₄)alkyl, preferably H,
   - preferably, N and CH;
n is 0 or 1, preferably 1; and
m is 0 and m' is 1.

In a particular embodiment, the aryl group is a phenyl group optionally substituted by at least one substituent, said at least one substituent being selected from:
- a halogen, preferably a fluorine or a chlorine;
- an alkyl optionally substituted by at least one halogen, preferably a fluorine, preferably - CF₃;
- -OH; and
- -OR, R being an alkyl, cycloalkyl, heterocycloalkyl, aryl or heteroaryl, preferably an alkyl, more preferably a methyl.

In a more particular embodiment,
> R₁ and R₂ represent independently:
   - H;
   - a benzyl, a phenethyl;
   - a phenyl group, optionally substituted by a fluorine, a chlorine, a hydroxy, a methoxy, or -CF₃, ;
   - a piperidinyl group;
wherein one of R₁ and R₂ is H, and the other one of R₁ and R₂ is different from H;
> X and Y are either CH and N, or N and CH, or CH and CH, respectively;
> n is 0, 1, or 2;
> m is 0 or 1; m' is 0 or 1;
> R₃ represents:
   - H
   - a cyclopentyl, a cyclohexyl, a cyclopropyl,
   - a pyridyl, an imidazolyl,
   - a phenyl, said phenyl being optionally substituted by a halogen such as a chlorine, or by OH,
   - a benzyl,
   - a tetrahydropyranyl,
   - a radical -C(O)₂R, R being a (C₁-C₆)alkyl preferably a methyl,
   - a radical -CHOHR, R being an aryl, preferably a phenyl optionally substituted by a fluorine,
   - a radical -C(O)R, R being an aryl, preferably a phenyl optionally substituted by a fluorine;
   - a methyl, optionally substituted by one or two substituents, each substituent being independently selected from the group consisting of:
      ▪ a phenyl,
      ▪ OH,
      ▪ a methoxy,
      ▪ a radical -NHC(O)R, R being a (C₁-C₆)alkyl, preferably a methyl;
   - an ethyl; or
   - an isobutenyl.

As used herein, "a compound of the invention" means a compound described above or a pharmaceutically acceptable salt, any isomer or solvate form thereof.

The term "isomer" refers to compounds which have identical molecular formulae as identified herein but which differ by nature or in the binding sequence of their atoms or in the layout of their atoms in space. Isomers which differ in the layout of their atoms in space are designated by "stereoisomers". Stereosiomers which are not mirror images of each other, are designated as "diastereoisomers", and stereoisomers which are non-superposable mirror images of each other are designated as "enantiomers" or "optical isomers". "Stereoisomers" refer to racemates, enantiomers and diastereoisomers.

The person skilled in the art will recognize that stereocenters exist in the compounds of the invention. Any chiral center of a compound of the invention can be (R), (S) or racemate. Accordingly, the present invention includes all possible stereoisomers and geometric isomers of the compounds of formula (I) and includes not only racemic compounds but also the optically active isomers as well. According to a preferred embodiment, compounds of the invention are of formula (II). When a compound of formula (I) is desired as a single enantiomer, it may be obtained either by resolution of the final product or by stereospecific synthesis from either isomerically pure starting material or any suitable intermediate. Resolution of the final product, an intermediate or a starting material may be carried out by any suitable method known in the art. See, for example, Stereochemistry of Carbon Compounds by E. L. Eliel (Mcgraw Hill, 1962) and Tables of Resolving Agents by S. H. Wilen.

The specialist in the art of organic chemistry will appreciate that many organic compounds can form complexes with solvents in which they are reacted or from which they are precipitated or crystallized. These complexes are known as "solvates". For example, a complex with water is known as a "hydrate". Solvates of the compounds of formula (I) or (II) are within the scope of the present invention.

It will also be appreciated by the specialist in organic chemistry that many organic compounds can exist in more than one crystalline form. For example, crystalline form may vary from solvate to solvate. Thus, all crystalline forms of the compounds of the invention or the pharmaceutically acceptable solvates thereof are within the scope of the present invention.

The term "pharmaceutically acceptable salts" or "pharmaceutical salts" of the compounds of the invention include conventional salts formed from pharmaceutically acceptable inorganic or organic acids or bases as well as quaternary ammonium salts. More specific examples of suitable acid salts include hydrochloric, hydrobromic, sulfuric, phosphoric, nitric, perchloric, fumaric, acetic, propionic, succinic, glycolic, formic, lactic, maleic, tartaric, citric, palmoic, malonic, hydroxymaleic, phenylacetic, glutamic, benzoic, salicylic, fumaric, toluenesulfonic, methanesulfonic, naphthalene-2-sulfonic, benzenesulfonic hydroxynaphthoic, hydroiodic, malic, steroic, tannic etc. More specific examples of suitable basic salts include sodium, lithium, potassium, magnesium, aluminium, calcium, zinc, N,N'-dibenzylethylenediamine, chloroprocaine, choline, diethanolamine, ethylenediamine, N-methylglucamine and procaine salts.

For example, preferred salt forms include hydrochloride.

In a particular embodiment, the compound of formula (I) is selected in the group consisting of:
2-(1-benzylpiperidin-4-yl)-4-phenylpyridazin-3(2H)-one hydrochloride;
2-(1-benzylpiperidin-4-yl)-5-phenylpyridazin-3(2H)-one hydrochloride;
4-phenyl-2-[1-(2-phenylethyl)piperidin-4-yl]-2,3-dihydropyridazin-3-one hydrochloride;
2-[1-(cyclopropylmethyl)piperidin-4-yl]-4-phenyl-2,3-dihydropyridazin-3-one hydrochloride;
2-[1-(cyclopentylmethyl)piperidin-4-yl]-4-phenyl-2,3-dihydropyridazin-3-one hydrochloride;
2-(1-benzylazepan-4-yl)-4-phenyl-2,3-dihydropyridazin-3-one hydrochloride;
2-((1-benzylpiperidin-4-yl)methyl)-6-methyl-4-phenylpyridazin-3(2H)-one;
2-((1-benzylpiperidin-4-yl)methyl)-5-phenylpyridazin-3(2H)-one hydrochloride;
2-[(1-benzylpiperidin-3-yl) methyl]-4-phenyl-2,3-dihydropyridazin-3-one hydrochloride;
2-((1-phenethylpiperidin-4-yl)methyl)-4-phenylpyridazin-3(2H)-one hydrochloride;
4-phenyl-2-((1-propylpiperidin-4-yl)methyl)pyridazin-3(2H)-one hydrochloride;
2-((1-(4-chlorobenzyl)piperidin-4-yl)methyl)-4-phenylpyridazin-3(2H)-one hydrochloride;
2-((1-(cyclohexylmethyl)piperidin-4-yl)methyl)-4-phenylpyridazin-3(2H)-one hydrochloride;
4-phenyl-2-((1-(pyridin-4-ylmethyl)piperidin-4-yl)methyl)pyridazin-3(2H)-one hydrochloride;
4-phenyl-2-((1-((tetrahydro-2H-pyran-4-yl)methyl)piperidin-4-yl)methyl)pyridazin-3(2H)-one, hydrochloride;
2-{[1-(1H-imidazol-5-ylmethyl)piperidin-4-yl]methyl}-4-phenyl-2,3-dihydropyridazin-3-one, hydrochloride;
4-phenyl-2-((1-((tetrahydro-2H-pyran-3-yl)methyl)piperidin-4-yl)methyl)pyridazin-3(2H)-one hydrochloride;
2-((1-(4-hydroxybenzyl)piperidin-4-yl)methyl)-4-phenylpyridazin-3(2H)-one hydrochloride;
2-((1-(2-methoxyethyl)piperidin-4-yl)methyl)-4-phenylpyridazin-3(2H)-one hydrochloride;
2-((1-(2-hydroxyethyl)piperidin-4-yl)methyl)-4-phenylpyridazin-3(2H)-one hydrochloride;
2-((1-(2-methoxy-1-phenylethyl)piperidin-4-yl)methyl)-4-phenylpyridazin-3(2H)-one hydrochloride;
N-(2-{4-[(6-oxo-5-phenyl-1,6-dihydropyridazin-1-yl)methyl]piperidin-1-yl}ethyl)acetamide hydrochloride;
2-((1-(2-(4-fluorophenyl)-2-hydroxyethyl)piperidin-4-yl)methyl)-4-phenylpyridazin-3(2H)-one hydrochloride;
2-((1-(2-(4-fluorophenyl)-2-oxoethyl)piperidin-4-yl)methyl)-4-phenylpyridazin-3(2H)-one hydrochloride;
2-((1-(2-hydroxy-2-phenylethyl)piperidin-4-yl)methyl)-4-phenylpyridazin-3(2H)-one hydrochloride;
2-(2-(1-benzylpiperidin-4-yl)ethyl)-4-phenylpyridazin-3(2H)-one hydrochloride;
2-(2-(1-benzylpiperidin-4-yl)ethyl)-5-phenylpyridazin-3(2H)-one hydrochloride;
2-(1-(1-benzylpiperidin-4-yl)ethyl)-4-phenylpyridazin-3(2H)-one hydrochloride;
2-((1-benzyl-4-hydroxypiperidin-4-yl)methyl)-4-phenylpyridazin-3(2H)-one hydrochloride;
2-(1-benzylpiperidin-4-yl)-4-(4-methoxyphenyl)pyridazin-3(2H)-one hydrochloride;
2-(1-benzylpiperidin-4-yl)-4-[4-(trifluoromethyl)phenyl]-2,3-dihydropyridazin-3-one hydrochloride;
2-(1-benzylpiperidin-4-yl)-4-[4-(chlorophenyl]-2,3-dihydropyridazin-3-one hydrochloride;
2-[(1-benzylpiperidin-4-yl)methyl]-4-(2-chlorophenyl)-2,3-dihydropyridazin-3-one hydrochloride;
2-[(1-benzylpiperidin-4-yl)methyl]-4-(4-hydroxyphenyl)-2,3-dihydropyridazin-3-one hydrochloride;
2-((1-benzylpiperidin-4-yl)methyl)-4-(4-fluorophenyl)pyridazin-3(2H)-one hydrochloride;
2-((1-benzylpiperidin-4-yl)methyl)-4-phenethylpyridazin-3(2H)-one,
2-(1-benzylpiperidin-4-yl)-4-(2-phenylethyl)-2,3-dihydropyridazin-3-one hydrochloride;
2-(1-benzylpiperidin-4-yl)-4-(piperidin-1-yl)-2,3-dihydropyridazin-3-one hydrochloride;
2-[(1-benzylpiperidin-4-yl)methyl]-4-(piperidin-1-yl)-2,3-dihydropyridazin-3-one hydrochloride;
2-((1-benzylpiperidin-4-yl)methyl)-4-morpholinopyridazin-3(2H)-one hydrochloride;
2-((1-benzylpiperidin-4-yl)methyl)-4-(4-phenylpiperazin-1-yl)pyridazin-3(2H)-one hydrochloride;
2-((1-benzylpiperidin-4-yl)methyl)-4-(3,4-dihydroisoquinolin-2(1H)-yl)pyridazin-3(2H)-one hydrochloride;
3-(1-benzylpiperidin-4-yl)-5-phenylpyrimidin-4(3H)-one hydrochloride;
1-(1-benzylpiperidin-4-yl)-3-phenyl-1,2-dihydropyridin-2-one hydrochloride;
3-((1-benzylpiperidin-4-yl)methyl)-5-phenylpyrimidin-4(3H)-one; and
1-[(1-benzylpiperidin-4-yl)methyl]-3-phenyl-1,2-dihydropyridin-2-one hydrochloride.

Preferably, said compound is selected in the group consisting of:
2-(1-benzylpiperidin-4-yl)-4-phenylpyridazin-3(2H)-one hydrochloride;
3-(1-benzylpiperidin-4-yl)-5-phenylpyrimidin-4(3H)-one hydrochloride;
2-((1-benzylpiperidin-4-yl)methyl)-4-phenethylpyridazin-3(2H)-one;
2-[(1-benzylpiperidin-4-yl)methyl]-4-(piperidin-1-yl)-2,3-dihydropyridazin-3-one hydrochloride;
2-((1-benzyl-4-hydroxypiperidin-4-yl)methyl)-4-phenylpyridazin-3(2H)-one hydrochloride;
4-phenyl-2-((1-((tetrahydro-2H-pyran-4-yl)methyl)piperidin-4-yl)methyl)pyridazin-3(2H)-one, hydrochloride;
2-(1-(1-benzylpiperidin-4-yl)ethyl)-4-phenylpyridazin-3(2H)-one hydrochloride; and
2-((1-(2-hydroxyethyl)piperidin-4-yl)methyl)-4-phenylpyridazin-3(2H)-one hydrochloride.

The compounds of the invention can be prepared by several methods, and in particular the methods as illustrated in the examples. The starting products are commercial products or products prepared according to known synthesis from commercial compounds or known to one skilled in the art.

### Therapeutic use of the compounds

The present invention relates to a pharmaceutical or veterinary composition comprising a compound according to the invention. Preferably, the pharmaceutical composition further comprises a pharmaceutically or veterinary acceptable carrier (or "support") or excipient. The invention further relates to a method for treating a disease in a subject, wherein a therapeutically effective amount of a compound according to the invention, is administered to said subject in need thereof. The present invention relates to the use of a compound according to the invention as a medicine. The invention also relates to the use of a compound according to the invention, for the manufacture of a medicine.

In addition, the present invention relates to a method for treating of a disorder modulated by sigma-1 receptor (e.g. cognitive or neurodegenerative disorders), in a subject, wherein a therapeutically effective amount of a compound according to the invention, is administered to said subject suffering of a disorder modulated by sigma-1 receptor (e.g. cognitive or neurodegenerative disorders).

The invention also relates to the use of the compounds according to the invention, for the manufacture of a medicine for the treatment of a disorder modulated by sigma-1 receptor (e.g. cognitive or neurodegenerative disorders). The invention relates to a compound according to the invention for use in the treatment of a disorder modulated by sigma-1 receptor (e.g. cognitive disorders).

In a particular embodiment of the invention, said disorder modulated by sigma-1 receptor is selected from the group consisting of (1) neurodegenerative diseases including, but not limited to, Alzheimer disease, Parkinson's disease, Huntington's disease, Amyotrophic lateral sclerosis, multiple sclerosis; (2) cognitive and memory alterations including, but not limited to, pathological ageing, ischemic amnesia, schizophrenia-related cognitive deficits, depression; (3) developmental cognitive disorders including, but not limited to, autism-related disorders, mental retardation-related disorders; and (4) genetic diseases associated with MAM dysfunctions.

### Pharmaceutical compositions

The present invention also relates to a pharmaceutical composition comprising a compound of the present invention. The composition further comprises at least one pharmaceutically acceptable carrier (or "support") or excipient.

The invention also concerns the pharmaceutical composition of the invention for use in the treatment of a disease. The invention also relates to the use of a pharmaceutical composition according to the invention for the manufacture of a medicine for treating a disease in a subject. The invention further relates to a method for treating a disease in a subject, wherein a therapeutically effective amount of a pharmaceutical composition according to the invention is administered to said subject suffering from said disease.

Preferably, the disease is a disorder modulated by sigma-1 receptor. More preferably, the disease is selected from the group consisting of (1) neurodegenerative diseases including, but not limited to, Alzheimer disease, Parkinson's disease, Huntington's disease, Amyotrophic lateral sclerosis, multiple sclerosis; (2) cognitive and memory alterations including, but not limited to, pathological ageing, ischemic amnesia, schizophrenia-related cognitive deficits, depression; (3) developmental cognitive disorders including, but not limited to, autism-related disorders, mental retardation-related disorders; and (4) genetic diseases associated with MAM dysfunctions.

### Subject, regimen and administration

The subj ect according to the invention is an animal, preferably a mammal, even more preferably a human. However, the term "subject" can also refer to non-human animals, in particular mammals such as dogs, cats, horses, cows, pigs, sheep, donkeys, rabbits, ferrets, gerbils, hamsters, chinchillas, rats, mice, guinea pigs and non-human primates, among others, that are in need of treatment.

The human subject according to the invention may be a human at the prenatal stage, a newborn, a child, an infant, an adolescent or an adult, in particular an adult of at least 40 years old, preferably an adult of at least 50 years old.

In a preferred embodiment, the subject has been diagnosed with a disease. Preferably, the subject has been diagnosed with a disease modulated by sigma-1 receptor.

Diagnostic methods of these diseases are well known by the man skilled in the art.

The compound according to the invention or the pharmaceutical composition according to the invention may be administered by any conventional route of administration. In particular, the compound or the pharmaceutical composition of the invention can be administered by a topical, enteral, oral, parenteral, intranasal, intravenous, intra-arterial, intramuscular, intratumoral, subcutaneous or intraocular administration and the like.

In particular, the compound according to the invention or the pharmaceutical composition according to the invention can be formulated for a topical, enteral, oral, parenteral, intranasal, intravenous, intra-arterial, intramuscular, subcutaneous or intraocular administration and the like.

Preferably, the compound according to the invention or the pharmaceutical composition according to the invention is administered by enteral or parenteral route of administration. When administered parenterally, the compound according to the invention or the pharmaceutical composition according to the invention is preferably administered by intravenous route of administration. When administered enterally, the compound according to the invention or the pharmaceutical composition according to the invention is preferably administered by oral route of administration.

The pharmaceutical composition comprising the molecule is formulated in accordance with standard pharmaceutical practice (Lippincott Williams & Wilkins, 2000 and Encyclopedia of Pharmaceutical Technology, eds. J. Swarbrick and J. C. Boylan, 1988-1999, Marcel Dekker, New York) known by a person skilled in the art.

For oral administration, the composition can be formulated into conventional oral dosage forms such as tablets, capsules, powders, granules and liquid preparations such as syrups, elixirs, and concentrated drops. Nontoxic solid carriers or diluents may be used which include, for example, pharmaceutical grades of mannitol, lactose, starch, magnesium stearate, sodium saccharine, talcum, cellulose, glucose, sucrose, magnesium, carbonate, and the like. For compressed tablets, binders, which are agents which impart cohesive qualities to powdered materials, are also necessary. For example, starch, gelatin, sugars such as lactose or dextrose, and natural or synthetic gums can be used as binders. Disintegrants are also necessary in the tablets to facilitate break-up of the tablet. Disintegrants include starches, clays, celluloses, algins, gums and crosslinked polymers. Moreover, lubricants and glidants are also included in the tablets to prevent adhesion to the tablet material to surfaces in the manufacturing process and to improve the flow characteristics of the powder material during manufacture. Colloidal silicon dioxide is most commonly used as a glidant and compounds such as talc or stearic acids are most commonly used as lubricants.

For transdermal administration, the composition can be formulated into ointment, cream or gel form and appropriate penetrants or detergents could be used to facilitate permeation, such as dimethyl sulfoxide, dimethyl acetamide and dimethylformamide.

For transmucosal administration, nasal sprays, rectal or vaginal suppositories can be used. The active compound can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate.

Pharmaceutical compositions according to the invention may be formulated to release the active drug substantially immediately upon administration or at any predetermined time or time period after administration.

Preferably, the treatment with the compound according to the invention or the pharmaceutical composition according to the invention starts no longer than one year, preferably no longer than six, five, four, three, two or one month(s), after the diagnosis of the disease.

The compound according to the invention or the pharmaceutical composition according to the invention may be administered as a single dose or preferably in multiple doses.

Preferably, the treatment is administered regularly, preferably between every day and every month, more preferably between every day and every two weeks, more preferably between every day and every week, even more preferably the treatment is administered every day. In a particular embodiment, the treatment is administered several times a day, such as 2 or 3 times a day.

The duration of treatment with the compound according to the invention or the pharmaceutical composition according to the invention is preferably comprised between one week, more preferably between one week and one or more year(s). Alternatively, the treatment may last as long as the disease persists.

The amount of compound according to the invention or of pharmaceutical composition according to the invention to be administered has to be determined by standard procedure well known by those of ordinary skills in the art. Physiological data of the patient (e.g. age, size, and weight) and the routes of administration have to be taken into account to determine the appropriate dosage, so as a therapeutically effective amount will be administered to the patient. In a preferred embodiment, the total compound dose for each administration of the compound according to the invention or of the pharmaceutical composition according to the invention is comprised between 0.00001 and 1 g, preferably between 0.01 and 10 mg.

The form of the pharmaceutical compositions, the route of administration and the dose of administration of the compound according to the invention, or the pharmaceutical composition according to the invention can be adjusted by the man skilled in the art according to the type and severity of the disease, and to the patient, in particular its age, weight, sex, and general physical condition.

The invention will also be described in further detail in the following examples, which are not intended to limit the scope of this invention, as defined by the attached claims.

### Examples

The following synthetic methods and schemes illustrate the general procedures by which the compounds of the present invention can be prepared. Starting materials have been obtained from commercially sources or prepared by using methods well known to those of ordinary skill in the art. For example, the compounds of the present invention can be prepared in accordance with or in analogy to the synthetic routes described in detail in the examples section. In particular compounds of the general formula (I) and their pharmaceutically acceptable salts can be synthesized according to methods described in the following schemes where X represents a halogen and R any group at the corresponding position of the general formula (I). While the numbering of the groups R in the following schemes differs from the designation of the groups in the general formula (I), it will be understood that these schemes explain the preparation of compounds of formula (I) and thus these groups R are defined in accordance with the corresponding groups at the same positions in attachment in the general formula (I). Purification of intermediates and final products was carried out via normal or reverse phase chromatography using a Dionex UltiMate 300 with the following parameters: Flow rate of 0.5 mL/min, column temperature: 30°C, solvent system: A (MeOH) and B (0.05% of TFA in H₂O), t = 0 min to 1 min: 50 to 60% of B then t = 1 min to t = 10 min: 60 to 100% of B and t = 10 min to t = 15 min: 100% of B.

### A. Preparation of compounds according to the invention

### General synthetic methods and examples deriving from pyridazine-3(2H)-ones

The preparation of compounds of formula (I) can be carried out along various synthetic routes using conventional methods (see Scheme 1). Starting from commercially available or previously described 4 or 5 -halogeno pyridazinones ^{1, 2} of general formula A, a Suzuki-Miyaura cross coupling reaction with aryl boronic acids in presence of tetrakis(triphenylphosphine) palladium led to the corresponding 4 or 5 Aryl pyridazinone derivatives (intermediates B). N-Alkylation of intermediate **B** with appropriate tert-butyl 4-methylsulfonyl oxypiperidine-1-carboxylate (or azepane-1-carboxylate) derivatives of general formula **C,** in an inert solvent such as 2-butanone or DMF, afforded intermediates **D.** The base used can be sodium hydride or alkaline sodium bicarbonate. Deprotection of the protective BOC group and subsequent reductive amination with the appropriate aldehydes in presence of NaBH₃CN gave examples of the present invention (see examples A.1, A.2 and A.5). Direct alkylation of the deprotected piperidine intermediates with appropriate halogenoalkyl derivatives could be performed by methods well-known in the art, as illustrated in scheme 1 (example A.3). Alkylation could also be performed with bromomethylketone derivatives, and the resulting ketones were finally converted to secondary alcohols by treatment with sodium borohydride (example A.4). All the final compounds were isolated as hydrochloride salts, prepared by standard methods from the corresponding bases.
¹ X = 4-Cl, Chin. E., Li. J., Lui. A. S.-T.; Talamas, F. X., WO2010133528; X =4-Br, Aciro. C. et al , WO2010131147
² X= 5-Cl, Lizos. D., Weiler. S., Stiefl. N. J., WO2009013335; X= 5-1, Becknell, N.C. et al, Bioorg. Med. Chem.,2012, 20(12), 3880-3886.

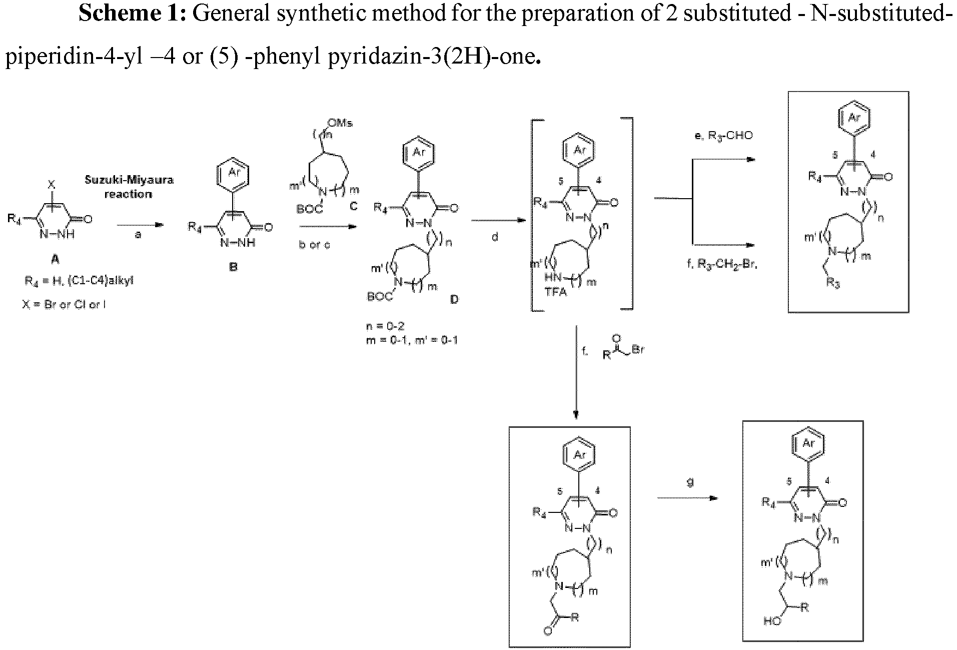

**Conditions:** a) K₂CO₃, Pd(PPh₃)₄, Ar-PhB(OH)₂, Toluene, EtOH, H₂O, 120°C, 16h ; b) n = 0, K₂CO₃, 2-butanone, 85°C, 24h ; c) n = 1, NaH, DMF, 110°C, 18h ; d) TFA, DCM, 25°C, 45 min; e) R₃-CHO, NaBH₃CN; DIEA, MeOH, 25°C, 18h. f ) K₂CO₃, DMF or MeCN, 80°C, 18h. g ) MeOH, NaBH₄, 25°C, 6.5h.

### Example A.1: 2-(1-benzylpiperidin-4-yl)-4-phenylpyridazin-3(2H)-one hydrochloride,(cpd 1).

### Step 1: tert-butyl 4-(methanesulfonyloxy)piperidine-1-carboxylate, intermediate C (m' = m = 0, n = 0).

To a stirring solution of tert-butyl 4-hydroxy-1-piperidinecarboxylate (1.28 g, 5.1mmol) and Et₃N (1.43 mL, 10.22 mmol) in DCM (35 mL) at room temperature was added methanesulfonyl chloride (0.44 mL, 5.62 mmol) dropwise. The reaction mixture was stirred at room temperature for 4 h and washed sequentially with 0.1N aq HCl solution, H₂O and brine. The organic layer was dried over Na₂SO₄ and concentrated in vacuo to yield tert-butyl 4-(methylsulfonyloxy)piperidine-1-carboxylate as a light orange solid, yield (1.4 g). ¹H NMR (400 MHz, CDCl₃) : δ 4.85 - 4.93 (m, 1 H), 3.66 - 3.75 (m, 2 H), 3.25 - 3.35 (m, 2 H), 3.04 (s, 3 H), 1.92 - 2.01 (m, 2 H), 1.77 - 1.86 (m, 2 H), 1.46 (s, 9 H). ¹³C NMR (101 MHz, CDCl₃) : δ 154.7, 80.1, 67.8, 40.5, 34.3, 28.5.

### Step 2: tert-butyl 4-(6-oxo-5-phenylpyridazin-1(6H)-yl)piperidine-1-carboxylate D (m' = m = 0, n = 0)

A 50 ml flask (oven-dried and under argon) was charged with 4-phenyl-2,3-dihydropyridazin-3-one (200 mg, 1.16 mmol, 1.0 equiv. for preparation see example A.2), N-Boc-4-(2-((methylsulfonyl) oxy)ethyl)piperidine (487 mg, 1.74 mmol, 1.5 equiv.) and K₂CO₃ (241.6 mg, 1.74 mmol, 1.5 equiv.). 2-butanone (10 mL) was added and the mixture was heated at 85°C for 48 h (HPLC monitoring: complete conversion). The mixture was quenched with H₂O (150 mL) and extracted with EtOAc (3 x 40 mL). The combined organic layers were washed with brine (2 x 50 mL), dried over Na₂SO₄, filtered and evaporated to dryness. The resulting residue was purified by silica gel flash chromatography using EtOAc /Heptane: 1:2 as eluent to give the title compound as a white solid (260 mg, 63%). ¹H NMR (400 MHz, CDCl₃) : δ 7.85 (d, 1H, J = 4.0 Hz), 7.76 (m, 2H), 7.42 (m, 3H), 7.24 (d, 1H, J = 4.0 Hz), 5.15 (m, 1H), 4.26 (bs, 2H), 2.88 (m, 2H), 2.00-1.86 (m, 4H), 1.46 (s, 9H). ¹³C NMR (101 MHz, CDCl₃) : δ 160.0, 154.9, 139.6, 136.4, 134.3, 129.7, 128.9, 129.6, 127.3, 79.8, 55.7, 43.4, 30.5, 28.7.

### Step 3: 4-phenyl-2-(piperidin-4-yl)pyridazin-3(2H)-one 2,2,2-trifluoroacetate

To an ice-cooled solution of tert-butyl 4-(6-oxo-5-phenylpyridazin-1(6H)-yl)piperidine-1-carboxylate (260 mg, 0.73 mmol, 1 equiv.) in DCM (5 mL) was added TFA (2 mL), and the resulting mixture was stirred for 1 h. The crude reaction was concentrated under vacuum with azeotropic removal of TFA with heptane. The compound was used in the reductive amination step without further purification. ¹H NMR (400 MHz, DMSO-d6) : δ 8.82 (bs, 1H), 8.48 (bs, 1H), 8.08 (d, 1H, J = 4.1 Hz), 7.82 (m, 2H), 7.59 (d, 1H, J = 4.1 Hz), 7.47 (m, 3H), 5.20 (m, 1H), 3.43 (d, 2H, J = 12.1 Hz), 3.16 (q, 2H, J = 11.2 Hz), 2.15-1.99 (m, 4H). ¹³C-NMR (101 MHz, DMSO-d6) : δ 158.7, 137.9, 137.0, 133.9, 129.3, 128.6, 128.2, 128.0, 51.9, 42.6, 27.0.

### Step 4: 2-(1-benzylpiperidin-4-yl)-4-phenylpyridazin-3(2H)-one (cpd 1).

4-phenyl-2-(piperidin-4-yl)pyridazin-3(2H)-one trifluoroacetate (94 mg, 0.368 mmol) was dissolved in MeOH (5 mL). Benzaldehyde (58.6 mg, 56 µL, 1.5 equiv.) was added followed by NaBH₃CN (46.3 mg, 0.74 mmol, 2 equiv.). The resulting mixture was stirred at 25°C for 48h. Volatiles were evaporated and the crude was taken up in EtOAc (25 mL). The organic phase was washed with brine, dried and concentrated under vacuum. The residue was purified by silica gel column chromatography using DCM: MeOH (99: 1) as eluent, to yield 2-(1-benzylpiperidin-4-yl)-4-phenylpyridazin-3(2H)-one **1** as colorless oil (94 mg, 74%). Hydrochloride salt gives a white powder. ¹H NMR (400 MHz, CDCl₃) : δ 7.85 (d, 1H, J = 4.1 Hz), 7.78-7.75 (m, 2H), 7.45-7.39 (m, 3H), 7.37-7.30 (m, 4H), 7.25 (m, 1H), 7.23 (d, 1H, J = 4.1 Hz), 5.04 (m, 1H), 3.55 (s, 2H), 3.02 (m, 2H), 2.23-2.08 (m, 4H), 1.89-1.86 (m, 2H). ¹³C NMR (101 MHz, CDCl₃) : δ 160.1, 139.3, 138.9, 136.2, 134.5, 129.6, 129.2, 128.9, 128.5, 128.4, 127.2, 127.1, 63.1, 56.1, 53.1, 30.7.
LC/MS (M+H) = 346.

### * 2-(1-benzylpiperidin-4-yl)-5-phenylpyridazin-3(2H)-one hydrochloride, (cpd 2)

Using the same procedure described in example A.1 and starting from 5-phenylpyridazin-3(2H)-one³ (250mg, 1.45 mmol, 1 equiv.) and N-Boc-4-(2-((methylsulfonyl)oxy)ethyl)piperidine (279.4 mg, 1.5 equiv) the tert-butyl 4-(6-oxo-4-phenylpyridazin-1(6H)-yl)piperidine-1-carboxylate was obtained in 63 % yield after purification on silica gel with EtOAc/ Hept 1/1. ¹H NMR (200 MHz, CDCl₃) : δ 8.10 (d, 1H, J= 2.3 Hz), 7.60-7.51 (m, 5H), 7.06 (d, 1H, J= 2.3 Hz), 5.15 (m, 1H), 3.91-3.79 (m, 2H), 3.36-3.23 (m, 2H), 2.17-2.05 (m, 2H), 1.89-1.49 (m, 2H), 1.49 (s, 9H).

After deprotection of the tert-butoxycarbonyl group with a suitable acid as trifluoroacetic acid and reductive amination with benzaldehyde the title compound was obtained as colorless oil in 88% yield. Hydrochloride salt gives a white powder. ¹H NMR (400 MHz, DMSO-d6) : δ 10.60 (bs, 1H), 8.46 (d, 1H, J = 2.4 Hz), 7.85-7.83 (m, 2H), 7.63-7.60 (m, 2H), 7.54-7.51 (m, 3H), 7.49-7.47 (m, 3H), 7.24 (d, 1H, J = 2.4 Hz), 5.07 (m, 1H), 4.31 (d, 2H, J = 5.2 Hz), 3.47-3.44 (m, 2H), 3.25-3.17 (m, 2H), 2.35-2.26 (m, 2H), 2.03-2.00 (m, 2H). ¹³C-NMR (101 MHz, DMSO-d6) : δ 159.1, 142.2, 135.9, 133.1, 131.4, 130.3, 129.7, 129.5, 129.2, 128.8, 127.2, 123.4, 59.0, 51.4, 50.4, 27.0.
LC/MS (M+H) = 346.
³ Tetrahedron, 2004, 60(52), 12177-12189.

### * 4-phenyl-2-[1-(2-phenylethyl)piperidin-4-yl]-2,3-dihydropyridazin-3-one hydrochloride (cpd 3).

Using the same procedure described in example A.1 and starting from 4-phenyl-2-(piperidin-4-yl) pyridazin-3(2H)-one 2,2,2-trifluoroacetate (50 mg, 0.13 mmol, 1 equiv) and phenyl acetaldehyde (24.4 mg, 23.7 µl, 0.203 mmol, 1.5 equiv.) the title compound was obtained as a white solid (40 mg, 75%). Hydrochloride salt gives a white powder. ¹H NMR (400 MHz, CDCl₃) : δ 7.80 (d, 1H, J = 4.1 Hz), 7.72-7.70 (m, 2H), 7.39-7.33 (m, 3H), 7.24-7.11 (m, 6H), 4.97 (m, 1H), 3.09 (d, 2H, J= 11.0 Hz), 2.79-2.75 (m, 2H), 2.60-2.56 (m, 2H), 2.22-2.04 (m, 4H), 1.88-1.85 (m, 2H). ¹³C NMR (101 MHz, CDCl₃) : δ 160.2, 140.6, 139.3, 136.2, 134.5, 129.6, 129.0, 128.9, 128.6, 128.5, 127.3, 126.2, 60.6, 56.1, 53.1, 34.1, 30.6. LC/MS (M+H) = 360.

### * 2-[1-(cyclopropylmethyl)piperidin-4-yl]-4-phenyl-2,3-dihydropyridazin-3-one hydrochloride, (cpd 4).

Using the same procedure described in example A.1 and starting from 4-phenyl-2-(piperidin-4-yl) pyridazin-3(2H)-one 2,2,2-trifluoroacetate (50 mg, 0.13 mmol, 1 equiv.) and cyclopropyl carboxaldehyde (14.23 mg, 15. 17µl, 0.203 mmol, 1.5 equiv.), the title compound was obtained as colorless oil (30 mg, 64%). Hydrochloride salt gives a white powder. ¹H NMR (400 MHz, CDCl₃) : δ 7.83 (d, 1H, J = 4.1 Hz), 7.75-7.73 (m, 2H), 7.39-7.35 (m, 3H), 7.21 (d, 1H, J= 4.1 Hz), 4.98 (m, 1H), 3.19 (m, 2H), 2.28 (d, 2H, J= 6.4 Hz), 2.20-2.08 (m, 4H), 1.89-1.87 (m, 2H), 0.87 (hept, 1H, J= 6.4 Hz), 0.52-0.48 (m, 2H), 0.11-0.08 (m, 2H). ¹³C NMR (101 MHz, CDCl₃) : δ 160.1, 139.2, 136.2, 134.4, 129.5, 128.9, 128.5, 127.2, 63.7, 56.0, 30.4, 8.7, 4.1.
LC/MS (M+H) = 310.

### * 2-[1-(cyclopentylmethyl)piperidin-4-yl]-4-phenyl-2,3-dihydropyridazin-3-one hydrochloride (cpd 5).

Using the same procedure described in example A.1 and starting from 4-phenyl-2-(piperidin-4-yl) pyridazin-3(2H)-one 2,2,2-trifluoroacetate (50 mg, 0.13 mmol, 1 equiv) and cyclopentane carbaldehyde (20.5 mg, 22.4 µl, 0.203 mmol, 1.5 equiv.) the title compound was obtained as colorless oil (38 mg, 75 %). Hydrochloride salt gives a white powder. ¹H NMR (400 MHz, CDCl₃) : δ 7.84 (d,1H, J = 4.1 Hz), 7.75-7.73 (m, 2H), 7.40-7.35 (m, 3H), 7.22 (d, 1H, J= 4.1 Hz), 4.99 (m, 1H), 3.05 (m, 2H), 2.30 (d, 2H, J = 7.2 Hz), 2.17-2.01 (m, 4H), 1.87-1.84 (m, 2H), 1.78-1.74 (m, 2H), 1.62-1.29 (m, 5H), 1.22-1.16 (m, 2H). ¹³C NMR (101 MHz, CDCl₃) : δ 160.1, 139.3, 136.2, 134.4, 129.5, 128.9, 128.5, 127.2, 64.5, 56.1, 53.3, 37.7, 31.7, 30.4, 25.4.
LC/MS (M+H) = 338.

### * 2-(1-benzylazepan-4-yl)-4-phenyl-2,3-dihydropyridazin-3-one hydrochloride, (cpd 6), (m' = m = 1, n = 0).

Using the same procedure described in example A.1 and starting from 4-phenyl-2,3-dihydropyridazin-3-one (45 mg, 0.26 mmol, 1 equiv.) and tert-butyl 4-(methanesulfonyloxy)azepane-1-carboxylate ⁴ (92.01 mg, 0.31 mmol, 1.2 equiv.), tert-butyl 4-(6-oxo-5-phenyl-1,6-dihydropyridazin-1-yl)azepane-1-carboxylate was obtained in 49% yield. After deprotection of the tert-butoxycarbonyl group by means of TFA, the resulting trifluoroacetate salt was submitted to reductive amination using NaBH₃CN in presence of benzaldehyde to yield the title compound as a white solid (28.2 mg, 64% yield). ¹H NMR (400 MHz, CDCl₃) : δ 7.70 (d, 1H, J = 4.0 Hz), 7.66-7.59 (m, 2H), 7.32-7.25 (m, 3H), 7.24-7.20 (m, 2H), 7.19-7.14 (m, 2H), 7.11-7.05 (m, 2H), 5.27-5.16 (m, 1H), 3.51 (s, 2H), 2.76-2.64 (m, 1H), 2.59-2.52 (m, 3H), 2.06-1.95 (m, 2H), 1.93-1.85 (m, 2H), 1.80-1.69 (m, 1H), 1.67-1.64 (m, 1H). ¹³C NMR (101 MHz, CDCl₃) : δ ppm 159.5, 139.7, 139.2, 136.0, 134.3, 129.3, 128.8, 128.3, 128.2, 126.9, 126.8, 62.8, 58.2, 55.7, 52.0, 33.7, 32.6, 25.6.
LC/MS (M+H) = 360.
⁴ ACS Medicinal Chemistry Letters, 2016, 7(4), 397-402.

### Example A.2: Preparation of 2-((1-phenethylpiperidin-4-yl)methyl)-4-phenylpyridazin-3(2H)-one hydrochloride (cpd 10)

### Step 1: Tert-butyl 4-[((methylsulfonyl)oxy)methyl]piperidine-1-carboxylate (intermediate C, n =1, m =0, m' =1)

To an ice-cooled solution of tert-butyl 4-(hydroxymethyl)piperidine-1-carboxylate (5g, 23.2 mmol, 1 equiv.) and NEt₃ (3.52 g, 4.86 mL, 34.85 mmol 1.5 equiv.) in DCM (75 mL) was added dropwise mesylchloride (3.99g, 2.70 mL, 34.84 mmol, 1.2 equiv.) and the resulting solution was stirred at rt until complete conversion of the starting material. The reaction conversion was monitored by TLC and HPLC and was usually completed within 3 hours. Volatiles were evaporated under vacuum and the crude was dissolved in EtOAc (100 mL). The organic phase was washed once with 1N K₂CO₃ (60 mL), water (60 mL), brine (60 mL) and dried over (Na₂SO₄), filtered and concentrated under reduce pressure. The resulting oil was taken in a mixture of ice-cooled Et₂O (15 mL) and pentane (10 mL) and sonicated for 2-3 min. The resulting solid was collected by suction filtration on a Büchner funnel, washed with ice-cooled pentane (2 x 5 mL) and dried under vacuum to give the title compound as an off-white solid (4.16 g, 61%). The filtrate was evaporated to approximately half volume to collect more material (2.0 g, total yield: 90%). ¹H NMR (400MHZ, CDCl₃) : δ ppm 4.16-4.12 (m, 2H), 4.06 (d, 2H, J = 6.4 Hz), 3.00 (s, 3H), 2.67 (t, 2H, J = 11.6 Hz), 1.92-1.88 (m , 1H), 1.73 (d, 2H, J = 11.6 Hz), 1.44 (s, 9H), 1.26-1.15 (m, 2H). ¹³C NMR (101 MHZ, CDCl₃) : δ ppm 154.8, 79.7, 73.5, 37.5, 36.1, 28.6, 28.4.

### Step 2: 4-phenyl-2,3-dihydropyridazin-3-one (intermediate B)

A microwave vial (oven-dried and under argon) was charged with phenylboronic acid (2.05 g, 16.85 mmol, 1.1 equiv.), 4-chloro-2,3-dihydropyridazin-3-one (2.0 g, 15.32 mmol, 1.0 equiv.), sodium carbonate (4.88 g, 45.9 mmol, 3 equiv.). Tetrakis(triphenylphosphine) palladium(0) (885 mg, 5 mol %.) was then added, followed by toluene (38 mL), EtOH (8 mL) and H₂O (8 mL). The vial was properly capped and the mixture vessel was evacuated and backfilled with argon (process repeated 3 times) and heated at 120°C until complete conversion of the starting material. The reaction conversion was monitored by HPLC and was usually completed within 16 hours. After cooling to room temperature, the reaction mixture was evaporated to dryness. The crude was partitioned through EtOAc (30 mL) and H₂O (50 mL). The aqueous phase was extracted twice with EtOAc (20 mL). The organic phases were combined, washed with brine and dried over Na₂SO₄ and evaporated. The crude material was flash chromatography using a gradient of 50 to 70% of EtOAc in heptane to give the title compound as an yellow solid (2.34 g, 13.6 mmol, 89%). mp: 217-220°C. ¹H NMR (400MHz, DMSO-d₆) : δ ppm 13.20 (bs, 1H), 7.94 (d, 1H, J = 4.0 Hz), 7.86 (m, 2H), 7.58 (d, 1H, J = 4.0 Hz), 7.51-7.42 (m, 3H). ¹³C NMR (101 MHz, DMSO-d₆): δ ppm 160.9, 138.7, 137.8,134.2, 129.8, 129.2, 128.9, 128.7

### Step 3: tert-butyl 4-((6-oxo-5-phenylpyridazin-1(6H)-yl)methyl)piperidine-1-carboxylate

The reaction was performed in anhydrous conditions under argon atmosphere. To an ice-cooled solution of 4-phenyl-2,3-dihydropyridazin-3-one (450 mg, 2.61 mmol, 1 equiv.) in dry DMF (26.1 mL) was added portionwise sodium hydride (209.1 mg, 5.23 mmol, 2 equiv.) and the mixture was stirred at 0°C for 30 min. Then tert-butyl 4-[(methanesulfonyloxy)methyl]piperidine-1-carboxylate (920.1 mg, 3.14 mmol, 1.2 equiv.) was added portionwise and the mixture was heated at 110°C overnight (HPLC monitoring: complete conversion). The mixture was quenched with H₂O (150 mL) and extracted with EtOAc (3 x 40 mL). The combined organic layers were washed with brine (2 x 50 mL), dried over Na₂SO₄, filtered and evaporated to dryness. The resulting residue was purified by silica gel flash chromatography using a gradient of 10 to 50% of EtOAc in heptane to give the title compound as a white solid (835.6 mg, 2.26 mmol, 87%). ¹H NMR (400 MHz, CDCl₃) : δ ppm 7.84-7.78 (m, 3H), 7.45-7.43 (m, 3H), 7.29 (d, 1H, J = 4.0 Hz), 4.15-4.13 (m, 4H), 2.69 (t, 2 H, J = 12.4 Hz), 2.21-2.19 (m, 1H), 1.65 (d, 2H, J = 12.4 Hz), 1.45 (s, 9H), 1.32-1.24 (m, 2H). ¹³C NMR (101 MHz, CDCl₃) : δ ppm 160.3, 154.8, 139.8, 136.1, 133.9, 129.6, 128.7, 128.4, 127,4, 79.3, 60.5, 57.7, 35.4, 29.7, 28.4

### Step 4: 2-((1-phenethylpiperidin-4-yl)methyl)-4-phenylpyridazin-3(2H)-one (cpd 10)

To a solution of tert-butyl-4-[(6-oxo-5-phenyl-1,6-dihydropyridazin-1-yl)methyl]piperidine-1-carboxylate (110 mg, 0.3 mmol, 1 equiv.) in DCM (5 mL) at 0 °C was added TFA (0.45 mL), and the resulting mixture was stirred at rt for 1 h. The crude reaction was concentrated under vacuum with azeotropic removal of TFA with heptane. The residue was triturated with ice-cooled ether, the supernatant was removed and the crude after evaporation was directly dissolved in MeOH (5 mL). 4-phenyl-2-(piperidin-4-yl methyl) pyridazin-3(2H)-one 2,2,2-trifluoroacetate (150 mg, 0.30 mmol, 1 equiv.) and phenyl acetaldehyde (43.5 mg, 42.3 µl, 0.36 mmol, 1.2 equiv.) were added, followed by DIEA (156 mg, 1.20 mmol, 0.20 ml) and NaBH₃CN (37.9 mg, 0.6 mmol, 2 equiv.). The resulting mixture was stirred at rt for 48h. Volatiles were evaporated and the crude was taken up in EtOAc (25 mL). The organic phase was washed with brine, dried and concentrated under vacuum. The residue was purified by silica gel column chromatography using DCM : MeOH (98: 2) as eluent to yield 2-((1-phenethylpiperidin-4-yl)methyl)-4-phenylpyridazin-3(2H)-one as a colorless gum (79.2 mg, 64%). %). ¹H NMR (400 MHz, CDCl₃) : δ ppm 7.84-7.77 (m, 3H), 7.48-7.40 (m, 3H), 7.30-7.26 (m, 3H), 7.21-7.16 (m, 3H), 4.17 (d, 2H, J = 6.8 Hz), 3.01(d, 2H, J = 12.8 Hz), 2.81(m, 2H), 2.59 (m, 2H), 2.07-2.01 (m, 3H), 1.72 (d, 2H, J = 12.8 Hz), 1.49 (q, 2H, J = 12.8 Hz). ¹³C NMR (101 MHz, CDCl₃) : δ ppm 160.4, 140.5, 139.9, 136.1, 134.1, 129.7, 128.9, 128.8, 128.5, 127.5, 126.1, 60.9, 58.0, 53.4, 35.3, 33.8, 30.0.

### Formation of the hydrochloride salt

60 mg of the above compound was dissolved in a minimum of methanol and the solution was treated with an excess of 2N HCl in Et₂O. After stirring for 15 min, the resulting mixture was concentrated under vacuum, coevaporated twice with Et₂O, and then triturated with ice-cooled pentane. The supernatant was removed and the residue was dried under vacuum to yield (79.2 mg of the title compound as a white solid). LC/MS (M+H) = 374.

### * 2-((1-benzylpiperidin-4-yl)methyl)-6-methyl-4-phenylpyridazin-3(2H)-one, (cpd 7).

Using the same procedure described in example A.2 and starting from 6-methyl-4-phenyl-2,3-dihydropyridazin-3-one⁵ (30 mg, 0.161 mmol, 1 equiv.) and tert-butyl 4-[(methylsulfonyl)oxy)methyl]piperidine-1-carboxylate (53.62 mg, 1.2 equiv.) the tert-butyl 4-[(3-methyl-6-oxo-5-phenyl-1,6-dihydropyridazin-1-yl)methyl]piperidine-1-carboxylate in 82 % yield ( after purification on silica gel AcOEt/ Hept 1/3. ¹H NMR (400 MHz, CDCl₃) : δ 7.77-7.72 (m, 2H), 7.42-7.35 (m, 3H), 7.16 (s, 1H), 4.12-3.99 (m, 4H), 2.82 (t, 2H, J = 11.6 Hz), 2.33 (s, 3H), 2.20-2.08 (m, 1H), 1.61 (d, 2H, J = 12.3 Hz), 1.41 (s, 9H), 1.25 (qd, 2H, J = 12.4 Hz, J = 5.2 Hz). ¹³C NMR (101 MHz, CDCl₃) : δ 159.6, 154.9, 144.3, 139.6, 134.2, 129.7, 129.6, 128.9, 128.5, 79.5, 57.5, 44.0, 43.83, 35.6, 29.9, 28.6, 21.2.

Deprotection of the tert-butyloxycarbonyl group with TFA followed by reductive amination using benzaldehyde and NaBH₃CN as described for example A.2 led to the title compound as colorless oil (33.4 mg, 62%). Hydrochloride salt gives a white powder. ¹H NMR (400 MHz, MeOD) : δ 7.76-7.71 (m, 2H), 7.47-7.39 (m, 4H), 7.48-7.45 (m, 3H), 7.34-7.31 (m, 4H), 7.30-7.20 (m, 1H), 4.08 (d, 2H, J= 7.2 Hz), 3.57 (s, 2H), 3.31 (qt, 1H, J = 1.7 Hz), 2.94 (dt, 2H, J= 11.8, J = 3.4 Hz), 2.36 (s, 3H), 2.09 (td, 2H, J = 11.5 Hz, J = 2.0 Hz), 2.04-1.94 (m, 1H), 1.66 (d, 2H, J= 12.3 Hz), 1.43 (qd, J = 12.6 Hz, J = 4.0 Hz, 2H). ¹³C NMR (101 MHz, CDCl₃) : δ 161.0, 1435, 138.7, 135.6, 134.2, 130.4, 129.6, 129.3, 128.3, 127.1, 127.0, 124.8, 63.5, 57.1, 53.4, 35.5, 30.1.
LC/MS (M+H) = 374.22
^{5a} Chin. J. Org. Chem., 2014, 34, 722-728; ^{5b} Pest Manag Sci., 2006, 62, 522-530.

### * 2-((1-benzylpiperidin-4-yl)methyl)-5-phenylpyridazin-3(2H)-one hydrochloride, (cpd 8).

Using the same procedure described in example A.2 and starting from 5-phenylpyridazin-3(2H)-one (25 mg, 0.145 mmol, 1 equiv.) and tert-butyl 4-[(methylsulfonyl)oxy)methyl]piperidine-1-carboxylate (51.12 mg, 1.2 equiv.) the tert-butyl 4-(6-oxo-4-phenylpyridazin-1(6H)-yl)piperidine-1-carboxylate was obtained in 72 % yield after purification on silica gel AcOEt/ Hept 1/3. ¹H NMR (400 MHz, CDCl₃) : δ 8.01 (d, 1H, J = 2.2 Hz), 7.55-7.53 (m, 2H), 7.46-7.44 (m, 3H), 7.01 (d, 1H, J = 2.2 Hz), 4.06-4.04 (m, 4H), 2.66 (t, 2H, J = 11.7 Hz), 2.13 (m, 1H), 1.62-1.59 (m, 2H), 1.41 (s, 9H), 1.26-1.23 (m, 2H).

Deprotection of the tert-butyloxycarbonyl group with TFA followed by reductive amination using benzaldehyde and NaBH₃CN as described for example A.2 led to the title compound as colorless oil (16 mg, 39%). Hydrochloride salt gives a white powder. ¹H NMR (400 MHz, CDCl₃) : δ 7.99 (d, 1H, J= 2.2 Hz), 7.55-7.53 (m, 2H), 7.48-7.45 (m, 3H), 7.31-7.21 (m, 5H), 7.03 (d, 1H, J= 2.2 Hz), 4.09 (d, 2H, J= 7.0 Hz), 3.47 (s, 2H), 2.87 (t, 2H, J= 11.7 Hz), 2.03-1.92 (m, 3H), 1.63 (d, 2H, J= 12.8 Hz), 1.48-1.38 (m, 2H). ¹³C NMR (101 MHz, CDCl₃) : δ 161.0, 1435, 138.7, 135.6, 134.2, 130.4, 129.6, 129.3, 128.3, 127.1, 127.0, 124.8, 63.5, 57.1, 53.4, 35.5, 30.1.
LC/MS (M+H) = 360.

### * 2-[(1-benzylpiperidin-3-yl)methyl]-4-phenyl-2,3-dihydropyridazin-3-one hydrochloride (cpd 9)

Using the same procedure described in example A.2 and starting from 4-phenyl-2,3-dihydropyridazin-3-one (45 mg, 0.26 mmol, 1 equiv.) and tert-butyl 3-[(methanesulfonyloxy)methyl]piperidine-1-carboxylate (92.01 mg, 0.31 mmol, 1.2 equiv.) the tert-butyl 3-[(6-oxo-5-phenyl-1,6-dihydropyridazin-1-yl)methyl]piperidine-1-carboxylate was obtained as a colorless oil (63.3 mg, 66%). ¹H NMR (400 MHz, CDCl₃) : δ ppm 7.70-7.50 (m, 3H), 7.32-7.21 (m, 3H), 7.11 (d, 1H, 4.0Hz), 4.13-3.83 (m, 2H), 3.73 (d, 2H, J= 13.3 Hz), 2.67 (t, 1H, J = 12.7 Hz), 2.67-2.48 (m, 1H), 2.10-1.97 (m, 1H), 1.71-1.47 (m, 2H), 1.35-1.27 (m, 1H), 1.25 (s, 9H), 1.14-1.02 (m, 1H) ¹³C NMR (101 MHz, CDCl₃) : δ ppm 160.2, 154.7, 139.7, 136.1, 133.9, 129.5, 128.7, 128.4, 127.4, 79.3, 55.08, 42.1, 35.5, 28.4, 28.1, 24.4

Deprotection of the tert-butyloxycarbonyl group with the help of TFA followed by reductive amination using benzaldehyde and NaBH₃CN as described for example A.2, led to the title compound as a white solid (29.7 mg, 47%). Tr (HPLC) = 7.06 min.. ¹H NMR (400 MHz, CDCl₃) : δ ppm 7.78-7.70 (m, 3H), 7.45-7.36 (m, 3H), 7.30- 7.15 (m, 6H), 4.19 (dd, 1H, J = 12.9 Hz, 7.2 Hz), 4.07 (dd, 1H, J = 12.9 Hz, J = 7.2 Hz), 3.50 (d, 1H, J = 12.9 Hz), 3.39 (d, 1H, J = 12.9 Hz), 2.66 (d, 1H, J = 10.8 Hz), 2.31 (d, 1H, J = 10.8 Hz), 2.36-2.25 (m, 1H), 2.05-1.92 (m, 2H), 1.67 (d, 2H, J = 10.2 Hz), 1.58-1.45 (m, 1H), 1.18-1.08 (m, 1H). ¹³C NMR (101 MHz, CDCl₃) : δ ppm 160.2, 139.6, 138.7 135.9, 134.1, 129.5, 129.0, 128.7, 128.3, 128.1, 127.3, 126.8, 63.5, 57.9, 55.7, 53.9, 35.9, 28.3, 24.6.
LC/MS (M+H) = 360.

### * 4-phenyl-2-((1-propylpiperidin-4-yl)methyl)pyridazin-3(2H)-one hydrochloride (cpd 11)

Using the same procedure described in example A.2 and starting from 4-phenyl-2-(piperidin-4-yl methyl) pyridazin-3(2H)-one 2,2,2-trifluoroacetate (178.6 mg, 0.39 mmol, 1 equiv.) and propanal (27.3 mg, 34.2 µl, 0.46 mmol, 1.2 equiv.) the title compound was obtained as a white solid (115.8 mg, 85%). ¹H NMR (400 MHz, CDCh) : δ ppm 7.83-7.76 (m, 3H), 7.46-7.39 (m, 3H), 7.27 (d, 1H, J = 4.0 Hz), 4.15 (d, 2H, J = 6.8 Hz), 2.96 (d, 2H, J = 11.6 Hz), 2.31 (t, 2H, J= 8.0 Hz), 2.09-2.01 (m, 1H), 1.96 (t, 2H, J = 11.6Hz), 1.72-1.66 (m, 2H), 1.57-1.42 (m, 4H), 0.88 (t, 3H, J = 7.6Hz). ¹³C NMR (101 MHz, CDCl₃) : δ ppm 160.3, 139.7, 136.0, 134.0, 129.5, 128.7, 128.4, 127.4, 60.8, 57.8, 53.1, 35.1, 29.7, 18.9, 11.7. LC/MS (M+H) = 312.

### * 2-((1-(4-chlorobenzyl)piperidin-4-yl)methyl)-4-phenylpyridazin-3(2H)-one hydrochloride (cpd 12)

Using the same procedure described in example A.2 and starting from 4-phenyl-2-(piperidin-4-yl methyl) pyridazin-3(2H)-one 2,2,2-trifluoroacetate (150 mg, 0.30 mmol, 1 equiv.) and 4-chlorobenzaldehyde (50.9 mg, 0.36 mmol, 1.2 equiv.) the title compound was obtained as a pale yellow solid (36.7 mg, 28%). ¹H NMR (400 MHz, CDCl₃) : δ ppm 7.82-7.76 (m, 3H), 7.46-7.39 (m, 3H), 7.28-7.21 (m, 5H), 4.15 (d, 2H, J = 6.8 Hz), 3.44 (s, 2H), 2.84 (d, 2H, J = 11.2 Hz), 2.09-2.01 (m, 1H), 1.96 (t, 2H, J = 11.2 Hz), 1.65 (d, 2H J= 12.8 Hz), 1.50-1.37 (m, 2H). ¹³C NMR (101 MHz, CDCl₃) : δ ppm 160.4, 139.8, 137.2, 136.1, 134.1, 132.7, 130.5, 129.7, 128.8, 128.5, 128.4, 127.4, 62.6, 58.0, 53.3, 35.2, 30.0.
LC/MS (M+H) = 394.

### * 2-((1-(cyclohexylmethyl)piperidin-4-yl)methyl)-4-phenylpyridazin-3(2H)-one hydrochloride, (cpd 13)

Using the same procedure described in example A.2 and starting from 4-phenyl-2-(piperidin-4-yl methyl) pyridazin-3(2H)-one 2,2,2-trifluoroacetate (178.6 mg, 0.39 mmol, 1 equiv.) and cyclohexane carboxaldehyde (52.9 mg, 0.36 mmol, 1.2 equiv.) the title compound was obtained in 60% yield. ¹H NMR (400 MHz, CDCl₃) : δ ppm 7.82-7.76 (m, 3H), 7.46-7.38 (m, 3H), 7.26 (d, 1H, J = 4.0 Hz), 4.13 (d, 2H, J = 6.8 Hz), 2.83 (d, 2H, J = 10.8 Hz), 2.08 (d, 2H, J = 6.8 Hz), 2.03-2.00 (m, 1H), 1.86 (t, 2H, J = 11.6 Hz), 1.78-1.60 (m, 7H), 1.50-1.38 (m, 3H), 1.25-1.11 (m, 3H), 0.91-0.77 (m, 2H).¹³CNMR (101 MHz, CDCl₃) : δ ppm 160.4, 139.8, 136.0, 134.2, 129.6, 128.8, 126.5, 127.4, 66.1, 58.1, 54.0, 35.4, 32.2, 30.0, 26.9, 26.3. LC/MS (M+H) = 366.

### * 4-phenyl-2-((1-(pyridin-4-ylmethyl)piperidin-4-yl)methyl)pyridazin-3(2H)-one hydrochloride, (cpd 14)

Using the same procedure described in example A.2 and starting from 4-phenyl-2-(piperidin-4-yl methyl) pyridazin-3(2H)-one 2,2,2-trifluoroacetate (150 mg, 0.30 mmol, 1 equiv.) and 4-pyridine- carboxaldehyde (38.8 mg, 34.1 µl, 0.36 mmol, 1.2 equiv.) the title compound was obtained as a beige solid (11.4 mg, 10%). ¹H NMR (400 MHz, CDCl₃) : δ ppm 8.55-8.49 (m, 2H), 7.83-7.77 (m, 3H), 7.46-7.40 (m, 3H), 7.30-7.23 (m, 3H). 4.16 (d, 2H, J = 6.8 Hz), 3.47 (s, 2H), 2.83 (d, 2H, J = 11.2 Hz). 2.00 (t, 2H, J = 11.6 Hz), 1.91-1.76 (m, 1H), 1.66 (d, 2H, J = 12.0 Hz), 1.51-1.45 (m, 2H). ¹³C NMR (101 MHz, CDCl₃) : δ ppm 154.9, 149.8, 148.2, 139.8, 136.1, 134.1, 129.7, 128.9, 128.5, 127.5, 123.9. 62.1, 57.9, 53.5, 35.1, 30.0.
LC/MS (M+H) = 362

### * 4-phenyl-2-((1-((tetrahydro-2H-pyran-4-yl)methyl)piperidin-4-yl)methyl)pyridazin-3(2H)-one dihydrochloride, (cpd 15)

Using the same procedure described in example A.2 and starting from 4-phenyl-2-(piperidin-4-yl methyl) pyridazin-3(2H)-one 2,2,2-trifluoroacetate (178.6 mg, 0.30 mmol, 1 equiv.) and oxane-4-carbaldehyde (53.7 mg, 48.8 µl, 0.47 mmol, 1.2 equiv.) the title compound was obtained in 76% yield (118.7 mg). ¹H NMR (400 MHz, DMSO-d6) : δ ppm10.1 (s, broad, 1H), 8.00 (d, 1H, J = 4.0 Hz), 7.86-7.80 (m, 2H), 7.60 (d, 1H, J = 4.0 Hz), 7.49-7.42 (m, 3H), 4.06 (d, 2H, J = 6.8 Hz), 3.83 (d, 2H, J = 10.8 Hz), 3.48-3.43 (m, 4H), 3.28 (t, 2H, J = 11.6 Hz), 2.89-2.84 (m, 4H), 2.22-2.11 (m, 1H), 2.09-2.00 (m, 1H), 1.82-1.70 (m, 4H), 1.23-1.19 (m, 2H). ¹³C NMR (101 MHz, DMSO-d6) : δ ppm 158.8, 137.4, 136.2, 133.3, 128.8, 128.0, 127.7, 127.6, 65.7, 60.9, 55.2, 51.4, 32.2, 30.1, 29.0, 25.7.
LC/MS (M+H) = 368.

### * 2-{[1-(1H-imidazol-5-ylmethyl)piperidin-4-yl]methyl}-4-phenyl-2,3-dihydropyridazin-3-one dihydro- chloride, (cpd 16).

Using the same procedure described in example A.2 and starting from 4-phenyl-2-(piperidin-4-yl methyl) pyridazin-3(2H)-one 2,2,2-trifluoroacetate (250 mg, 0.59 mmol, 1 equiv) and 4- 1H-imidazole-5-carbaldehyde (67.7 mg, 0.70 mmol, 1.2 equiv.) the title compound was obtained as a white solid (20.9 mg, 8%). ¹H NMR (400 MHz, CDCl₃) : δ ppm 7.65 (d, 1H, J = 4.1Hz), 7.63-7.58 (m, 2H), 7.37 (s, 1H), 7.31-7.23 (m, 3H), 7.11 (d, 1H , J = 4.1Hz), 6.71 (s, 1H), 3.98 (d, 2H, J = 6.9 Hz), 3.34 (s, 2H), 2.74 (dt, 2H, J = 11.7 Hz, J = 3.7 Hz), 1.83 (dt, 2H, J = 11.7 Hz, J = 1.7 Hz), 1.57 (d, 2H, J =12.9 Hz), 1.25 (dq, 2H, J = 12.0, J = 3.2Hz), 1.12-1.07 (m, 1H). ¹³ C NMR (101 MHz, CDCl₃) : δ ppm 160.3, 139.7, 136.1, 135.1133.9, 129.6, 128.7, 128.4, 127.5, 57.7, 54.1, 53.0, 35.1, 29.8.
LC/MS (M+H) = 350.

### * 4-phenyl-2-((1-((tetrahydro-2H-pyran-3-yl)methyl)piperidin-4-yl)methyl)pyridazin-3(2H)-one hydrochloride, (cpd 17).

Using the same procedure described in example A.2 and starting from 4-phenyl-2-(piperidin-4-yl methyl) pyridazin-3(2H)-one 2,2,2-trifluoroacetate (125 mg, 0.32 mmol, 1 equiv.) and oxane-3-carbaldehyde (55.8 mg, 0.49 mmol, 1.2 equiv.) the title compound was obtained in 58% yield (69 mg).¹H NMR (400 MHz, CDCl₃) : δ ppm 7.81 (d, 1H, J = 4.2 Hz), 7.80-7.77 (m, 2H), 7.46-7.40 (m, 3H), 7.28 (d, 1H, J = 4.2 Hz), 4.13 (d, 2H, J = 7.2 Hz), 3.93 (ddd, 1H, J =10.6 Hz, J = 5.3 Hz, J = 2.6 Hz), 3.85 (dt, 1H, J = 12.5 Hz, J = 4.1 Hz), 3.40-3.33 (m, 1H), 3.08 (dd, 1H, J = 8.4 Hz, J = 12.2 Hz), 2.92-2.85 (m, 1H), 2.82-2.74 (m, 1H), 2.15-1.95 (m, 3H), 1.94-1.77 (m, 4H), 1.671.55 (m, 4H), 1.48-1.34 (m, 2H), 1.20-1.11 (m, 1H). ¹³CNMR (101 MHz, CDCl₃) : δ ppm 160.3, 139.7, 135.9, 134.0, 129.5, 128.7, 128.4, 127.3, 72.5, 68.6, 61.4, 58.0, 54.2, 53.5, 35.2, 33.6, 2930.0, 29.9, 28.6, 25.6.
LC/MS (M+H) = 368.

### * 2-((1-(4-hydroxybenzyl)piperidin-4-yl)methyl)-4-phenylpyridazin-3(2H)-one hydrochloride, (cpd 18).

Using the same procedure described in example A.2 and starting from 4-phenyl-2-(piperidin-4-yl methyl)pyridazin-3(2H)-one 2,2,2-trifluoroacetate (131 mg, 0.34 mmol, 1 equiv.) and para-hydroxy-benzaldehyde (62.6 mg, 0.51 mmol, 1.5 equiv.), the title compound was obtained in 51% yield (66 mg). ¹H NMR (400MHz, CDCl₃) : δ ppm 7.80 (d, 1H, J = 4.2 Hz), 7.79-7.75 (m, 2H), 7.46-7.38 (m, 3H), 7.27 (d, 1H, J = 4.2 Hz), 7.08 (d, 2H, J = 8.6 Hz), 6.64 (d, 1H, J = 8.6 Hz), 4.14 (d, 2H, J = 7.2 Hz), 3.46 (s, 2H), 2.97 (dt, 2H, J =11.5 Hz, J = 5.1 Hz), 2.12-1.97 (m, 3H), 1.72-1.63 (m, 2H), 1.5(qd, 2H, J= 11.5Hz, J = 5.1 Hz). ¹³C NMR (101 MHz, CDCl₃) : δ ppm 160.3, 155.8, 155.7, 139.7, 136.0, 133.9, 130.9, 129.6, 128.7, 128.4, 127.5, 115.4, 62.4, 57.8, 52.7, 34.8, 29.2.
LC/MS (M+H) = 376.

### Example A.3: 2-((1-(2-methoxyethyl)piperidin-4-yl)methyl)-4-phenylpyridazin-3(2H)-one hydrochloride, (cpd 19).

To a solution of tert-butyl-4-[(6-oxo-5-phenyl-1,6-dihydropyridazin-1-yl)methyl]piperidine-1-carboxylate (100 mg, 0.27 mmol, 1 equiv.) in DCM (1.5 mL) at 0 °C was added TFA (0.41 mL), and the resulting mixture was stirred at rt for 1 h. The crude reaction was concentrated under vacuum with azeotropic removal of TFA with heptane. The residue was triturated with ice-cooled ether, the supernatant was removed and the crude after evaporation was directly dissolved in DMF (1.0 mL). 1-bromo-2-methoxyethane (44.8 mg, 30.7 µL, 1.2 equiv.) was added, followed by K₂CO₃ (148.5 mg, 1.07 mmol, 4 equiv.). The resulting mixture was refluxed for 18h. Volatiles were evaporated and the crude was taken up in EtOAc (25 mL). The organic phase was washed with brine, dried and concentrated under vacuum. The residue was purified by silica gel column chromatography using EtOAc/MeOH/NH₄OH 95/5/0.5 as eluent, to yield the title compound as an off-white solid (67%) after salification with 2M HCl in Et₂O. ¹H NMR (400 MHz, CDCl₃) : δ ppm 7.80 (d, 1H, J = 4.0 Hz), 7.79- 775 (m, 2H), 7.45-7.39 (m, 3H), 7.26 (d, 1H, J= 4.0 Hz), 4.13 (d, 2H, J= 6.8 Hz), 3.47 (t, 2H, J = 5.6 Hz), 3.33 (s, 3H), 2.97-2.91 (m, 2H), 2.54 (t, 2H, J = 5.6 Hz), 2.07-1.95 (m, 3H), 1.68-1.65 (m, 2H), 1.53-1.43 (m, 2H).
LC/MS (M+H) = 328.

### * 2-((1-(2-hydroxyethyl)piperidin-4-yl)methyl)-4-phenylpyridazin-3(2H)-one hydrochloride, (cpd 20).

Using the same procedure described in example A.3 and starting from 4-phenyl-2-(piperidin-4-ylmethyl)pyridazin-3(2H)-one 2,2,2-trifluoroacetate (178.6 mg, 0.39 mmol, 1 equiv.) and 2-chloroethanol (37.8 mg, 31.5 µl, 0.47 mmol, 1.2 equiv.) the title compound was obtained in 79% yield. ¹H NMR (400 MHz, DMSO-d6) : δ 10.30 (bs, 1H), 8.01 (d, 1H, J= 4.3 Hz), 7.84-7.82 (m, 2H), 7.60 (d, 1H, J= 4.3 Hz), 7.47-7.44 (m, 3H), 5.33 (t, 1H, J= 5.0 Hz), 4.05 (d, 2H, J= 6.9 Hz), 3.76 (q, 2H, J= 5.0 Hz), 3.29-3.23 (m, 2H), 3.07 (q, 2H, J= 5.0 Hz), 2.97-2.88 (m, 2H), 2.19-2.12 (m, 1H), 1.76-1.65 (m, 4H). ¹³C-NMR (101 MHz, DMSO-d6) : δ 159.4, 138.0, 136.9, 133.9, 129.4, 128.7, 128.3, 128.2, 58.3, 55.8, 55.1, 51.8, 32.8, 26.5. LC/MS (M+H) = 314.

### * 2-((1-(2-methoxy-1-phenylethyl)piperidin-4-yl)methyl)-4-phenylpyridazin-3(2H)-one hydrochloride, (cpd 21)

Using the same procedure described in example A.3 and starting from 4-phenyl-2-(piperidin-4-ylmethyl)pyridazin-3(2H)-one 2,2,2-trifluoroacetate (160 mg, 0.42 mmol, 1 equiv.) and 1-bromo-2-methoxyethyl)benzene ⁵ (107.7 mg, 0.50 mmol, 1.2 equiv.) the title compound was obtained in 28% yield. ¹H NMR (400 MHz, CDCl₃) : δ ppm 7.81-7.74 (m, 3H), 7.47-7.37 (m, 3H), 7.33-7.21 (m, 6H), 3.813.71 (m, 1H), 3.67-3.62 (m, 1H), 3.59-3.52 (m, 1H), 3.30 (s, 3H), 3.09-2.99 (m, 1H), 2.85-2.74 (m, 1H), 2.12-2.05 (m, 1H)1.98-1.87 (m, 2H), 1.72-1.33 (m, 4H). ¹³C NMR (101 MHz, CDCl₃) : δ ppm 160.2, 139.7, 135.9, 134.0, 129.5, 128.7, 128.4, 128.3, 128.0, 127.3, 74.1, 69.2, 58.7, 57.9, 51.1, 50.3, 35.7, 30.0.
LC/MS (M+H) = 314.
⁵ Neuss J.C., Orchard M. G.; Scopes, D. I. C., WO 2003070239

### * N-(2-{4-[(6-oxo-5-phenyl-1,6-dihydropyridazin-1-yl)methyl]piperidin-1-yl}ethyl)acetamide hydrochloride, (cpd 22).

Using the same procedure described in example A.3 and starting from 4-phenyl-2-(piperidin-4-ylmethyl)pyridazin-3(2H)-one 2,2,2-trifluoroacetate (60 mg, 0.16 mmol, 1 equiv.) and N-(2-chloroethyl)acetamide (19 mg, 0.16 mmol, 1.0 equiv.) the title compound was obtained in 34% yield (19 mg). ¹H NMR (400 MHz, CDCl₃) : δ ppm 7.79 (d, 1H, J = 4.0 Hz), 7.77-7.74 (m, 2H), 7.42-7.39 (m, 3H), 7.26 (d, 1H, J = 4.0 Hz), 6.44 (bs, 1H) , 4.13 (d, 2H, J = 7.2 Hz), 3.34 (dd, 2H, J = 10.6 Hz, J = 5.9 Hz), 2.95 (d, 2H, J = 11.4 Hz), 2.53 (t, 2H, J = 5.8 Hz), 2.09 (m, 3H), 1.95 (s, 3H), 1.7 (d, 2H, J = 11.8 Hz), 1.52-1.44 (m, 2H). ¹³C NMR (101 MHz, CDCl₃) : δ ppm 170.4, 160.3, 139.7, 136.2, 133.8, 129.6, 128.7, 128.4, 127.5, 57.4, 56.7, 53.0, 35.8, 34.7, 29.9, 23.2.
LC/MS (M+H) = 355.

### Example A.4: 2-((1-(2-(4-fluorophenyl)-2-hydroxyethyl)piperidin-4-yl)methyl)-4-phenylpyridazin-3(2H)-one hydrochloride, (cpd 23).

### Step 1: 2-((1-(2-(4-fluorophenyl)-2-oxoethyl)piperidin-4-yl)methyl)-4-phenylpyridazin-3(2H)-one hydrochloride (cpd 24).

To a solution of 4-phenyl-2-(piperidin-4-ylmethyl)pyridazin-3(2H)-one 2,2,2-trifluoroacetate (350 mg, mg, 0.82 mmol, 1 equiv.) in MeCN (6.3 mL) was added 2-chloro-4'-fluoroacetophenone (170 mg, 0.99 mmol, 1.2 equiv.) followed by K₂CO₃ (454.2 mg, 3.29 mmol, 4 equiv.). The resulting mixture was refluxed for 17h. Volatiles were evaporated and the crude was taken up in EtOAc (25 mL). The organic phase was washed with brine, dried and concentrated under vacuum. The residue was purified by reverse phase chromatography using MeOH/H₂O +0.05% TFA. The resulting product was taken in EtOAc and washed with a saturated solution of NaHCOs. After evaporation, the resulting oil (170 mg) was dissolved in a minimum of methanol, and the solution was treated with an excess of 2N HCl in Et₂O. After stirring for 15 min the resulting mixture was concentrated under vacuum, coevaporated twice with Et₂O and then triturated with ice-cooled pentane. The supernatant was removed and the residue was dried under vacuum, to yield the title compound in 53% yield. ¹H NMR (400 MHz, CDCl₃) : δ ppm 7.89 (dd, 2H, J = 8.9 Hz, J = 5.3 Hz), 7.84 (d, 1H, J = 4.2 Hz), 7.75-7.69 (m, 2H), 7.46-7.36 (m, 3H), 7.30 (d, 1H, J = 4.2 Hz), 7.13 (t, 2H, J = 8.5 Hz), 4.62 (s, 2H), 4.32-4.15 (m, 2H), 3.79-3.65 (m, 2H), 3.23-3.06 (m, 2H), 2.45-2.30 (m, 1H), 2.01-1.87 (m, 4H). ¹³C NMR (101 MHz, CDCl₃) : δ ppm 195.4, 167.0, 164.5, 160.3, 139.7, 136.0, 133.9, 132.5, 131.1, 130.9, 129.5, 128.7, 128.4, 127.4, 115.6, 115.4, 65.1, 57.8, 53.6, 34.7, 29.7. ¹⁹F NMR (376 MHz, CDCl₃) : δ ppm -104.9.
LC/MS (M+H) = 406.

### Step 2: 2-((1-(2-(4-fluorophenyl)-2-hydroxyethyl)piperidin-4-yl)methyl)-4-phenylpyridazin-3(2H)-one hydrochloride, (cpd 23).

To a solution of 2-((1-(2-(4-fluorophenyl)-2-oxoethyl)piperidin-4-yl)methyl)-4-phenylpyridazin-3(2H)-one (112 mg, 0.28 mmol, 1 equiv.) in MeOH (3 mL) was added NaBH₄ (21.1 mg, 0.55 mmol, 2 equiv.) and the resulting mixture was stirred for 6.5 h. H₂O (0.8 mL) was then added and the volatiles were evaporated. The crude was purified by reverse phase chromatography using MeOH/H₂O +0.05% TFA The resulting product was taken in EtOAc and washed with a saturated solution of NaHCOs. After evaporation, the resulting oil was dissolved in a minimum of methanol and the solution was treated with an excess of 2N HCl in Et₂O. After stirring for 15 min the resulting mixture was concentrated under vacuum, coevaporated twice with Et₂O and then triturated with ice-cooled pentane. The supernatant was removed and the residue was dried under vacuum to yield the title compound as a white solid (36 mg, 32%). ¹H NMR (400 MHz, CDCl₃) : δ ppm 7.66 (d, 1H, J = 4.0 Hz), 7.63 (dd, J = 7.2 Hz, J = 2.3 Hz), 4.51 (dd, 1H, J= 10.8 Hz, J = 3.2 Hz), 4.0 (d, 2H, J = 7.1 Hz), 2.97 (d, 1H, J = 12.0 Hz), 2.64 (d, 1H , J = 11.6 Hz), 2.30 (dd, 1H, J = 12.3 Hz, J = 3.6 Hz), 2.23 (d, 1H, J = 10.6 Hz), 2.14 (dt 1H, J= 11.3 Hz, J= 2.8 Hz), 1.99-1.81(m, 2H), 1.61-1.48 (m, 2H), 1.39-1.23 (m, 2H). ¹³C NMR (101 MHz, CDCl₃) : δ ppm 163.4, 160.9, 160.3, 139.7, 138.0, 137.9, 136.0, 133.9, 129.6, 128.7, 128.4, 128.3, 127.5, 127.4, 127.3, 115.2, 115.0, 67.9, 66.3, 57.8, 54.8, 51.6, 34.9, 30.1, 29.8. ¹⁹F NMR (376 MHz, CDCl₃) : δ ppm -115.5.
LC/MS (M+H) = 408

### * 2-((1-(2-hydroxy-2-phenylethyl)piperidin-4-yl)methyl)-4-phenylpyridazin-3(2H)-one hydrochloride, (cpd 25).

Using the same procedure described in example A.4 and starting from 4-phenyl-2-(piperidin-4-ylmethyl)pyridazin-3(2H)-one 2,2,2-trifluoroacetate and 2-chloro-1-phenylethan-1-one, the title compound was isolated in 35% yield after a reduction step of the carbonyl group with NaBH₄. ¹H NMR (400 MHz, CDCl₃) : δ ppm 7.73 (d, 1H, J = 4.0 Hz), 7.72-7.68 (m, 2H), 7.39-7.31 (m, 3H), 7.29-7.12 (m, 6H), 4.69 (dd, 1H, J = 10.9 Hz, J = 3.5 Hz), 4.08 (d, 2H, J = 8.2 Hz), 3.02 (d, 1H, J = 11.7 Hz), 2.72 (d, 1H, J = 11.3 Hz), 2.41 (dd, 1H, J = 12.4 Hz, J = 4.5 Hz), 2.35 (q, 1H, J = 11.9 Hz), 2.21 (dt, 1H, J = 11.7 Hz, J = 2.8 Hz), 2.06-1.89 (m, 2H), 1.68-1.57 (m, 2H), 1.47-1.30 (m, 2H). ¹³CNMR (101 MHz, CDCl₃) : δ ppm 160.3, 142.3, 139.7, 136.0, 133.9, 129.6, 128.7, 128.4, 128.3, 127.4, 127.3, 125.8, 68.8, 66.4, 57.8, 54.8, 51.6, 35.0, 30.2, 29.9.
LC/MS (M+H) = 390.

### Example A.5: 2-(2-(1-benzylpiperidin-4-yl)ethyl)-4-phenylpyridazin-3(2H)-one hydrochloride, (cpd 26).

### Step 1: tert-butyl 4-(2-((chlorosulfonyl)oxy)ethyl)piperidine-1-carboxylate

At room temperature under nitrogen, mesylchloride (0.52 mL, 2 equiv.) was added dropwise to a solution of tert-butyl 4-(2-hydroxyethyl)piperidine-1-carboxylate (840 mg, 3.7 mmol, 1.0 equiv.) and triethylamine (1.0 mL, 2 equiv.) in anhydrous DCM (20 mL). The solution was stirred for 4 h and was quenched with 20 mL of 1 M HCl. The aqueous phase was extracted with EtOAc (3x20 mL), the combined organic layers were washed with brine (20 mL), dried over Na₂SO₄, filtered and then concentrated under vacuum. The residual oil was crystallized from heptane (40 mL) to afford the title compound (1.05 g, 93%). ¹H NMR (400 MHz, CDCl₃) : δ ppm 4.30 (t, 2H, J =6.4 Hz), 3.04 (s, 3H), 2.71 (t, 2H, J = 12.5 Hz), 1.75-1.60 (m, 7H), 1.47 (s, 9H), 1.21-1.15 (m, 2H). ¹³C NMR (101 MHz, CDCl₃) : δ ppm 154.8, 79.4, 77.4, 67.4, 37.5, 35.6, 32.4, 31.7, 28.5.

### Step 2: tert-butyl 4-(2-(6-oxo-4-phenylpyridazin-1(6H)-yl)ethyl)piperidine-1-carboxylate

A 50 ml flask (oven-dried and under argon) was charged with 4-phenyl-2,3-dihydropyridazin-3-one (200 mg, 1.16 mmol, 1.0 equiv.), tert-butyl 4-(2-((chlorosulfonyl)oxy)ethyl)piperidine-1-carboxylate (357.2 mg, 1.16 mmol, 1.0 equiv.) and K₂CO₃ (160.6 mg, 1.16 mmol, 1.0 equiv.). 2-butanone (10 mL) was added and the mixture was heated at 85°C for 16 h (HPLC monitoring : complete conversion). The mixture was quenched with H₂O (150 mL) and extracted with EtOAc (3 x 40 mL). The combined organic layers were washed with brine (2 x 50 mL), dried over Na₂SO₄, filtered and evaporated to dryness. The resulting residue was purified by silica gel flash chromatography using EtOAc /Heptane : 1:2 as eluent to give the title compound as a white solid (357 mg, 80%). ¹H NMR (200 MHz, CDCl₃) : δ ppm 7.87 (d, 1H, J = 4.1 Hz), 7.83-7.80 (m, 2H), 7.49-7.46 (m, 3H), 7.32 (d, 1H, J = 4.1 Hz), 4.32 (t, 2H, J = 7.4 Hz), 4.15-4.08 (m, 2H), 2.73 (td, 2H, J =13.0 Hz, 2.4 Hz), 1.88-1.75 (m, 4H), 1.52-1.49 (m, 1H), 1.49 (s, 9H), 1.32-1.17 (m, 2H).

### Step 3: 2-(2-(1-benzylpiperidin-4-yl)ethyl)-4-phenylpyridazin-3(2H)-one, (cpd 26).

To a solution of tert-butyl 4-(2-(6-oxo-5-phenylpyridazin-1(6H)-yl)ethyl)piperidine-1-carboxylate (357 mg, 0.93 mmol, 1 equiv.) in DCM (5 mL) at 0 °C was added TFA (2 mL), and the resulting mixture was stirred at rt for 1 h. The crude reaction was concentrated under vacuum with azeotropic removal of TFA with heptane. The crude was quenched with aqueous NaHCOs saturated solution (10 mL) and extracted twice with EtOAc (2x 8 mL). The combined organic layers were washed with brine (2 x 50 mL), dried over Na₂SO₄, filtered and evaporated to dryness to obtain 4-phenyl-2-(2-(piperidin-4-yl)ethyl)pyridazin-3(2H)-one as a viscous oil (252 mg, 96%). The compound was used in the reductive amination step without further purification. ¹H NMR (200 MHz, CDCl₃) : δ ppm 7.86 (d, 1H, J = 4.1 Hz), 7.84-7.80 (m, 2H), 7.50-7.43 (m, 3H), 7.32 (d, 1H, J = 4.1 Hz), 4.32 (t, 2H, J = 7.4 Hz), 3.53 (s, 1H), 3.22-3.14 (m, 2H), 2.67 (td, 2H, J =13.0 Hz, 2.4 Hz), 1.99-1.79 (m, 4H), 1.52-1.49 (m, 1H), 1.41-1.26 (m, 2H).

4-phenyl-2-(2-(piperidin-4-yl)ethyl)pyridazin-3(2H)-one (118 mg, 0.41 mmol) was dissolved in MeOH (5 mL). Benzaldehyde (66.2 mg, 63.2 µL, 1.5 equiv.) was added followed by NaBH₃CN (52.3 mg, 0.83 mmol, 2 equiv.) and AcOH. The resulting mixture was stirred at rt for 48h. Volatiles were evaporated and the crude was taken up in EtOAc (25 mL). The organic phase was washed with brine, dried and concentrated under vacuum. The residue was purified by silica gel column chromatography using DCM: MeOH (95: 5) as eluent to yield 2-(2-(1-benzylpiperidin-4-yl)ethyl)-4-phenylpyridazin-3(2H)-one as an colorless oil (112 mg, 72%). Hydrochloride salt gives white powder. ¹H NMR (400 MHz, DMSO-d6) : δ ppm 10.06 (bs, 1H), 8.00 (d, 1H, J = 4.2 Hz), 7.84-7.81 (m, 2H), 7.59 (d, 1H, J = 4.2 Hz), 7.57-7.54 (m, 2H), 7.47-7.37 (m, 6H), 4.24 (d, 2H, J = 5.2 Hz), 4.18 (t, 2H, J = 7.1 Hz), 3.32-3.30 (m, 2H), 2.87 (q, 2H, J = 10.6 Hz), 1.94-1.86 (m, 3H), 1.69 (q, 2H, J = 6.6 Hz), 1.53-1.43 (m, 2H). ¹³C NMR (101 MHz, DMSO-d6) : δ ppm 159.1, 137.9, 136.9, 133.9, 131.4, 129.7, 129.5, 129.3, 128.7, 128.6, 128.3, 128.2, 59.1, 51.4, 48.8, 33.9, 30.6, 28.5.
LC/MS (M+H) = 374.

### * 2-(2-(1-benzylpiperidin-4-yl)ethyl)-5-phenylpyridazin-3(2H)-one hydrochloride, (cpd 27).

Using the same procedure described in example A.3 and starting from 5-phenylpyridazin-3(2H)-one (100 mg, 0.58 mmol) and N-Boc-4-(2-((methylsulfonyl)oxy)ethyl)piperidine (178.5 mg, 0.58 mmol, 1 equiv.), tert-butyl 4-(2-(6-oxo-5-phenylpyridazin-1(6H)-yl)ethyl)piperidine-1-carboxylate was obtained after purification on silica gel using EtOAc/Heptane 1/2 as eluent (206 mg, 93%). ¹H NMR (400 MHz, CDCl₃) : δ ppm 8.01 (d, 1H, J = 2.0 Hz), 7.54-7.52 (m, 2H), 7.46-7.45 (m, 3H), 7.00 (d, 1H, J = 2.0 Hz), 4.20 (t, 2H, J = 7.3 Hz), 4.04 (bs, 2H), 2.64 (t, 2H, J =13.0 Hz), 1.78-1.70 (m, 4H), 1.47-1.41 (m, 1H), 1.41 (s, 9H), 1.18-1.11 (m, 2H). ¹³C NMR (101 MHz, CDCl₃) : δ ppm 160.6, 155.0, 143.6, 135.8, 134.1, 130.4, 129.5, 127.0, 124.7, 79.4, 49.3, 35.1, 33.7, 32.1, 28.7, 28.6.

Deprotection of the tert-butoxycarbonyl group with TFA followed by reductive amination reaction with benzaldehyde and NaBH₃CN led to the title compound in 75% yield. Hydrochloride salt gives white powder. ¹H NMR (400 MHz, CDCl₃) : δ ppm 8.00 (d, 1H, J = 2.1 Hz), 7.54-7.52 (m, 2H), 7.46-7.45 (m, 3H), 7.27-7.26 (m, 4H), 7.22-7.19 (m, 1H), 7.00 (d, 1H, J = 2.1 Hz), 4.19 (t, 2H, J = 7.5 Hz), 3.45 (s, 2H), 2.84 (d, 2H, J =10.8 Hz), 1.91 (t, 2H, J =10.8 Hz), 1.77-1.70 (m, 4H), 1.34-1.28 (m, 3H). ¹³C NMR (101 MHz, CDCl₃) : δ ppm 160.7, 143.5, 138.7, 135.8, 134.2, 130.4, 129.6, 129.4, 128.3, 127.1, 127.0, 124.8, 63.7, 53.9, 49.6, 35.2, 33.6, 32.4.
LC/MS (M+H) = 374.

### Substitution of the piperidine

The invention provides also a process for the preparation of compounds of general formula (I) carrying small substituents R' (fluoro, methyl or hydroxyl) or R" (C1-C5-alkyl, C3-C7 cycloalkyl) on C-4 carbon of the piperidine. Illustrative general synthetic methods are set out below by a description of exemplary synthesis of specific compounds of the invention, as illustrated in scheme 2.

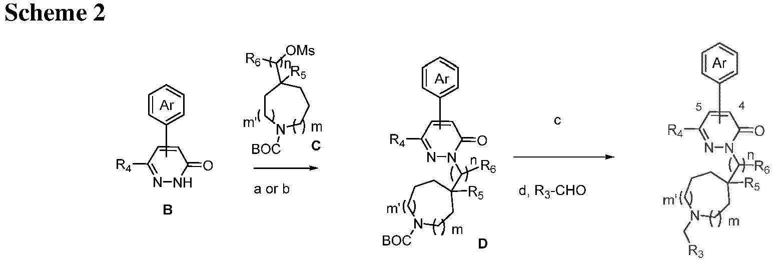

**Conditions:** a) n = 0, K₂CO₃, 2-butanone, 85°C, 24h ; b) n = 1, NaH, DMF, 110°C, 18h; c) TFA, DCM, 25°C, 45 min; d) R₃CHO, NaBH₃CN; DIEA, MeOH, 25°C, 18h or R₃-CH₂Br, K₂CO₃, DMF or MeCN, 80°C, 18h.

### Example A.6: 2-(1 -(1 -benzylpiperidin-4-yl)ethyl)-4-phenylpyridazin-3(2H)-one hydro chloride, (cpd 28).

Using the same procedure described in example A.2 and starting from 4-phenyl-2,3-dihydropyridazin-3-one (200 mg, 1.16 mmol, 1.0 equiv.) and tert-butyl 4-(1-((methylsulfonyl)oxy)ethyl)piperidine-1-carboxylate⁶ (357.1 mg, 1.16 mmol, 1.0 equiv.), tert-butyl 4-(1-(6-oxo-5-phenylpyridazin-1(6H)-yl)ethyl)piperidine-1-carboxylate was obtained as an off white solid (145 mg, 33%). ¹H NMR (400 MHz, CDCl₃) : δ ppm 7.87 (d, 1H, J = 4.0Hz), 7.79-7.75 (m, 2H), 7.49-7.38 (m, 3H), 7.24(d 1H, J= 4.0 Hz), 5.15-5.06 (m, 1H), 4.23-3.91 (m, 2H), 2.80-2.53 (m, 2H), 2.04-1.92 (m, 1H), 1.85-1.61(m, 2H), 1.42 (s, 9H), 1.39 (d, 3H, J = 6.6 Hz), 1.36-1.16 (m, 2H).

After deprotection of the BOC with the help of TFA, the resulting trifluoroacetate salt was submitted to reductive amination with benzaldehyde using NaBH₃CN to yield the title compound (44 mg, 35%) ¹H NMR (400 MHz, CDCl₃) : δ ppm 7.86 (d, 1H, J = 4.0 Hz), 7.80-7.75 (m, 2H), 7.47-7.39 (m, 3H), 7.32-7.20 (m, 6H), 5.17-5.08 (m, 1H), 3.74 (t, 2H, J = 6.1 Hz), 3.46 (s, 2H), 2.93 (d, 1H, J = 11.2 Hz), 2.82 (d, 1H, J = 11.2 Hz), 1.99 (t, 1H, J = 11.3 Hz), 1.91-1.75 (m, 2H), 1.52-1.31 (m, 2H), 1.38 (d, 3H, J = 6.8 Hz). ¹³C NMR (101 MHz, CDCl₃) : δ ppm 160.4, 139.4, 138.5, 136.1, 134.4, 129.5, 129.2, 128.8, 128.4, 128.2, 127.0, 126.9, 68.1, 63.2, 57.8, 53.5, 40.5, 28.04, 25.7, 17.1.
LC/MS (M+H) = 374.
⁶ ACS Medicinal Chemistry letters, 4 (11), 1064-1068, 2013.

### * 2-((1-benzyl-4-hydroxypiperidin-4-yl)methyl)-4-phenylpyridazin-3(2H)-one hydrochloride, (cpd 29).

Using the same procedure described in example A.6 and starting from 4-phenyl-2,3-dihydropyridazin-3-one (200 mg, 1.16 mmol, 1.0 equiv.) and freshly prepared tert-butyl 4-hydroxy-4- ((tosyloxy) methyl)piperidine-1-carboxylate (1.0 equiv. from tert-butyl 4-hydroxy-4-(hydroxymethyl) piperidine-1-carboxylate)⁷, tert-butyl 4-hydroxy-4-((6-oxo-5-phenylpyridazin-1(6H)-yl)methyl) piperidine-1-carboxylate was obtained in 75% yield. ¹H NMR (400 MHz, CDCl₃) : δ ppm 7.87 (d, 1H, J = 4.0 Hz), 7.77-7.73 (m, 2H), 7.46-7.42 (m, 3H), 7.33-7.29 (m, 5H), 4.37 (s, 2H), 3.75-3.72 (m, 3H), 3.63-3.58 (m, 2H), 2.72-2.69 (m, 2H), 2.55 (t, 2H, J= 11.0 Hz), 1.81-1.77 (m, 2H), ¹³C NMR (101 MHz, CDCl₃) : δ ppm 162.2, 140.4, 136.9, 133.5, 129.8, 129.4, 128.7, 128.5, 128.4, 128.1, 127.4 , 70.4, 67.7, 62.7, 48.9, 35.4.
LC/MS (M+H) = 376
⁷ Hartman G.D., Flores, O. A., WO 2013096744

Alternatively, the Suzuki-Miyaura cross coupling reaction can be performed later in the sequence enabling more convergent methods for i) the introduction of various aryls on positions 4 or 5 of the pyridazinones rings (method a) or ii) the functionalization of the piperidine moieties (method b) Using the same conditions described previously, N-alkylation of chloropyridazinone derivatives A with appropriate tert-butyl 4- methylsulfonyl oxy-piperidine-1-carboxylate derivatives of general formula C, afforded intermediates E. A derivatization step of E with suitable reagents performed by methods well known in the art, led to intermediates F which were finally involved in a Suzuki-Miyaura reaction, giving examples of the present invention (example A.9). The Suzuki Miyaura reaction can also be performed from intermediates E leading to the previously described intermediates D as illustrated in scheme 3 (examples A.7-8)

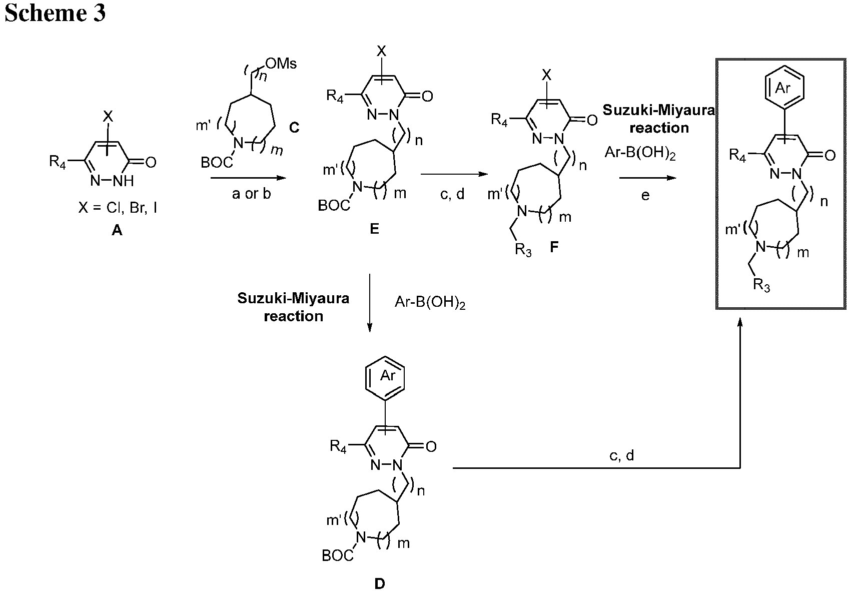

**Conditions** : a) K₂CO₃, 2-butanone, 85°C, 24h ; b) NaH, DMF, 110°C, 18h ; c) TFA, DCM, 25°C, 45 min; d) R₃CHO, NaBH₃CN; DIEA, MeOH, 25°C, 18h, or R₃-CH₂Br, K₂CO₃, DMF or MeCN, 80°C, 18h. e) K₂CO₃, Pd(PPh₃)₄, PhB(OH)₂, Toluene, EtOH, H₂O, 120°C, 16h .

### Example A.7: 2-(1-benzylpiperidin-4-yl)-4-(4-methoxyphenyl)pyridazin-3(2H)-one hydrochloride , (cpd 30).

### Step 1: tert-butyl 4-((5-chloro-6-oxopyridazin-1(6H)-yl))piperidine-1-carboxylate

The reaction was performed under argon atmosphere. To a solution of 4-chloro-2, 3-dihydropyridazin-3-one (100 mg, 0.77 mmol, 1.0 equiv.) in butanone (7.0 mL) was added tert-butyl 4-(methanesulfonyloxy)piperidine-1-carboxylate (321.0 mg, 1.15 mmol, 1.5 equiv.) and K₂CO₃. The resulting mixture was heated at 85°C overnight (HPLC monitoring: complete conversion). The mixture was quenched with H₂O (150 mL) and extracted with EtOAc (3 x 40 mL). The combined organic layers were washed with brine (2 x 50 mL), dried over Na₂SO₄, filtered and evaporated to dryness. The resulting residue was purified by silica gel flash chromatography using a gradient of 0% to 50% of EtOAc in heptane, to give the title compound (180 mg, 0.57 mmol, 75%). ¹H NMR (400 MHz, CDCl₃) : δ ppm 7.71 (d, 1H, J = 4.4 Hz), 7.33 (d, 1H, J = 4.4 Hz), 5.21-5.00 (m, 1H), 4.40-4.20 (m, 2H,), 1.90-1.80 (m, 4H), 1.47 (s, 9H).

### Step 2: tert-butyl 4-(5-(4-methoxyphenyl)-6-oxopyridazin-1(6H)-yl)piperidine-1-carboxylate

A microwave vial (oven-dried and under argon) was charged with 4-methoxyphenylboronic acid (125.9 mg, 0.83 mmol, 1.3 equiv.), tert-butyl 4-(5-chloro-6-oxopyridazin-1(6H)-yl)piperidine-1-carboxylate (200 mg, 0.64 mmol, 1.0 equiv.), sodium carbonate (135.1 mg, 1.27 mmol, 2 equiv.). Tetrakis(triphenylphosphine) palladium(0) (74.4 mg, 10 mol %.) was then added, followed by DME (3.34 mL), and H₂O (1.1 mL). The vial was properly capped and the mixture vessel was evacuated and backfilled with argon (process repeated 3 times), and heated at 100°C until complete conversion of the starting material. The reaction conversion was monitored by HPLC and was usually completed within 16 hours. After cooling to room temperature, the reaction mixture was evaporated to dryness. The crude was partitioned through EtOAc (30 mL) and H₂O (50mL). The aqueous phase was extracted twice with EtOAc (20 mL). The organic phases were combined, washed with brine and dried (Na₂SO₄) and evaporated. The crude material was flash chromatography using a gradient of 0 to 40% of EtOAc in heptane to give the title compound as a white solid (193 mg, 0.5 mmol, 79%). ¹H NMR (400MHz, CDCl₃) : δ ppm 7.84 (d, 1H, J = 4.0 Hz), 7.81 (d, 2H, J = 8.4 Hz), 7.21 (d, 1H, J = 4.4 Hz), 6.96 (d, 2H, J = 8.4 Hz), 5.18 (m, 1H), 4.28-4.21 (m, 2H), 2.95-2.90 (m, 2H), 2.05-1.90 (m, 4H), 1.44 (s, 9H). ¹³C NMR (101 MHz, CDCl₃) : δ ppm 160.9, 160.1, 154.8, 138.8, 136.4, 130.3, 126,6, 125.9, 113.979.6, 56.5, 43.3, 30.4, 28.6.

### Step 3: 2-(1-benzylpiperidin-4-yl)-4-(4-methoxyphenyl)-2,3-dihydro pyridazin-3-one hydrochloride, (cpd 30).

To a solution of tert-butyl 4-(5-(4-methoxyphenyl)-6-oxopyridazin-1(6H)-yl)piperidine-1-carboxylate (180 mg, 0.467 mmol, 1 equiv.) in DCM (2 mL) at 0 °C was added TFA (2.0 mL), and the resulting mixture was stirred at rt for 1 h. The crude reaction was concentrated under vacuum with azeotropic removal of TFA with heptane. The residue was triturated with ice-cooled ether, the supernatant was removed and the crude after evaporation was directly dissolved in MeOH (4 mL). Benzaldehyde (74.3 mg, 70.8 µL, 1.5 equiv.) was added followed NaBH₃CN (61. 8 mg, 0.9 mmol, 2 equiv.) and AcOH (2.7 µl). The resulting mixture was stirred at rt for 16h. Volatiles were evaporated and the crude was taken up in EtOAc (25 mL). The organic phase was washed with brine, dried and concentrated under vacuum. The residue was purified by silica gel column chromatography using DCM: MeOH (98: 2) as eluent to yield 2-(1-benzylpiperidin-4-yl)-4-(4-methoxyphenyl)-2,3-dihydropyridazin-3-one hydrochloride as a white solid after salification with 2N HCl in Et₂O (78mg, 41%). Tr (HPLC) = 7.35min . ¹H NMR (400 MHz, CDCl₃) : δ ppm 7.76 (d, 1H, J = 4.2 Hz), 7.73(d, 2H, J = 9.0 Hz), 7.36 (d, 2H, J = 6.4 Hz), 7.34(m, 2H), 7.14 (d, 1H, J= 4.2 Hz), 7.24 (t, 2H, J = 7.9 Hz), 7.19-7.15 (m, 1H), 6.88 (d, 2H, J = 9.2 Hz), 5.02-4.93 (m, 1H), 3.83 (s, 3H), 3.54 (s, 2H), 3.04-3.02 (m, 2H), 2.18 (qd, J = 11.5 Hz, J = 1.5 Hz), 2.12 (qd, J = 11.9 Hz, J= 3.6 Hz), 1.89-1.86 (m, 2H). ¹³C NMR (101 MHz, CDCl₃, DEPT 135) : δ ppm 136.1, 130.2, 129.1, 128.2, 127.0, 125.8, 113.8, 62.8, 55.7, 55.4, 52.8, 30.4.
LC/MS (M+H) = 376.

### * 2-(1-benzylpiperidin-4-yl)-4-[4-(trifluoromethyl)phenyl]-2,3-dihydropyridazin-3-one hydro chloride, (cpd 31).

Using the same procedure described in example A.7 and starting from tert-butyl 4-((5-chloro-6-oxopyridazin-1(6H)-yl))piperidine-1-carboxylate (100 mg, 0.32 mmol, 1 equiv.) and 4-trifluoromethyl phenylboronic acid (78.7 mg, 0.41 mmol, 1.3 equiv.), tert-butyl 4-{6-oxo-5-[4-(trifluoromethyl)phenyl]-1,6-dihydropyridazin-1-yl}piperidine-1-carboxylate was obtained as a white solid (65 mg, 48%). ¹H NMR (400 MHz, CDCl₃) : δ ppm 8.05-7.98 (m, 3H), 7.71 (d, 2H, J = 8.0 Hz), 7.31 (d, 1H, J = 4.0 Hz), 5.22-5.13 (m, 1H), 4.30-4.26 (m, 2H), 2.96-2.88 (m, 2H), 2.02-1.90 (m, 4H), 1.49 (s, 9H). ¹³C NMR (101 MHz, CDCl₃) : δ ppm 159.6, 154.8, 138.1, 137.7,136.2, 129.2, 127.9, 125.5, 125.4, 79.9, 55.9, 43.1, 30.5, 28.6. ¹⁹F NMR (376 MHz, CDCl₃) : δ ppm - 62.84.

After deprotection of the *tert*-butyloxycarbonyl group with TFA, the crude (74 mg, 0.229 mmol, 1 equiv.) was submitted to a reductive amination with the help of benzaldehyde (36.45 mg, 34.7µL, 0.34 mmol, 1.5 equiv.), NaBH₃CN (30.3 mg, 0.46 mmol, 2 equiv.) and DIEA (4 equiv.) to give the title compound after salification as a white solid (30 mg, 29 %). ¹H NMR (400 MHz, CDCl₃) : δ ppm 7.95 7.85 (m, 3H), 7.69 (d, 2H, J = 8.0 Hz), 7.40 -7.26 (m, 6H), 5.08-5.00 (m, 1H), 3.57 (s, 2H), 3.10-3.02 (m, 2H), 2.28-3.02 (m, 4H), 1.92-1.87 (m, 2H). ¹³C NMR-DEPT-135 (101 MHz, CDCl₃) : δ ppm 135.9, 129.2, 128.4, 127.8, 127.2, 125.4, 125.4, 62.9, 56.2, 52.9, 30.5. ¹⁹F NMR (376MHz, CDCl₃) ; δ ppm - 62.86.
LC/MS (M+H) = 414.

### * 2-(1-benzylpiperidin-4-yl)-4-[4-(chlorophenyl]-2,3-dihydropyridazin-3-one hydrochloride, (cpd 32).

Using the same procedure described in example A.6 and starting from tert-butyl 4-((5-chloro-6-oxopyridazin-1(6H)-yl))piperidine-1-carboxylate (200 mg, 0.64 mmol, 1 equiv.) and 4-chlorophenylboronic acid (99.7 mg, 0.63 mmol, 1.0 equiv.), tert-butyl 4-{6-oxo-5-[4-chlorophenyl]-1,6-dihydropyridazin-1-yl}piperidine-1-carboxylate was obtained as a yellow solid (195 mg, 78%). ¹H NMR (400 MHz, CDCl₃) : δ ppm 7.87 (d, 1H, J = 4.0 Hz) 7.76 (d, 2H, J = 8.0 Hz), 7.42 (d, 2H, J = 8.0 Hz), 7.26 (d, 1H, J = 4.0 Hz), 5.22-5.13 (m, 1H), 4.40-4.20 (m, 2H), 2.98-2.91 (m, 2H), 2.02-1.90 (m, 4H), 1.49 (s, 9H).

After deprotection of the *tert*-butyloxycarbonyl group with TFA, the crude (150 mg, 0.229 mmol, 1 equiv.) was submitted to a reductive amination with the help of benzaldehyde (79.6 mg, 75.8µL, 0.75mmol, 1.5 equiv.) and NaBH₃CN (66.1 mg, 1mmol, 2 equiv.) to yield the title compound after salification with 2N HCl as a white solid (32 mg, 15 %). ¹H NMR (400 MHz, CDCl₃) : δ ppm 7.87 (d, 1H, J = 4.0 Hz), 7.75 (d, 2H, J = 8.0 Hz), 7.40 (d, 2H, J = 8.0 Hz), 7.37-7.23 (m, 6H), 5.05-4.99 (m, 1H), 3.57 (s, 2H), 3.05-3.00 (m, 2H), 2.28-2.10 (m, 4H), 1.90-1.88 (m, 2H). ¹³C NMR-DEPT-135 (101 MHz, CDCl₃) : δ ppm 159.8, 138.7, 137.9, 136.0, 135.6, 132.7, 130.2, 129.2, 128.7, 128.3, 127.1, 127.0, 62.9, 56.1, 52.9, 30.5.
LC/MS (M+H) = 380.

### Example A.8: 2-[(1-benzylpiperidin-4-yl)methyl]-4-(2-chlorophenyl)-2,3-dihydropyridazin-3-one hydrochloride, (cpd 33).

### Step 1: tert-butyl 4-((5-chloro-6-oxopyridazin-1(6H)-yl)methyl)piperidine-1-carboxylate

The reaction was performed in anhydrous conditions under argon atmosphere. To an ice-cooled solution of 4-chloro-2,3-dihydropyridazin-3-one (110 mg, 0.84 mmol, 1 equiv.) in dry DMF (8.42 mL) was added portionwise sodium hydride (67.41 mg, 1.68 mmol, 2 equiv.) and the mixture was stirred at 0°C for 30 min. Then tert-butyl 4-(((methylsulfonyl)oxy)methyl)piperidine-1-carboxylate (296.7 mg, 1.01 mmol, 1.2 equiv.) was added portionwise and the mixture was heated at 110°C overnight (HPLC monitoring: complete conversion). The mixture was quenched with H₂O (150 mL) and extracted with EtOAc (3 x 40 mL). The combined organic layers were washed with brine (2 x 50 mL), dried over Na₂SO₄, filtered and evaporated to dryness. The resulting residue was purified by silica gel flash chromatography using a gradient of 10 to 50% of EtOAc in heptane to give the title compound **3** as a white solid (164 mg, 0.50 mmol, 60%). ¹H NMR (400 MHz, CDCl₃) : δ ppm 7.66 (d, 1H, J = 4.4 Hz), 7.35 (d, 1H, J = 4.4 Hz), 4.11-4.08 (m, 4H), 2.66 (t, 2H, J = 12.4 Hz), 2.16-2.11 (m, 1H), 1.59 (d, 1H, J = 12.4 Hz), 1.44 (s, 9H), 1.29-1.19 (m, 2H). ¹³C NMR (101 MHz, CDCl₃) : δ ppm 157.9, 154.7, 137.2, 134.8, 129.0, 79.4, 58.1, 43.4, 35.3, 29.6, 28.4. Purity (100%) , t_{R} (HPLC) : 3.71 min.

### Step 2: tert-butyl 4-{[5-(2-chlorophenyl)-6-oxo-1,6-dihydropyridazin-1-yl]methyl}piperidine-1-carboxylate

A microwave vial (oven-dried and under argon) was charged with 2-chlorophenylboronic acid (78.7 mg, 0.50 mmol, 1.1 equiv.), tert-butyl 4-[(5-chloro-6-oxo-1,6-dihydropyridazin-1-yl)methyl]piperidine-1-carboxylate (150 mg, 0.46 mmol, 1.0 equiv.), sodium carbonate (145.5 mg, 1.37 mmol, 3 equiv.). Tetrakis(triphenylphosphine) palladium(0) (26.4 mg, 5 mol %.) was then added, followed by toluene (1.74 mL), and H₂O (0.35 mL) and EtOH (0.35 mL) . The vial was properly capped and the mixture vessel was evacuated and backfilled with argon (process repeated 3 times) and heated at 120°C until complete conversion of the starting material. The reaction conversion was monitored by HPLC and was usually completed within 17 hours. After cooling to room temperature, the reaction mixture was evaporated to dryness. The crude was partitioned through EtOAc (30 mL) and H₂O (50mL). The aqueous phase was extracted twice with EtOAc (20 mL). The organic phases were combined, washed with brine and dried (Na₂SO₄) and evaporated. The crude material was flash chromatography using a gradient of 0 to 40% of EtOAc in heptane to give the title compound as a yellow oil (138 mg, 0.34 mmol, 75%). ¹H NMR (400 MHz, CDCl₃) : δ ppm 7.83(d, 1H, J= 4.0 Hz), 7.48 (d, 1H, J = 7.6 Hz), 7.99-7.26 (m, 3H), 7.23 (d, 1H, J = 4.0 Hz), 4.20-4.00 (m, 4H), 2.69 (m, 2H), 2.20-2.16 (m, 1H), 1.66-1.60 (m, 2H), 1.45 (s, 9H), 1.32-1.20 (m, 2H). ¹³C NMR (101 MHz, CDCl₃) : δ ppm 160.1, 154.8, 139.3, 135.5, 133.1, 133.0, 130.9, 130.2, 129.9, 126.7, 79.37, 60.4, 57.6, 35.5, 29.7, 28.4.

### Step 3: 2-[(1-benzylpiperidin-4-yl)methyl]-4-(2-chlorophenyl)-2,3-dihydropyridazin-3-one hydrochloride, (cpd 33).

To a solution of tert-butyl 4-{[5-(2-chlorophenyl)-6-oxo-1,6-dihydropyridazin-1-yl]methyl}piperidine-1-carboxylate (123 mg, 0.30 mmol, 1 equiv.) in DCM (0.8 mL) at 0 °C was added TFA (0.8 mL), and the resulting mixture was stirred at rt for 1 h. The crude reaction was dissolved in water and ammonium hydroxyde was added until pH =10. The residue was extracted with EtOAc (twice) and dried over Na₂SO₄. After evaporation, the crude was directly dissolved in MeOH (4 mL). Benzaldehyde (36.5 mg, 34.7 µL, 1.2 equiv.) was added followed by NaBH₃CN (35. 9 mg, 0.57 mmol, 2 equiv.). The resulting mixture was stirred at rt for 19h. Volatiles were evaporated and the crude was taken up in EtOAc (25 mL). The organic phase was washed with brine, dried and concentrated under vacuum. The residue was purified by silica gel column chromatography using DCM : MeOH (98: 2) as eluent to yield 22-[(1-benzylpiperidin-4-yl)methyl]-4-(2-chlorophenyl)-2,3-dihydropyridazin-3-one hydrochloride as a white solid after salification with 2N HCl in Et₂O (62.1 mg, 50%). Tr (HPLC) = 7.30 min. ¹H NMR (400 MHz, CDCl₃ ) : δ ppm 7.79 (d, 1H, J = 4.0 Hz), 7.50-7.17 (m, 10H), 4.10 (d, 2H, J = 7.2 Hz), 3.46 (s, 2H), 2.84 (t, 2H, J = 11.8 Hz), 2.05-1.90 (m, 3H), 1.65-1.60 (m, 2H), 1.47-1.35 (m, 2H). ¹³C NMR (101 MHz, CDCl₃) : δ ppm 159.6, 139.2, 138.4, 135.3, 133.2, 133.0, 131.0, 130.3, 130.1 129.9, 129.1, 128.1, 126.9, 126.7, 63.3, 57.8, 53.2, 35.3, 29.8 .
LC/MS (M+H) = 394.

### * 2-[(1-benzylpiperidin-4-yl)methyl]-4-(4-hydroxyphenyl)-2,3-dihydropyridazin-3-one hydro chloride (cpd 34)

Using the same procedure described in example A.8 and starting from tert-butyl 4-[(5-chloro-6-oxo-1,6-dihydropyridazin-1-yl)methyl]piperidine-1-carboxylate (150 mg, 0.46 mmol, 1 equiv.) and 4-hydroxyphenylboronic acid (88.36 mg, 1.4 equiv.), tert-butyl 4-{[5-(4-hydroxyphenyl)-6-oxo-1,6-dihydropyridazin-1-yl]methyl}piperidine-1-carboxylate was obtained in 77% yield (135 mg). ¹H NMR (400 MHz, CDCl₃) : δ ppm 7.81 (d, 1H, J = 4.0 Hz), 7.67 (d, 2H, J = 8.8 Hz), 7.45-7.30 (bs, 1H), 7.25 (d, 1H, J = 4.0 Hz), 6.86 (d, 2H, J = 8.8 Hz), 4.15 (d, 1H, J = 7.2 Hz), 4.13-4.07 (m, 3H), 2.70 (t, 2H, J = 12.8 Hz), 2.26-2.15 (m, 1H), 1.65 (d, 2H, J = 12.8 Hz). 1.46 (s, 9H), 1.37-1.23 (m, 2H). ¹³C NMR (101 MHz, CDCl₃) : δ ppm 160.7, 157.9, 154.9, 139.7, 136.6, 130.3, 126.3, 125.4, 115.7, 79.7, 57.7, 43.6, 35.4, 29.7, 28.5.

After deprotection of the *tert*-butyloxycarbonyl group with TFA, the crude trifluoroacetate salt (140 mg, 0.35mmol, 1 equiv.) was submitted to a reductive amination with the help of benzaldehyde (55.8 mg, 53.1µL, 0.52mmol, 1.5 equiv.), NaBH₃CN (44.1 mg, 0.7mmol, 2 equiv.) and DIEA (181.2 mg, 1.40 mmol, 4 equiv.) to yield the title compound after salification with 2N HCl in 54% yield (71 mg). ¹H NMR (400 MHz, CDCl₃) : δ ppm 7.74 (d, 1H, J= 4.0 Hz), 7.60 (d, 2H, J = 8.8 Hz), 7.28-7.22 (m, 5H), 7.16 (d, 1H, J = 4.0 Hz), 6.74 (d, 2H, J = 8.8 Hz), 4.11 (d, 2H, J = 6.8 Hz), 3.51 (s, 2H), 2.90 (dt, J = 12.0 Hz, J = 5.2 Hz), 2.01 (td, 3H, J = 11.6 Hz, J = 2.0 Hz), 1.68-1.59 (m, 2H), 1.50 (qd, 2H, J = 12.0 Hz, J = 4.0 Hz). ). ¹³C NMR (101 MHz, CDCl₃) : δ ppm 160.7, 157.9, 139.5, 137.2, 136.4, 130.3, 129.5, 128.2, 127.3, 126.2, 125.4, 115.7, 16.1, 57.7, 53.1, 35.1, 29.4.
LC/MS (M+H) = 376.

### Example A.9: 2-((1-benzylpiperidin-4-yl)methyl)-4-(4-fluorophenyl)pyridazin-3(2H)-one hydrochloride (cpd 35).

### Step 1: tert-butyl 4-((5-chloro-6-oxopyridazin-1(6H)-yl)methyl)piperidine-1-carboxylate

The reaction was performed in anhydrous conditions under argon atmosphere. To an ice-cooled solution of 4-chloro-2, 3-dihydropyridazin-3-one (110 mg, 0.84 mmol, 1.0 equiv.) in dry DMF (8.42 mL) was added portionwise sodium hydride (67.41 mg, 1.68 mmol, 2.0 equiv.) and the mixture was stirred at 0°C for 30 min. Then tert-butyl 4-[(methylsulfonyl)oxy)methyl]piperidine-1-carboxylate (296.7 mg, 1.01 mmol, 1.2 equiv.) was added portionwise and the mixture was heated at 110°C overnight (HPLC monitoring: complete conversion). The mixture was quenched with H₂O (150 mL) and extracted with EtOAc (3 x 40 mL). The combined organic layers were washed with brine (2 x 50 mL), dried over Na₂SO₄, filtered and evaporated to dryness. The resulting residue was purified by silica gel flash chromatography using a gradient of 10% to 50% of EtOAc in heptane to give the title compound as a white solid (164 mg, 0.50 mmol, 60%). ¹H NMR (400 MHZ, CDCl₃) : δ ppm 7.66 (d, 1H, J = 4.4 Hz), 7.35 (d, 1H, J = 4.4 Hz), 4.11-4.08 (m, 4H), 2.66 (t, 2H, J = 12.4 Hz), 2.16-2.11 (m, 1H), 1.59 (d, 1H J = 12.4Hz), 1.44 (s, 9H), 1.29-1.19 (m, 2H); ¹³C NMR (101 MHz, CDCl₃) : δ ppm 157.9, 154.7, 137.2, 134.8, 129.0, 79.4, 58.1, 43.4, 35.3, 29.6, 28.4. Purity (100%), tr (HPLC) : 3.71 min.

### Step 2: 2-((1-benzylpiperidin-4-yl)methyl)-4-chloropyridazin-3(2H)-one

To a solution of tert-butyl 4-((5-chloro-6-oxopyridazin-1(6H)-yl)methyl)piperidine-1-carboxylate (1 g, 3.05 mmol, 1 equiv.) in DCM (15 mL) at 0 °C was added TFA (4.7 mL), and the resulting mixture was stirred at rt for 1.30 h. The crude reaction was concentrated under vacuum with azeotropic removal of TFA with heptane. The residue was triturated with ice-cooled ether, the supernatant was removed and the crude after evaporation was directly dissolved in MeOH (4 mL). Benzaldehyde (388 mg, 370 µL, 1.2 equiv.) was added, followed by NaBH₃CN (61.8 mg, 0.9 mmol, 2 equiv.) and DIEA (2.0 mL, 4 equiv.). The resulting mixture was stirred at rt for 16h. Volatiles were evaporated and the crude was taken up in EtOAc (25 mL). The organic phase was washed with brine, dried and concentrated under vacuum. The residue was purified by silica gel column chromatography using a gradient of 5% to 7% of MeOH (98: 2) in EtOAc yielding 2-((1-benzylpiperidin-4-yl)methyl)-4-chloropyridazin-3(2H)-one as a yellow gum (516 mg, 53%). ¹H NMR (400 MHz, CDCl₃) : δ ppm 7.64 (d, 1H, J = 4.4 Hz), 7.33 (d, 1H, J = 4.4 Hz), 7.30-7.22 (m, 5H), 4.10 (d, 2H), 3.5 (s, 2H), 2.90-2.87 (m, 2H), 2.00-1.95 (m, 3H), 1.64-1.56 (m, 2H), 1.49-1.37 (m, 2H). ¹³C NMR (101 MHZ, CDCl₃): δ ppm 157.9, 138.0, 137,1, 134.7, 129.2, 129.0, 128.2, 127.1, 63.2, 60.4, 58.3, 53.1, 35.0, 29.6.

### Step 3: 2-((1-benzylpiperidin-4-yl)methyl)-4-(4-fluorophenyl)pyridazin-3(2H)-one hydrochloride, (cpd 35).

A microwave vial (oven-dried and under argon) was charged with 4-fluorophenylboronic acid (48.4 mg, 0.32 mmol, 1.1 equiv.), 2-((1-benzylpiperidin-4-yl)methyl)-4-chloropyridazin-3(2H)-one (100 mg, 0.31 mmol, 1.0 equiv.), sodium carbonate 100 mg, 0.94 mmol, 3 equiv.). Tetrakis(triphenylphosphine) palladium(0) (18.2 mg, 5 mol %.) was then added, followed by toluene (1.0 mL), EtOH (0.2 mL) and H₂O (0.2 mL). The vial was properly capped and the mixture vessel was evacuated and backfilled with argon (process repeated 3 times) and heated at 120°C until complete conversion of the starting material. The reaction conversion was monitored by HPLC and was usually completed within 16 hours. After cooling to room temperature, the reaction mixture was evaporated to dryness. The crude was partitioned through EtOAc (30 mL) and H₂O (50mL). The aqueous phase was extracted twice with EtOAc (20 mL). The organic phases were combined, washed with brine and dried (Na₂SO₄) and evaporated. The crude material was purified by flash chromatography using DCM : MeOH (98: 2) as eluent to give the title compound as a orange gum (23.9 mg, 18 %) after salification with 2N HCl in Et₂O. ¹H NMR (400 MHz, CDCl₃) ¹H NMR (400 MHz, CDCl₃) : δ ppm 7.80-7.73 (m, 2H), 7.30-7.25 (m, 4 H), 7.23-7.17 (m, 3H), 7.09 (t, 2H, J = 8.6 Hz), 4.11 (d, 2H, J = 7.2 Hz), 3.48 (s, 2H), 2.85 (d, 2H, J = 11.5 Hz), 1.97-1.89 (m, 2H), 1.61 (d, 2H, J= 12.7 Hz), 1.42 (dq, 2H, J = 11.8 Hz, J = 5.5 Hz). ¹³C NMR (101 MHz, CDCl₃) : δ ppm 164.8, 160.3, 138.7, 136.6, 136.0, 130.9, 130.8, 129.3, 128.3, 127.2, 127.0, 115.7, 115.4, 63.4, 58.5, 53.3, 35.3, 30.0.
LC/MS (M+H) = 378

In an alternative method, a Sonogashira cross coupling reaction could be performed starting from the title intermediate of general structure E (see scheme 4). The resulting alkynes derivatives of general structure F (where R7 is an aryl group, such as phenyl) were hydrogenated using Pd/C as catalyst. Deprotection of the tert-butyloxycarbonyl group followed by alkylation, or reductive amination of the resulting piperidine derivatives gave examples of the present invention. A Suzuki-Miyaura cross-coupling reaction with the help of alkyl boronic acids could be an alternative way to introduce alkyl or aralkyl groups in positions 4 or 5 of the pyridazinones moieties. General conditions used are depicted in scheme 4.

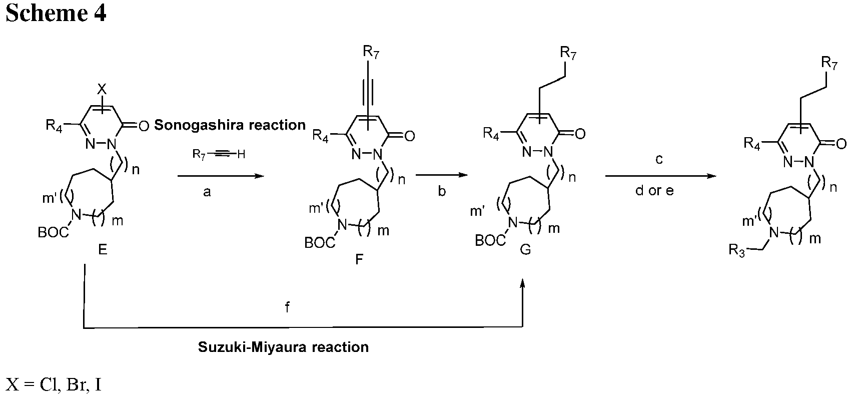

**Conditions** : a) Pd(PPh₃)₂Cl₂, CuI, Et₃N, MeCN, 80°C, 24 h; b) H₂, Pd/C, MeOH, 18h ; c) TFA, DCM, 45 min, 25°C ; d) R₃CHO, DIEA, NaBH₃CN, MeOH, 25°C, 18 h e) R₃-CH₂Br, K₂CO₃, DMF or MeCN, 80°C, 18h. f) R₇-(CH₂)₂ B(OH)₂ (1.5 equiv.), K₂CO₃ (3 equiv.), Pd(PPh₃)₄ (5 mol%), 1,4-dioxane, 100°C, 16h.

### Example A.10: 2-((1-benzylpiperidin-4-yl)methyl)-4-phenethylpyridazin-3(2H)-one, (cpd 36).

### Step 1: tert-butyl 4-((6-oxo-5-(phenylethynyl)pyridazin-1(6H)-yl)methyl)piperidine-1-carboxylate

A microwave vial (oven-dried and under argon) was charged with ethynylbenzene (40.5mg, 43 µL, 0.40 mmol, 1.3 equiv.), tert-butyl 4-((5-chloro-6-oxopyridazin-1(6H)-yl)methyl)piperidine-1-carboxylate (100 mg, 0.30 mmol, 1.0 equiv.), Et₃N (277.8 mg, 2.75 mmol, 9 equiv.). Bis(triphenylphosphine)palladium(II) chloride (6.42 mg, 3 mol%.) and CuI (2.9 mg, 5 mol %) was then added, followed by MeCN (1.5 mL). The vial was properly capped and the mixture vessel was evacuated and backfilled with argon (process repeated 3 times) and heated at 80°C until complete conversion of the starting material. The reaction conversion was monitored by HPLC and was usually completed within 24 hours. After cooling to room temperature, the reaction mixture was evaporated to dryness. The crude was partitioned through EtOAc (30 mL) and H₂O (50mL). The aqueous phase was extracted twice with EtOAc (20 mL). The organic phases were combined, washed with brine and dried (Na₂SO₄) and evaporated. The crude material was purified by flash chromatography using a gradient of 20 to 50% of EtOAc in Heptane to give the title compound as a light brown gum (85 mg, 71%). ¹H NMR (400 MHz, CDCl₃) : δ ppm 7.73 (d, 1H, J = 4.0 Hz), 7.60-7.56 (m, 2H), 7.40-7.32 (m, 4H), 4.17-3.92 (m, 4H), 2.75-2.60 (m, 2H), 2.19-2.14 (m, 1H), 1.66-1.54 (m,2H), 1.30-1.19 (m, 2H). ¹³C NMR (101 MHz, CDCl₃) : δ ppm 159.7, 154.9, 135.6, 132.3,132.0, 129.7, 128.5, 125.7, 125.6, 122.0, 100.8, 83.3, 79.5, 58.1, 57.940.3, 35.4, 29.7, 28.6

### Step 2 : tert-butyl 4-((6-oxo-5-phenethylpyridazin-1(6H)-yl)methyl)piperidine-1-carboxylate

Pd/C 10% (4.75 mg, 0.05 equiv.) and MeOH (1.5 mL) were placed into the reactor (2 mL capacity) equipped with a magnetic stirrer. tert-butyl 4-((6-oxo-5-(phenylethynyl)pyridazin-1(6H)-yl)methyl)piperidine-1-carboxylate (85 mg, 0.22 mmol, 1 equiv.) was added, and the micro-reactor was purged by flushing first with argon than four times with hydrogen at atmospheric pression. The resulting mixture was magnetically stirred overnight at room temperature. The catalyst particles were removed from the solution by filtration through celite, the volatiles were evaporated to give the title compound as a pale yellow gum (83 mg, 97%). The crude product was used directly without further purification. ¹H NMR (400 MHz, CDCl₃) : δ ppm 7.60 (d, 1H, 4 Hz), 7.31-7.22 (m, 2H), 7.21-7.16 (m, 3H), 6.86 (d, 1H , 4Hz), 4.19-4.00 (m, 4H), 2.95-2.90 (m, 4H), 2.76-2.62 (m, 1H), 1.65-1.57 (m, 2H), 1.34-1.24 (m, 2H). ¹³C NMR (101 MHz, CDCl₃) : δ ppm 161.5, 154.9, 143.1, 140.8, 135.6, 128.6, 128.5, 127.8, 126.3, 79.5, 57.2, 35.5, 33.5, 32.2, 29.8, 28.6.

### Step 3: 2-((1-benzylpiperidin-4-yl)methyl)-4-phenethylpyridazin-3(2H)-one hydrochloride, (cpd 36).

To a solution of tert-butyl 4-((6-oxo-5-phenethylpyridazin-1(6H)-yl)methyl)piperidine-1-carboxylate (83 mg, 0.21 mmol, 1 equiv.) in DCM (1 mL) at 0 °C was added TFA (0.32 mL), and the resulting mixture was stirred at rt for 1h. The crude reaction was concentrated under vacuum with azeotropic removal of TFA with heptane. The residue was triturated with ice-cooled ether, the supernatant was removed and the crude after evaporation was directly dissolved in MeOH (1 mL). Benzaldehyde (26.6 mg, 25 µL, 1.2 equiv.) was added followed by NaBH₃CN (26.2 mg, 0.42 mmol, 2 equiv.) and DIEA (145 µL, 4 equiv). The resulting mixture was stirred at rt for 16h. Volatiles were evaporated and the crude was taken up in EtOAc (25 mL). The organic phase was washed with brine, dried and concentrated under vacuum. The residue was purified by silica gel column chromatography using a gradient of 0% to 2% of MeOH in EtOAc yielding 2-((1-benzylpiperidin-4-yl)methyl)-4-phenethylpyridazin-3(2H)-one as a colorless foam after salification (36.5 mg, 41%). Tr (HPLC) = 8.11min ; ¹H NMR (400 MHz, CDCl₃) : δ ppm 7.59 (d, 1H, J= 4.0 Hz), 7.32-7.21 (m, 7H), 7.20-7.14 (m, 3H), 6.85(d, 1H, J= 4.0 Hz), 4.08 (d, 2H, J = 7.2 Hz), 3.51, (s, 2H), 2.93-2.87 (m, 6H), 2.05-1.91 (m, 3H), 1.59-1.46 (m, 2H), 1.48-1.44 (m, 2H). ). ¹³C NMR (101 MHz, CDCl₃) : δ ppm 161.5, 143.0, 140.9, 138.3, 135.7, 129.3, 128.6, 128.5, 128.3, 127.8, 127.1, 126.3, 63.4, 57.4, 53.3, 35.3, 33.5, 32.3, 29.9.
LC/MS (M+H) = 388.

### Example A.11: 2-(1-benzylpiperidin-4-yl)-4-(2-phenylethyl)-2,3-dihydropyridazin-3-one hydrochloride, (cpd 37).

### Step 1: tert-butyl 4-[6-oxo-5-(2-phenylethyl)-1,6-dihydropyridazin-1-yl]piperidine-1-carboxylate

A microwave vial (oven-dried and under argon) was charged with tert-butyl 4-(5-chloro-6-oxo-1,6-dihydropyridazin-1-yl)piperidine-1-carboxylate (200 mg, 0.64 mmol, 1.0 equiv.), (2-phenylethyl)boronic acid (143.4 mg, 0.96 mmol, 1.5 equiv.), K₂CO₃ (264.3 mg, 1.91 mmol, 3 equiv.). tetrakis(triphenylphosphine)palladium (37.2 mg, 5 mol %) followed by dioxane (2.2 mL). The vial was properly capped, and the mixture vessel was evacuated and backfilled with argon (process repeated 3 times) and heated at 100°C until complete conversion of the starting material. The reaction conversion was monitored by HPLC and was usually completed within 16 hours. After cooling to room temperature, the reaction mixture was evaporated to dryness. The crude was partitioned through EtOAc (30 mL) and H₂O (50 mL). The aqueous phase was extracted twice with EtOAc (20 mL). The organic phases were combined, washed with brine and dried (Na₂SO₄) and evaporated. The crude material was flash chromatography using a gradient of 0 % to 100% EtOAc in heptane to give the title compound as a colorless oil (66 mg, 27%). ¹H NMR (400 MHz, CDCl₃) : δ ppm 7.67 (d, 1H, J = 4.0 Hz), 7.32-7.25 (m, 2H), 7.22-7.17 (m, 3H), 6.88 (d, 1H, J = 4.0 Hz), 5.16-5.05 (m, 1H), 4.38-4.15 (m, 2H), 2.97- 2.88 (m, 6H), 1.96-1.84 (m, 4H), 1.82 (s, 9H). ¹³C NMR (101 MHz, CDCl₃) : δ ppm 161.0, 154.8, 142.6, 141.0, 136.0, 128.6, 127.4, 126.3, 79.8, 55.1, 33.5, 32.4, 30.4, 28.6.

### Step 2: 2-(1-benzylpiperidin-4-yl)-4-(2-phenylethyl)-2,3-dihydropyridazin-3-one hydrochloride, (cpd 37).

To a solution of tert-butyl 4-[6-oxo-5-(2-phenylethyl)-1,6-dihydropyridazin-1-yl]piperidine-1-carboxylate (88 mg, 0.23 mmol, 1 equiv.) in DCM (1 mL) at 0 °C was added TFA (0.32 mL), and the resulting mixture was stirred at rt for 1h. The crude reaction was concentrated under vacuum with azeotropic removal of TFA with heptane. The residue was triturated with ice-cooled ether, the supernatant was removed and the crude after evaporation was directly dissolved in MeOH (1 mL). Benzaldehyde (36.6 mg, 35 µL, 1.5 equiv.) was added, followed by NaBH₃CN (30.4 mg, 0.46 mmol, 2 equiv.) and AcOH (1-2 drops). The resulting mixture was stirred at rt for 16h. Volatiles were evaporated and the crude was taken up in EtOAc (25 mL). The organic phase was washed with brine, dried and concentrated under vacuum. The residue was purified by flash chromatography using a gradient of 0% to 5% of iPrOH in DCM yielding the title compound as a white solid after salification with HCl 2N in MeOH (52 mg, 55%). Tr (HPLC) = 8.03 min. ¹H NMR (free base, 400 MHz, CDCl₃) : δ ppm 7.67(d, 1H, J = 4.0 Hz), 7.36-7.19 (m, 10H), 6.86 (d, 1H, J = 4.0 Hz), 5.02-4.95 (m, 1H), 3.55 (s, 2H), 3.03-3.00 (d, 2H, J = 12.0 Hz), 2.96-2.86 (m, 4H), 2.18-2.10 (m, 4H), 2.05-1.83 (m, 2H). ¹³C NMR (101 MHz, CDCl₃) : δ ppm 161.1, 142.4, 141.1, 138.8, 135.8, 129.1, 128.6, 128.3, 127.3, 127.1, 126.3, 63.0, 55.5, 53.0, 33.5, 32.4, 30.6.
LC/MS (M+H) = 388

In alternative methods, a Buchwald-Hartwig cross coupling reaction with the help of Pd₂(dba)₃ and RuPhos can be performed starting from intermediates E or F previously described. Both routes, as outlined in scheme 5, provided examples of the present invention.

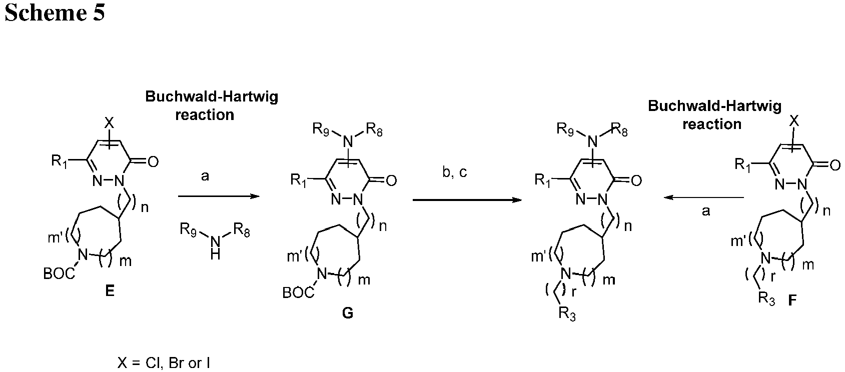

Where -NR₈R₉ forms a nitrogen-containing heterocycle, as defined above.

**Conditions** : a) tris((1E,4E)-1,5-diphenylpenta-1,4-dien-3-one) dipalladium (5 mol%), RuPhos (20 mol%), Cs₂CO₃, dioxane, 100°-120°C, 16h; b) TFA, DCM, 45 min, 25°C ; c) R₃CHO, DIEA, NaBH₃CN, MeOH, 25°C, 18 h.

### Example A.12: 2-(1-benzylpiperidin-4-yl)-4-(piperidin-1-yl)-2,3-dihydropyridazin-3-one hydrochloride, (cpd 38).

A microwave vial (oven-dried and under argon) was charged with 2-(1-benzylpiperidin-4-yl)-4-chloro-2,3-dihydropyridazin-3-one (50 mg, 0.16 mmol, 1.0 equiv.), piperidine (28.0 mg, 0.33 mmol, 2.0 equiv.), Cs₂CO₃ (135.4 mg, 0.41 mmol, 2.5 equiv.). Tris((1E,4E)-1,5-diphenylpenta-1,4-dien-3-one) dipalladium (7.7 mg, 5 mol%.) and RuPhos (15.3 mg, 20 mol%) was then added, followed by dioxane (1.5 mL). The vial was properly capped and the mixture vessel was evacuated and backfilled with argon (process repeated 3 times) and heated at 100°C until complete conversion of the starting material. The reaction conversion was monitored by HPLC and was usually completed within 16 hours. After cooling to room temperature, the reaction mixture was evaporated to dryness. The crude was partitioned through EtOAc (30 mL) and H₂O (50 mL). The aqueous phase was extracted twice with EtOAc (20 mL). The organic phases were combined, washed with brine and dried (Na₂SO₄) and evaporated. The crude material was purified by flash chromatography using a gradient of 1% to 5% MeOH in EtOAc to give the title compound as a light yellow solid after salification with 2M HCl in Et₂O (44 mg, 69%). Tr (HPLC) = 7.38min. ¹H NMR (400 MHz, DMSO-d6) : δ ppm 7.69 (d, 1H, J = 4.8 Hz), 7.32-7.24 (m, 5H), 6.41 (d, 1H, J = 4.8 Hz). 4.80-4.7 (m, 1H), 3.49 (s, 2H), 3.37-3.28 (m, 4H), 2.95-2.88 (m, 2H), 2.15-1.99 (m, 2H), 1.90-1.88 (m, 2H), 1.69-1.62 (m, 2H), 1.58-1.49 (m, 6H). ¹³C NMR (101 MHz, DMSO-d6) : δ ppm 158.0, 148.2, 139.0, 137.1, 129.1, 128.3, 127.0, 107.8, 63.0, 60.5, 55.5, 53.1, 49.2, 30.4, 25.6, 24.6, 21.2.
LC/MS (M+H) = 353.

### * 2-[(1-benzylpiperidin-4-yl)methyl]-4-(piperidin-1-yl)-2,3-dihydropyridazin-3-one hydrochloride (cpd 39).

Using the same procedure described in example A.12 and starting from 2-((1-benzylpiperidin-4-yl)methyl)-4-chloropyridazin-3(2H)-one (60 mg, 0.16 mmol, 1 equiv.) and piperidine (27.2 mg, 0.32 mmol, 2 equiv.) the title compound was obtained as a white solid (38%) after purification by flash chromatography using a gradient of 1% to 5% MeOH in EtOAc and salification. Tr (HPLC) = 7.41 min. ¹H NMR (400 MHz, CDCl₃) : δ ppm 7.50 (d, 1H, J= 4.4 Hz), 7.25-7.15 (m, 5H), 6.18 (d, 1H, J = 4.4 Hz), 3.98(d, 1H, J = 7.2 Hz), 3.43 (s, 2H), 3.35-3.32 (m, 4H), 2.81 (d, 2H, J = 12.0 Hz), 2.02- 1. 2.03-1.85 (m, 4H), 1.70-1.52 (m, 4H), 1.45-1.38 (m, 4H), 1.38-1.34 (m, 2H). ¹³C NMR (101 MHz, CDCl₃) : δ ppm 158.1, 148.5, 138.6, 136.8, 129.1, 128.1, 126.8, 107.7, 63.3, 57.6, 53.3, 49.1, 35.1, 30.0, 25.5, 24.4. LC/MS (M+H) = 367.

### Example A.13: 2-((1-benzylpiperidin-4-yl)methyl)-4-morpholinopyridazin-3(2H)-one hydrochloride (cpd 40).

### Step 1: tert-butyl 4-((5-morpholino-6-oxopyridazin-1(6H)-yl)methyl)piperidine-1-carboxylate

A microwave vial (oven-dried and under argon) was charged with tert-butyl 4-((5-chloro-6-oxopyridazin-1(6H)-yl)methyl)piperidine-1-carboxylate (100 mg, 0.30 mmol, 1.0 equiv.), morpholine (53.1 mg, 0.61 mmol, 53 µl, 2.0 equiv.), Cs₂CO₃ (298.2 mg, 0.91 mmol, 3.0 equiv.). Tris((1E,4E)-1,5-diphenylpenta-1,4-dien-3-one) dipalladium (14.0 mg, 5 mol %.) and RuPhos (28.5 mg, 20 mol %) was then added, followed by dioxane (1.5 mL). The vial was properly capped and the mixture vessel was evacuated and backfilled with argon (process repeated 3 times) and heated at 120°C until complete conversion of the starting material. The reaction conversion was monitored by HPLC and was usually completed within 16 hours. After cooling to room temperature, the reaction mixture was evaporated to dryness. The crude was partitioned through EtOAc (30 mL) and H₂O (50 mL). The aqueous phase was extracted twice with EtOAc (20 mL). The organic phases were combined, washed with brine and dried (Na₂SO₄) and evaporated. The crude material was purified flash chromatography using a DCM/MeOH 98/2 as eluent to give the title compound as a yellow gum (86.9 mg, 75%). ¹H NMR (400 MHz, CDCl₃) : δ ppm 7.59 (d, 1H, J = 4.8 Hz), 6.24 (d, 1H, J = 4.8 Hz), 4.14-3.96 (m, 4H), 3.87-3.82 (m, 4H), 3.44-3.37 (m, 4H), 2.73-2.59 (m, 2H), 2.19-2.07 (m, 1H), 1.60-1.54 (m, 2H), 1.44 (s, 9H), 1.31-1.18 (m, 2H). ¹³C NMR (101 MHz, DMSO-d6) : δ ppm 158.0, 154.9, 147.9, 136.9, 108.2, 79.4, 66.5, 57.3, 53.6, 48.2, 35.5, 29.8, 28.6.

### Step 2: 2-((1-benzylpiperidin-4-yl)methyl)-4-morpholinopyridazin-3(2H)-one hydrochloride, (cpd 40).

To a solution of tert-butyl 4-((5-morpholino-6-oxopyridazin-1(6H)-yl)methyl)piperidine-1-carboxylate (86 mg, 0.21 mmol, 1 equiv.) in DCM (1.5 mL) at 0 °C was added TFA (0.35 mL), and the resulting mixture was stirred at rt for 1 h. The crude reaction was concentrated under vacuum with azeotropic removal of TFA with heptane. The residue was triturated with ice-cooled ether, the supernatant was removed and the crude after evaporation was directly dissolved in MeOH (4 mL). Benzaldehyde (36.3 mg, 34.5 µL, 1.5 equiv.) was added followed NaBH₃CN (28.6 mg, 0.46 mmol, 2 equiv.) and DIEA (117.8 mg, 158 µl, 4 equiv.). The resulting mixture was stirred at rt for 48h. Volatiles were evaporated and the crude was taken up in EtOAc (25 mL). The organic phase was washed with brine, dried and concentrated under vacuum. The residue was purified by silica gel column chromatography using EtOAc/MeOH (97: 3) as eluent to yield 2-((1-benzylpiperidin-4-yl)methyl)-4-morpholinopyridazin-3(2H)-one hydrochloride as a pale yellow solid after salification with 2N HCl in Et₂O (63.8 mg, 63 %).Tr (HPLC, short column) = 2.42 min ..¹H NMR (400MHz, CDCl₃) : δ ppm 7.58 (d, 1H, J = 5.2 Hz), 7.33-7.21 (m, 5H), 6.23 (d, 1H, J= 2.0 Hz), 4.03 (d, 2H, J = 7.2 Hz), 3.89-3.81 (m, 4H), 3.52 (s, 2H), 3.44-3.37 (m, 4H), 2.93-2.85 (m, 2H), 2.05-1.95 (m, 3H), 1.67-1.57 (m, 2H), 1.52-1.38 (m, 2H). ¹³C NMR (101 MHz, CDCl₃) : δ ppm 157.9, 147.8, 137.8, 136.8, 129.4, 128.3, 127,3 108.2, 66.5, 63.2, 57.5, 53.2, 48.2, 35.1, 29.8; LC/MS (M+H) = 369.

### * 2-((1-benzylpiperidin-4-yl)methyl)-4-(4-phenylpiperazin-1-yl)pyridazin-3(2H)-one hydrochloride (cpd 41)

### Step 1: tert-butyl 4-((6-oxo-5-(4-phenylpiperazin-1-yl)pyridazin-1(6H)-yl)methyl)piperidine-1-carboxylate

Using the same procedure described in example A.13 and starting from tert-butyl 4-((5-chloro-6-oxopyridazin-1(6H)-yl)methyl)piperidine-1-carboxylate (100 mg, 0.30 mmol, 1 equiv.) and 1-phenylpiperazine (99.0 mg, 93 µl, 0.6 mmol, 1.5 equiv.), the title compound was obtained as a yellow gum (78.6 mg, 57%). ¹H NMR (400 MHz, CDCl₃) : δ ppm 7.61 (d, 1H, J = 4.0 Hz), 7.29 (t, J = 2H, J = 6.4 Hz), 6.96 (d, 1H, J = 6.4 Hz), 6.90 (t, 1H, J = 6.0 Hz), 6.31 (d, 1H, J = 4.0 Hz), 4.10-4.04 (m, 2H), 3.62-3.58 (m, 4H), 3.56-3.32 (m, 4H), 2.72-2-61 (m, 2H), 2.19-2.13 (m, 1H), 1.62-1.58 (m, 2H), 1.44 (s, 9H), 1.32-1.21 (m, 3H). ¹³C NMR (101 MHz, CDCl₃) : δ ppm 158.0, 154.9, 151.1, 147.9, 137.0, 129.3, 120.5, 116.6, 108.5, 79.4, 57.4, 53.6, 49.1, 47.8, 35.5, 29.8, 28.6

### Step 2: 2-(1-benzylpiperidin-4-yl)-4-(4-phenylpiperazin-1-yl)pyridazin-3(2H)-one hydrochloride, (cpd 41).

Starting from tert-butyl 4-((6-oxo-5-(4-phenylpiperazin-1-yl)pyridazin-1(6H)-yl)methyl)piperidine-1-carboxylate (87 mg, 0.17 mmol, 1 equiv.) a sequential Boc-deprotection followed by reductive amination using benzaldehyde (27.48 mg, 26.2 µl, 0.25 mmol, 1.5 equiv.) as described in example A.13 led to the title product, after salification with HCl 2N, as a white solid (68 mg, 71%). ¹H NMR (400 MHz, CDCl₃) : δ ppm 7.0 (d, 1H, J = 4.8 Hz), 7.32-7.24 (m, 7H), 6.96 (d, 2H, J = 8.0 Hz). 6.90 (t, 1H, J = 8.0 Hz), 6.30 (d, 1H, J = 4.8 Hz), 4.06 (d, 2H, J = 7.2 Hz), 3.61-3.58 (m, 4H), 3.53 (s, 2H), 3.35-3.57 (m, 4H), 2.93-2.88 (m, 2H), 2.04-1.97 (m, 3H), 1.66-1.63 (m, 2H), 1.51-1.40 (m, 2H). ¹³C NMR (101 MHz, CDCl₃) : δ ppm 158.0, 151.2, 147.8, 137.8, 136.8, 129.4, 129.3, 128.3, 127.3, 120.4, 116.5, 108.5, 63.2, 57.6, 53.2, 49.1, 47.8, 35.1, 29.8. LC/MS (M+H) = 444.

### * 2-((1-benzylpiperidin-4-yl)methyl)-4-(3,4-dihydroisoquinolin-2(1H)-yl)pyridazin-3(2H)-one hydrochloride, (cpd 42).

### Step 1: tert-butyl 4-((5-(3,4-dihydroisoquinolin-2(1H)-yl)-6-oxopyridazin-1(6H)-yl)methyl)-piperidine-1-carboxylate

Using the same procedure described in example A.13 and starting from tert-butyl 4-((5-chloro-6-oxopyridazin-1(6H)-yl)methyl)piperidine-1-carboxylate (100 mg, 0.30 mmol, 1 equiv.) and 1,2,3,4-tetrahydroisoquinoline (81.2 mg, 76.6 µl, 0.6 mmol, 1.5 equiv.), the title compound was obtained as a yellow gum (97 mg, 75%). ¹H NMR (500 MHz, CDCl₃) : δ ppm 7. 57(d, 1H, J = 5.0 Hz), 7.20-7.11(m, 4H), 6.27 (d, 1H, J = 5.0 Hz), 4.59 (s, 2H), 4.10-4.03 ( (m, 4H), 3.90 (t, 2H, J = 6.0 Hz), 3.01 (t, 2H, J = 6.0 Hz), 2.71-2.61 (m, 2H), 2.18-2.13 (m, 1H), 1.65-1.57 (m, 2H), 1.43 (s, 9H), 1.40-1.21 (m, 2H). ¹³C NMR (125 MHz, CDCl₃) : δ ppm 158.0, 154.9, 146.9, 137.3, 134.7, 133.3, 128.9, 126.8, 126.5, 126.3, 106.5, 79.4, 57.4, 50.4, 45.7, 35.5, 29.8, 29.7, 28.8.

### Step 2 2-(1-benzylpiperidin-4-yl)-4-(4-phenylpiperazin-1-yl)pyridazin-3(2H)-one hydrochloride, (cpd 42).

Starting from tert-butyl 4-((5-(3,4-dihydroisoquinolin-2(1H)-yl)-6-oxopyridazin-1(6H)-yl)methyl)piperidine-1-carboxylate (97 mg, 0.23 mmol, 1 equiv.) a sequential Boc-deprotection followed by reductive amination using benzaldehyde (34.0 mg, 32.4 µl, 0.32 mmol, 1.5 equiv.) as described in example A.13 led to the title product, after salification with HCl 2N, as a yellow solid (69 mg, 66%). ¹H NMR (400 MHz, CDCl₃) : δ ppm 7.56 (d, 1H, J = 5.2 Hz), 7.32-7.10 (m, 9H), 6.25 (d, 1H, J = 5.2 Hz), 4.58 (s, 2H), 4.05 (d, 2H, J = 6.8 Hz), 3.90(t, 2H, J = 6.0 Hz), 3.52 (s, 2H), 3.00 (t, 2H, J = 6.0 Hz), 2.92-2.86 (m, 2H), 2.89 (dt, 2H, J = 8.0 Hz, J =3.2 Hz), 2.04-1.96 (m, 2H), 1.68-1.60 (m, 2H), 1.45 (dq, 2H, J = 12.0 Hz, J = 4.0 Hz). ¹³C NMR (125 MHz, CDCl₃) : δ ppm 158.0, 147.0, 137.1, 134.7, 133.4, 129.3, 128.9, 128.3, 127.2, 126.8, 126.5, 126.3, 106.6, 63.3, 57.6, 53.3, 50.4, 45.7, 35.1, 29.9, 28.9. LC/MS (M+H) = 415.

### General methods and examples deriving from pyridones and pyrimidones.

Alternatively, the pyridazinones ring can be replaced by a pyridone or pyrimidone ring of general structure **H**, in a similar 4-step sequence as depicted in the following scheme 6.

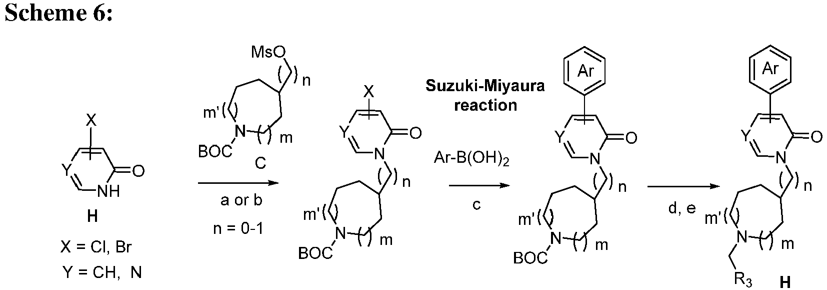

**Conditions** : a) n = 0, K₂CO₃, 2-butanone, 85°C, 24h ; b) NaH, DMF, 110°C, 18h ; c) K₂CO₃, Pd(PPh₃)₄, Ar-PhB(OH)₂, Toluene, EtOH, H₂O, 120°C, 16h; d) TFA, DCM, 25°C, 45 min; e) NaBH₃CN; DIEA, MeOH, 25°C, 18h or R₃-CH₂Br, K₂CO₃, DMF or MeCN, 80°C, 18h.

### Example A.14: 3-(1-benzylpiperidin-4-yl)-5-phenylpyrimidin-4(3H)-one hydrochloride (cpd 43).

### Step 1: tert-butyl 4-(6-oxo-5-phenylpyrimidin-1(6H)-yl)piperidine-1-carboxylate

A microwave vial (oven-dried and under argon) was charged with 5-bromopyrimidin-4-ol hydrobromide (345 mg, 1.35 mmol, 1.0 equiv), tert-butyl 4-(methanesulfonyloxy)piperidine-1-carboxylate (564.9 mg, 2.02 mmol, 1.5 equiv.) and K₂CO₃ (462.5 mg, 3.36 mmol, 2.5 equiv.). 2-butanone (12.3 mL) was added and the mixture was heated at 110°C for 24 h (HPLC monitoring: complete conversion). The mixture was quenched with H₂O (150 mL) and extracted with EtOAc (3 x 40 mL). The combined organic layers were washed with brine (2 x 50 mL), dried over Na₂SO₄, filtered and evaporated to dryness. The resulting residue was purified by silica gel flash chromatography using a gradient of 0% to 50% of EtOAc in heptane to give the tert-butyl 4-(6-oxo-5-phenyl-1,6-dihydropyrimidin-1-yl)piperidine-1-carboxylate as an yellow oil (191 mg, purity ~70%). The compound was used without further purification.

A microwave vial (oven-dried and under argon) was charged with tert-butyl 4-(6-oxo-5-phenyl-1,6-dihydropyrimidin-1-yl)piperidine-1-carboxylate (191 mg, 0.53 mmol, 1.0 equiv.), Phenylboronic acid (102.6 mg, 0.80 mmol, 1.5 equiv.), Na₂CO₃ (113 mg, 1.06 mmol, 2.0 equiv.). Tetrakis(triphenylphosphine)palladium (62.3 mg, 5 mol %, 0.1 equiv.) was then added, followed by DME (2.8 mL) and water (0.85 mL). The vial was properly capped and the mixture vessel was evacuated and backfilled with argon (process repeated 3 times) and heated at 100°C until complete conversion of the starting material. The reaction conversion was monitored by HPLC and was usually completed within 16 hours. After cooling to room temperature, the reaction mixture was evaporated to dryness. The crude was partitioned through EtOAc (30 mL) and H₂O (50 mL). The aqueous phase was extracted twice with EtOAc (20 mL). The organic phases were combined, washed with brine and dried (Na₂SO₄) and evaporated. The crude material was purified by flash chromatography using a gradient of 0% to 40% of EtOAc in Heptane to give the tert-butyl 4-(6-oxo-5-phenylpyrimidin-1(6H)-yl)piperidine-1-carboxylate (108 mg, purity of 86% ). ¹H NMR (400 MHz, CDCl₃) : δ ppm 8.72 (s, 1H), 8.51 (s, 1H), 7.60-7.50 (m, 2H), 7.46-7.42 (m, 3H), 5.49-5.44 (m, 1H), 3.60-3.52 (m, 2H), 3.44-3.39 (m, 2H), 2.00-1.95 (m, 2H), 1.86-1.82 (m, 2H), 1.46 (s, 9H).

### Step 2: 3-(1-benzylpiperidin-4-yl)-5-phenylpyrimidin-4(3H)-one hydrochloride, (cpd 43)

To a solution of tert-butyl 4-(6-oxo-5-phenylpyrimidin-1(6H)-yl)piperidine-1-carboxylate (108 mg, 0.30 mmol, 1 equiv.) in DCM (1.3 mL) at 0 °C was added TFA (1.3 mL), and the resulting mixture was stirred at rt for 1 h. The crude reaction was concentrated under vacuum with azeotropic removal of TFA with heptane. The residue was triturated with ice-cooled ether, the supernatant was removed and the crude after evaporation was directly dissolved in MeOH (2.6 mL). Benzaldehyde (48.4mg, 46.1 µL, 1.5 equiv.) was added followed NaBH₃CN (40.22 mg, 0.6 mmol, 2 equiv.) and AcOH (1.7 µl). The resulting mixture was stirred at rt for 16h. Volatiles were evaporated and the crude was taken up in EtOAc (25 mL). The organic phase was washed with brine, dried and concentrated under vacuum. The residue was purified by silica gel column chromatography using DCM/iPrOH (92/8) as eluent to yield 3-(1-benzylpiperidin-4-yl)-5-phenylpyrimidin-4(3H)-one hydrochloride as a white solid after salification with 2N HCl in Et₂O (41 mg, 35%). Mp 144°C, Tr (HPLC) = 7.14min.. ¹H NMR (400 MHz, CDCl₃, free base) : δ ppm 8.72 (s, 1H), 8.50 (s, 1H), 7.60-7.55 (m, 2H), 7.48-7.40 (m, 3H), 7.35-7.26 (m, 5H), 5.36-5.32 (m, 1H), 3.54 (s, 2H), 2.75-2.60 (m, 2H), 2.45-2.30 (m, 2H), 2.10- 2.04 (m, 2H), 1.95-1.85 (m, 2H). ¹³C NMR (101 MHz, CDCl₃) : δ ppm 165.3, 157, 0, 156.3, 138.3, 133.2, 129.2, 129.1, 128.5, 128.3, 127.2, 122.5, 72.1, 63.1, 50.5, 30.7.
LC/MS (M+H) = 346.

### * 1-(1-benzylpiperidin-4-yl)-3-phenyl-1,2-dihydropyridin-2-one hydrochloride, (cpd 44)

Under the same procedure described in example A.14 and starting from 3-bromopyridin-2-ol (300 mg, 1.27 mmol, 1 equiv.) the title compound was obtained after salification with 2N HCL as a white solid (45 mg, 11% overall yield). Mp = 152°C, Tr (HPLC) = 7.21min ¹H NMR (400 MHz, CDCl₃, free base) : δ ppm 7.67 (d, 2H, J = 7.6 Hz), 7.46-7.26 (m, 10H), 6.31 (t, 1H, J = 6.8 Hz), 5.07-5.01 (m, 1H), 3.57 (s, 2H), 3.06 (d, 2H, J = 11.6 Hz), 2.24(t, 2H, J= 11.6 Hz), 1.97-1.85 (m, 4H). ¹³C NMR (101 MHz, CDCl₃) : δ ppm 161.3, 138.2, 137.3, 137.0, 132.5, 131.6, 129.3, 128.8, 128.4, 128.1, 127.7, 127.3, 62.9, 53.1, 52.9, 31.7.
LC/MS (M+H) = 345.

### * 3-((1-benzylpiperidin-4-yl)methyl)-5-phenylpyrimidin-4(3H)-one, (cpd 45).

Under the same procedure described in example A.14 and starting from 5-bromopyrimidin-4-ol hydrobromide (200 mg, 0.78 mmol, 1 equiv.) and tert-butyl 4-[(methanesulfonyloxy)methyl] piperidine-1-carboxylate (298.1 mg, 1.01 mmol, 1.3 equiv.), tert-butyl 4-[(5-bromo-6-oxo-1,6-dihydropyrimidin-1-yl)methyl]piperidine-1-carboxylate was obtained after flash chromatography on silica gel (Hept/EtOAc) as a yellow oil (51.7 mg, 18%). ¹H NMR (400 MHz, CDCl₃) : δ ppm 8.63 (s, 1H), 8.57 (s, 1H), 4.29 (d, 2H, J = 6.8 Hz), 4.21-4.04 (m, 2H), 2.78-2.71 (m, 2H), 2.04-2.00 (m, 1H), 1.80 (d, 2H, J = 12.8 Hz), 1.40 (s, 9H), 1.31-1.25 (m, 2H).

Starting from tert-butyl 4-[(5-bromo-6-oxo-1,6-dihydropyrimidin-1-yl)methyl]piperidine-1-carboxylate (73 mg, 0.19 mmol, 1 equiv.) a Suzuki Miyaura cross coupling reaction with the help of phenylboronic acid (26.3 mg, 0.21 mmol, 1.1 equiv.), tert-butyl 4-[(6-oxo-5-phenyl-1,6-dihydropyrimidin-1-yl)methyl]piperidine-1-carboxylate was obtained after after flash chromatography on silica gel (Hept/EtOAc) as a yellow oil (32.7 mg, 45%). ¹H NMR (400 MHz, CDCl₃) : δ ppm 8.72 (s, 1H), 8.49 (s, 1H), 7.54-7.47 (m, 2H), 7.45-7.39 (m, 3H), 4.29 (d, 2H, J = 6.4 Hz), 4.13-4.07 (m, 2H), 2.73-2.69 (m, 2H), 1.99-1.96 (m, 1H), 1.75-1.71 (m, 2H), 1.46 (s, 9H), 1.26-1.23 (m, 2H). ¹³C NMR (101 MHZ, CDCl₃) : δ ppm 157.0, 156.2, 154.8, 128.9, 128.5, 128.3, 79.4, 70.9, 35.6, 28.8, 28.5, 28.4.

After deprotection of the BOC group with the help of TFA, the crude was submitted to a reductive amination reaction with the help of benzaldehyde leading to the title compound after salification with 2N HCl. (light yellow solid, 18.6 mg, 54%). ¹H NMR (400 MHz, CDCl₃) : δ ppm 8.65 (s, 1H), 8.42 (s, 1H), 7.47-7.45 (m, 2H), 7.40-7.30 (m, 3H), 7.28-.7.21 (m, 5H), 4.23 (d, 1H, J = 6.7 Hz), 3.56 (s, 2H), 2.95 (d, 2H, J = 11.3 Hz), 2.12-2.00 (m, 2H), 1.86-1.78 (m, 1H), 1.72 (d, 2H, J = 13.5Hz), 1.42 (q, 2H, J = 12.5 Hz). ¹³C NMR (101 MHz, CDCl₃) : δ ppm 165.5, 156.9, 156.1, 133.0, 129.6, 128.9, 128.5, 128.4, 128.3, 127.6, 122.3, 70.9, 62.8, 52.8, 34.9, 28.3.
LC/MS (M+H) = 360.

### * 1-[(1-benzylpiperidin-4-yl)methyl]-3-phenyl-1,2-dihydropyridin-2-one hydrochloride, (cpd 46).

Under the same procedure described in example A.14 and starting from 3-bromopyridin-2-ol (180 mg, 1.035 mmol, 1 equiv.) and tert-butyl 4-[(methanesulfonyloxy)methyl]piperidine-1-carboxylate (364.2 mg, 1.24 mmol, 1.2 equiv.), tert-butyl 4-[(3-bromo-2-oxo-1,2-dihydropyridin-1-yl)methyl]piperidine-1-carboxylate was obtained after flash chromatography on silica gel (Hept/EtOAc) as a white solid (133.5 mg, 35%). ¹H NMR (400 MHz, CDCl₃) : δ ppm 8.07 (d, 1H, J = 4.3 Hz), 7.8 (d, 1H, J = 7.5 Hz), 6.76 (t, 1H, J = 6.2 Hz), 4.21 (d, 2H J = 6.8 Hz), 4.19-4.09 (m, 2H), 2.75 (t, 2H, J = 12.7 Hz), 2.09-1.95 (m, 1H), 1.83 (d, 2H, J= 13.3 Hz) 1.47 (s, 9H), 1.3 (dq, 2H, J = 12.7 Hz, J = 3.8 Hz). ¹³C NMR (101 MHz, CDCl₃) : δ ppm 159.8, 154.9, 154.4, 141.6, 117.8, 107.3, 79.3, 71.0, 35.8, 28.8, 28.5.

Starting from tert-butyl 4-[(3-bromo-2-oxo-1,2-dihydropyridin-1-yl)methyl]piperidine-1-carboxylate (123 mg, 0.33 mmol, 1 equiv.) a Suzuki Miyaura cross coupling reaction with the help of phenylboronic acid (44.4 mg, 0.36 mmol, 1.1 equiv.), tert-butyl 4-[(2-oxo-3-phenyl-1,2-dihydropyridin-1-yl)methyl]piperidine-1-carboxylate was obtained after flash chromatography on silica gel (Hept/EtOAc) as a yellow oil (100 mg, 82%). ¹H NMR (40 0MHz, CDCl₃) : δ ppm 7.97 (dd, 1H, J = 4.1 Hz, J = 1.3 Hz), 7.46 (dd, J = 7.6 Hz, J = 1.3 Hz), 7.42-7.35 (m, 2H), 7.30-7.23 (m, 2H), 7.22-7.16 (m, 1H), 6.81(dt, 1H, J = 6.3 Hz, J = 1.3 Hz), 4.05 (d, 2H, J = 6.2 Hz), 4.02-3.88 (m, 2H), 2.55 (t, 2H, J = 12.7 Hz), 1.85-1.64 (m, 1H), 1.58 (d, 2H, J = 13.2 Hz), 1.31 (s, 9H), 1.15-1.00 (m, 2H). ¹³C NMR (101 MHz, CDCl₃) : δ ppm 160.6, 154.8, 145.7, 138.7, 136.8, 129.1, 128.1, 127.4, 124.7, 117.1, 79.3, 70.3, 35.9, 29.0, 28.5.

After deprotection of the BOC group with the help of TFA, the crude was submitted to a reductive amination reaction with the help of benzaldehyde leading to the title compound **46** after salification with 2N HCl. (light yellow solid, 44.4 mg, 55%). ¹H NMR (400 MHz, CDCl₃) : δ ppm 8.08 (dd, 1H, J = 4.4 Hz, J = 1.3 Hz), 7.57 (dd, 1H, J = 7.2 Hz, J = 1.3 Hz), 7.53-7.47 (m, 2H), 7.40-7.33 (m, 2H), 7.32-7.25 (m, 5H), 7.22-7.15 (m, 1H), 6.91 (dt, 1H, J = 6.9 Hz, J = 1.3 Hz), 4.15 (d, 2H, J = 6.7 Hz), 3.45( s, 2H), 2.85 (d, 2H, J = 11.5 Hz), 1.92 (t, 2H, J = 12.1 Hz), 1.80-1.62 (m, 3H), 1.34 (q, 2H, J = 12.4 Hz). ¹³C NMR (101 MHz, CDCl₃) : δ ppm 160.7, 145.7, 138.6, 136.8, 129.2, 128.1, 127.4, 126.9, 124.6, 116.9, 70.7, 63.4, 53.4, 35.6, 29.2.
LC/MS (M+H) = 359.

### B. In Vitro Pharmacology: Binding Assays to sigma-1 receptors

The sigma-1 receptor binding assays were performed according to Ganapathy et al. (J Pharmacol Exp Ther 28:251-60, 1999). The sigma-1 receptor binding assay was carried out by incubing Jurkat cell membranes (10-20 mg protein per tube) with [³H](+)-pentazocine a selective sigma-1 radiotracer (15 nM) and a range of concentration of tested compounds, at 37°C for 2 hours in 5 mM tris/HCl buffer (pH = 7.4). The inhibition of [³H](+)-pentazocine binding assay is mainly used to determine the inhibition constant (Ki) of potential sigma-1 receptor ligands. These assays are performed with a single concentration of [³H](+)-pentazocine at a concentration near its K_{D} and increasing concentrations of non-radioactive ligands.

**Table 1**

| **Assay** | **Source** | **Ligand** | **Cone** | **Kd** | **Non specific** | **Incubation** | **Detection method** |
|---|---|---|---|---|---|---|---|
| **Receptors** | | | | | | | |
| **sigma-1 (h) (agonist radioligand)** | Jurkat cell (endogenous | [³H](+)-pentazocine | 15 nM | 16 nM | Haloperidol (10 µM) | 120 min 37°C | Scintillation counting |
| **sigma-1 (h) (agonist radioligand)** | Jurkat cells (endogenous) | [³H]DTG (+1 µM (+)-Pentazocine) | 25 nM | 80.84 nM | Haloperidol (10 µM) | 60min RT | Scintillation counting |

The results of the sigma-1 receptor binding assays are shown in Table 2.

**Table 2**

| | sigma-1 receptor **% inhibition** | |
|---|---|---|
| **Cpds** | 100 nM | 10 nM |
| **1** | 97.1 | 59.9 |
| **2** | 90 | 86.0 |
| **3** | | 41.9 |
| **4** | | 13.5 |
| **5** | | 60.7 |
| **6** | | 81.4 |
| **8** | | 31.5 |
| **9** | | 38.8 |
| **10** | | 29.2 |
| **11** | | 3.9 |
| **12** | | 73.1 |
| **13** | | 59.3 |
| **14** | | 16.4 |
| **15** | | 19.3 |
| **16** | | 3.8 |
| **18** | | 16.7 |
| **20** | | 18.9 |
| **23** | | 1.3 |
| **24** | | 6.2 |
| **25** | | 15.3 |
| **26** | 95 | 80.9 |
| **27** | | 61.7 |
| **28** | | 37 |
| **29** | | 10.6 |
| **30** | 75.8 | |
| **31** | 80.0 | |
| **32** | 91.1 | |
| **33** | | 51.2 |
| **35** | | 44.6 |
| **36** | | 44.2 |
| **37** | 100 | 90 |
| **38** | 90 | 52.3 |
| **39** | | 51.8 |
| **40** | | 8.2 |
| **41** | | 5.2 |
| **42** | | 25.4 |
| **43** | 90 | 86.0 |
| **44** | | 29.6 |
| **45** | | 75.7 |
| **46** | | 75.6 |

## Claims

1. A compound having the following formula (I): 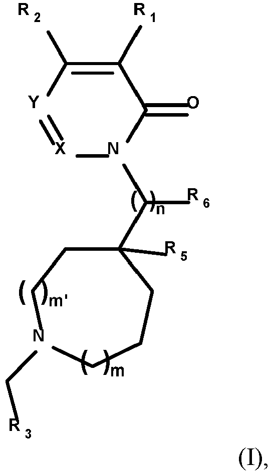 wherein:
R₁ represents:
• H;
• an aryl(C₁-C₆)alkyl group;
• an aryl group;
• a cycloalkyl group;
• a heterocyclic group; or
• SR, R being alkyl, aryl or aralkyl;
R₂ represents:
• H;
• an aryl(C₁-C₆)alkyl group;
• an aryl group;
• a cycloalkyl group; or
• SR, R being alkyl, aryl or aralkyl;
wherein one of R₁ and R₂ is H, and the other one of R₁ and R₂ is different from H;
X and Y are either, respectively:
• CH and N, or
• N and CR₄, or
• CH and CR₄,
wherein R₄ represents H or a (C₁-C₄)alkyl;
n is 0, 1, or 2; m is 0 or 1; m' is 0 or 1;
R₃ represents:
• a radical selected from the group consisting of H, (C₁-C₆)alkyl, (C₂-C₆)alkenyl, aryl, heteroaryl, cycloalkyl, heterocycloalkyl, (C₁-C₆)alkoxy, and aryl(C₁-C₆)alkyl said radical being optionally substituted by at least one group chosen among (C₁-C₆)alkyl, OH, halogen, aryl, heteroaryl, cycloalkyl, heterocycloalkyl, (C₁-C₆)alkoxy, - C(O)R, -CHOHR, C(O)₂R, C(O)NRR', -CONHOR, -CONHSO₂R, -NRR', - N(R)C(O)R', -N(R)NR'R", -N(R)C(O)₂R', -N(R)C(O)NR'R", -N(R)S(O)₂R', -SR, - S(O)R, -S(O)₂R, -S(O)NRR', and -S(O)₂NRR'; R, R', and R" being independently H, (C₁-C₆)alkyl, cycloalkyl, aryl, heterocycloalkyl, heteroaryl, (C₁-C₆)alkylcycloalkyl, (C₁-C₆)alkylaryl, (C₁-C₆)alkylheterocycloalkyl), or (C₁-C₆)alkylheteroaryl; or
• a radical selected from the group consisting of OH, -C(O)R, -CHOHR, C(O)₂R, C(O)NRR', -CONHOR, -CONHSO₂R, -NRR', -N(R)C(O)R', -N(R)NR'R", - N(R)C(O)₂R', -N(R)C(O)NR'R", -N(R)S(O)₂R', -SR, -S(O)R, -S(O)₂R, -S(O)NRR', and -S(O)₂NRR'; R, R', and R" being independently H, (C₁-C₆)alkyl, cycloalkyl, aryl, heterocycloalkyl, heteroaryl, (C₁-C₆)alkylcycloalkyl, (C₁-C₆)alkylaryl, (C₁-C₆)alkylheterocycloalkyl), or (C₁-C₆)alkylheteroaryl;
R₅ represents H or OH; and
each of R₆ represents independently H or a (C₁-C₆)alkyl group;
a stereoisomer, a solvate or any pharmaceutical salt thereof.

2. The compound according to claim 1, wherein:
R₁ represents:
• an aryl(C₁-C₆)alkyl group;
• an aryl group;
• a cycloalkyl group;
• a heterocyclic group; or
• SR, R being alkyl, aryl or aralkyl;
and R₂ is H.

3. The compound according to claim 1 or 2, wherein:
R₁ represents:
• an aryl(C₁-C₆)alkyl group;
• an aryl group; or
• a heterocyclic group;
preferably R₁ represents an aryl group; and
R₂ represents H.

4. The compound according to any one of claims 1 to 3, wherein X and Y are N and CR₄, respectively, wherein R₄ is H or a (C₁-C₄)alkyl, preferably H or methyl, more preferably H.

5. The compound according to any one of claims 1 to 4, wherein R₅ and each R₆ are H.

6. The compound according to any one of claims 1 to 5, wherein at least one of the following features is fulfilled:
n is 1; and/or
m is 0; and/or
m' is 1; and/or
R₁ represents an aryl group; and/or
R₂ is H; and/or
R₃ represents:
• a radical selected from H, an aryl (e.g. phenyl), a (C₁-C₆)alkyl (e.g. ethyl), a (C₂-C₆)alkenyl (e.g. isobutenyl), a cycloalkyl (e.g. cyclopropyl, cyclopentyl, cyclohexyl), a heterocycloalkyl (e.g. tetrahydropyranyl), a heteroaryl (e.g. pyridyl, imidazolyl), (C₁-C₆)alkoxy (e.g. methoxy), aryl(C₁-C₆)alkyl (e.g. benzyl)
said radical being optionally substituted by one or two (C₁-C₆)alkyl, OH, halogen (e.g. fluorine, chlorine), aryl (e.g. phenyl), heteroaryl, cycloalkyl, heterocycloalkyl, (C₁-C₆)alkoxy, -C(O)₂R, -N(R)C(O)R' ; R and R' being independently H, (C₁-C₆)alkyl, cycloalkyl, aryl (such as a phenyl optionally substituted by a halogen (preferably a fluorine)), heterocycloalkyl, heteroaryl, (C₁-C₆)alkylcycloalkyl, (C₁-C₆)alkylaryl, (C₁-C₆)alkylheterocycloalkyl), or (C₁-C₆)alkylheteroaryl; or
a radical selected from the group consisting of -CHOHR, -C(O)R, -C(O)₂R ; R being H, (C₁-C₆)alkyl, cycloalkyl, aryl, heterocycloalkyl, heteroaryl, (C₁-C₆)alkylcycloalkyl, (C₁-C₆)alkylaryl, (C₁-C₆)alkylheterocycloalkyl), or (C₁-C₆)alkylheteroaryl; and/or
R₅ is H; and/or
each R₆ is H.

7. The compound according to any one of claims 1 to 6, wherein the aryl group is a phenyl group optionally substituted by at least one substituent, said at least one substituent being selected from:
• a halogen, preferably a fluorine or a chlorine;
• an alkyl optionally substituted by at least one halogen, preferably a fluorine, preferably - CF₃;
• -OH; and
• -OR, R being an alkyl, cycloalkyl, heterocycloalkyl, aryl or heteroaryl, preferably an alkyl, more preferably a methyl.

8. The compound according to any one of claims 1 to 7, wherein it is selected in the group consisting of:
2-(1-benzylpiperidin-4-yl)-4-phenylpyridazin-3(2H)-one hydrochloride;
2-(1-benzylpiperidin-4-yl)-5-phenylpyridazin-3(2H)-one hydrochloride;
4-phenyl-2-[1-(2-phenylethyl)piperidin-4-yl]-2,3-dihydropyridazin-3-one hydrochloride;
2-[1-(cyclopropylmethyl)piperidin-4-yl]-4-phenyl-2,3-dihydropyridazin-3-one hydrochloride;
2-[1-(cyclopentylmethyl)piperidin-4-yl]-4-phenyl-2,3-dihydropyridazin-3-one hydrochloride;
2-(1-benzylazepan-4-yl)-4-phenyl-2,3-dihydropyridazin-3-one hydrochloride;
2-((1-benzylpiperidin-4-yl)methyl)-6-methyl-4-phenylpyridazin-3 (2H)-one;
2-((1-benzylpiperidin-4-yl)methyl)-5-phenylpyridazin-3(2H)-one hydrochloride;
2-[(1-benzylpiperidin-3-yl) methyl]-4-phenyl-2,3-dihydropyridazin-3-one hydrochloride;
2-((1-phenethylpiperidin-4-yl)methyl)-4-phenylpyridazin-3(2H)-one hydrochloride;
4-phenyl-2-((1-propylpiperidin-4-yl)methyl)pyridazin-3(2H)-one hydrochloride;
2-((1-(4-chlorobenzyl)piperidin-4-yl)methyl)-4-phenylpyridazin-3(2H)-one hydrochloride;
2-((1-(cyclohexylmethyl)piperidin-4-yl)methyl)-4-phenylpyridazin-3(2H)-one hydrochloride;
4-phenyl-2-((1-(pyridin-4-ylmethyl)piperidin-4-yl)methyl)pyridazin-3(2H)-one hydrochloride;
4-phenyl-2-((1-((tetrahydro-2H-pyran-4-yl)methyl)piperidin-4-yl)methyl)pyridazin-3(2H)-one, hydrochloride;
2-{[1-(1H-imidazol-5-ylmethyl)piperidin-4-yl]methyl}-4-phenyl-2,3-dihydropyridazin-3-one, hydrochloride;
4-phenyl-2-((1-((tetrahydro-2H-pyran-3-yl)methyl)piperidin-4-yl)methyl)pyridazin-3(2H)-one hydrochloride;
2-((1-(4-hydroxybenzyl)piperidin-4-yl)methyl)-4-phenylpyridazin-3(2H)-one hydrochloride;
2-((1-(2-methoxyethyl)piperidin-4-yl)methyl)-4-phenylpyridazin-3(2H)-one hydro- chloride;
2-((1-(2-hydroxyethyl)piperidin-4-yl)methyl)-4-phenylpyridazin-3(2H)-one hydrochloride;
2-((1-(2-methoxy-1-phenylethyl)piperidin-4-yl)methyl)-4-phenylpyridazin-3(2H)-one hydrochloride;
N-(2-{4-[(6-oxo-5-phenyl-1,6-dihydropyridazin-1-yl)methyl]piperidin-1-yl}ethyl)acetamide hydrochloride;
2-((1-(2-(4-fluorophenyl)-2-hydroxyethyl)piperidin-4-yl)methyl)-4-phenylpyridazin-3 (2H)-one hydrochloride;
2-((1-(2-(4-fluorophenyl)-2-oxoethyl)piperidin-4-yl)methyl)-4-phenylpyridazin-3 (2H)-one hydrochloride;
2-((1-(2-hydroxy-2-phenylethyl)piperidin-4-yl)methyl)-4-phenylpyridazin-3 (2H)-one hydrochloride;
2-(2-(1-benzylpiperidin-4-yl)ethyl)-4-phenylpyridazin-3(2H)-one hydrochloride;
2-(2-(1-benzylpiperidin-4-yl)ethyl)-5-phenylpyridazin-3(2H)-one hydrochloride;
2-(1-(1-benzylpiperidin-4-yl)ethyl)-4-phenylpyridazin-3(2H)-one hydrochloride;
2-((1-benzyl-4-hydroxypiperidin-4-yl)methyl)-4-phenylpyridazin-3(2H)-one hydrochloride;
2-(1-benzylpiperidin-4-yl)-4-(4-methoxyphenyl)pyridazin-3(2H)-one hydrochloride;
2-(1-benzylpiperidin-4-yl)-4-[4-(trifluoromethyl)phenyl]-2,3-dihydropyridazin-3-one hydrochloride;
2-(1-benzylpiperidin-4-yl)-4-[4-(chlorophenyl]-2,3-dihydropyridazin-3-one hydrochloride;
2-[(1-benzylpiperidin-4-yl)methyl]-4-(2-chlorophenyl)-2,3-dihydropyridazin-3-one hydrochloride;
2-[(1-benzylpiperidin-4-yl)methyl]-4-(4-hydroxyphenyl)-2,3-dihydropyridazin-3-one hydrochloride;
2-((1-benzylpiperidin-4-yl)methyl)-4-(4-fluorophenyl)pyridazin-3(2H)-one hydrochloride;
2-((1-benzylpiperidin-4-yl)methyl)-4-phenethylpyridazin-3(2H)-one, 2-(1-benzylpiperidin-4-yl)-4-(2-phenylethyl)-2,3-dihydropyridazin-3-one hydrochloride;
2-(1-benzylpiperidin-4-yl)-4-(piperidin-1-yl)-2,3-dihydropyridazin-3-one hydrochloride;
2-[(1-benzylpiperidin-4-yl)methyl]-4-(piperidin-1-yl)-2,3-dihydropyridazin-3-one hydrochloride;
2-((1-benzylpiperidin-4-yl)methyl)-4-morpholinopyridazin-3(2H)-one hydrochloride;
2-((1-benzylpiperidin-4-yl)methyl)-4-(4-phenylpiperazin-1-yl)pyridazin-3(2H)-onehydrochloride;
2-((1-benzylpiperidin-4-yl)methyl)-4-(3,4-dihydroisoquinolin-2(1H)-yl)pyridazin-3(2H)-one hydrochloride;
3-(1-benzylpiperidin-4-yl)-5-phenylpyrimidin-4(3H)-one hydrochloride;
1-(1-benzylpiperidin-4-yl)-3-phenyl-1,2-dihydropyridin-2-one hydrochloride;
3-((1-benzylpiperidin-4-yl)methyl)-5-phenylpyrimidin-4(3H)-one; and
1-[(1-benzylpiperidin-4-yl)methyl]-3-phenyl-1,2-dihydropyridin-2-one hydrochloride.

9. The compound according to any one of claims 1 to 8, wherein it is selected in the group consisting of:
2-(1-benzylpiperidin-4-yl)-4-phenylpyridazin-3(2H)-one hydrochloride;
3-(1-benzylpiperidin-4-yl)-5-phenylpyrimidin-4(3H)-one hydrochloride;
2-((1-benzylpiperidin-4-yl)methyl)-4-phenethylpyridazin-3(2H)-one;
2-[(1-benzylpiperidin-4-yl)methyl]-4-(piperidin-1-yl)-2,3-dihydropyridazin-3-one hydrochloride;
2-((1-benzyl-4-hydroxypiperidin-4-yl)methyl)-4-phenylpyridazin-3(2H)-one hydrochloride;
4-phenyl-2-((1-((tetrahydro-2H-pyran-4-yl)methyl)piperidin-4-yl)methyl)pyridazin-3(2H)-one hydrochloride;
2-(1-(1-benzylpiperidin-4-yl)ethyl)-4-phenylpyridazin-3(2H)-one hydrochloride; and
2-((1-(2-hydroxyethyl)piperidin-4-yl)methyl)-4-phenylpyridazin-3(2H)-one hydrochloride.

10. A compound according to any one of claims 1 to 9, for use as a medicine.

11. A pharmaceutical composition comprising a compound according to any one of claims 1 to 9, and a pharmaceutically acceptable support.

12. A compound or pharmaceutical composition according to anyone of the preceding claims, for use in the treatment of disorder modulated by sigma-1 receptor.

13. The compound or pharmaceutical composition for use according to claim 12, wherein said disorder is selected from the group consisting of neurodegenerative diseases such as Alzheimer disease, Parkinson's disease, Huntington's disease, Amyotrophic lateral sclerosis, multiple sclerosis; cognitive and memory alterations such as pathological ageing, ischemic amnesia, schizophrenia-related cognitive deficits and depression; developmental cognitive disorders such as autism-related disorders and mental retardation-related disorders; and genetic diseases associated with MAM dysfunctions.

## Patentansprüche

1. Verbindung gemäß folgender Formel (I): 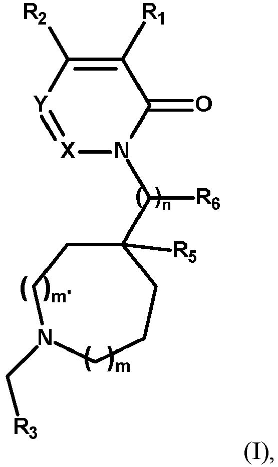 wobei:
R₁ darstellt:
• H,
• eine Aryl-(C₁-C₆)Alkyl-Gruppe;
• eine Aryl-Gruppe;
• eine Cycloalkyl-Gruppe;
• eine heterocyclische Gruppe; oder
• SR, wobei R Alkyl, Aryl oder Aralkyl ist;
R₂ darstellt:
• H,
• eine Aryl-(C₁-C₆)Alkyl-Gruppe;
• eine Aryl-Gruppe;
• eine Cycloalkyl-Gruppe; oder
• SR, wobei R Alkyl, Aryl oder Aralkyl ist;
wobei eines von R₁ und R₂ H ist, und das andere von R₁ und R₂ anders als H ist;
X und Y entweder jeweils sind:
• CH und N oder
• N und CR₄ oder
• CH und CR₄,
wobei R₄ H oder ein (C₁-C₄)Alkyl darstellt;
n 0, 1, oder 2 ist; m 0 or 1 ist; m' 0 oder 1 ist;
R₃ darstellt:
• einen Rest ausgewählt aus der Gruppe bestehend aus H, (C₁-C₆Aalkyl, (C₂-C₆)Alkenyl, Aryl, Heteroaryl, Cycloalkyl, Heterocycloalkyl, (C₁-C₆)Alkoxy und Aryl-(C₁-C₆)Alkyl, wobei dieser Rest gegebenenfalls durch wenigstens eine Gruppe ausgewählt aus (C₁-C₆)Alkyl, OH, Halogen, Aryl, Heteroaryl, Cycloalkyl, Heterocycloalkyl, (C₁-C₆)Alkoxy, -C(O)R, -CHOHR, C(O)₂R, C(O)NRR', -CONHOR, -CONHSO₂R, -NRR', -N(R)C(O)R', -N(R)NR'R", -N(R)C(O)₂R', -N(R)C(O)NR'R", -N(R)S(O)₂R', -SR, - S(O)R, -S(O)₂R, -S(O)NRR' und -S(O)₂NRR' substituiert ist; wobei R, R' und R" unabhängig voneinander H, (C₁-C₆)Alkyl, Cycloalkyl, Aryl, Heterocycloalkyl, Heteroaryl, (C₁-C₆)Alkylcycloalkyl, (C₁-C₆)Alkylaryl, (C₁-C₆)Alkylheterocycloalkyl) oder (C₁-C₆)Alkylheteroaryl sind; oder
• einen Rest ausgewählt aus der Gruppe bestehend aus OH, -C(O)R, -CHOHR, C(O)₂R, C(O)NRR', -CONHOR, -CONHSO₂R, -NRR', -N(R)C(O)R', -N(R)NR'R", - N(R)C(O)₂R', -N(R)C(O)NR'R", -N(R)S(O)₂R', -SR, -S(O)R, -S(O)₂R, -S(O)NRR', und -S(O)₂NRR'; wobei R, R' und R" unabhängig voneinander H, (C₁-C₆)Alkyl, Cycloalkyl, Aryl, Heterocycloalkyl, Heteroaryl, (C₁-C₆)Alkylcycloalkyl, (C₁-C₆)Alkylaryl, (C₁-C₆)Alkylheterocycloalkyl) oder (C₁-C₆)Alkylheteroaryl sind;
R₅ H oder OH darstellt; und
jedes R₆ unabhängig H oder ein (C₁-C₆)Alkyl-Gruppe darstellt;
ein Stereoisomer, ein Solvat oder irgendein pharmazeutisches Salz davon.

2. Verbindung nach Anspruch 1, wobei:
R₁ darstellt:
• eine Aryl-(C₁-C₆)Alkyl-Gruppe;
• eine Aryl-Gruppe;
• eine Cycloalkyl-Gruppe;
• eine heterocyclische Gruppe; oder
• SR, wobei R Alkyl, Aryl oder Aralkyl ist;
und R₂ H ist.

3. Verbindung nach Anspruch 1 oder 2, wobei:
R₁ darstellt:
• eine Aryl-(C₁-C₆)Alkyl-Gruppe;
• eine Aryl-Gruppe; oder
• eine heterocyclische Gruppe;
vorzugsweise stellt R₁ eine Aryl-Gruppe dar; und
R₂ stellt H dar.

4. Verbindung nach einem der Ansprüche 1 bis 3, wobei X und Y jeweils N und CR₄, sind, wobei R₄ H oder ein (C₁-C₄)Alkyl ist, vorzugsweise H oder Methyl, bevorzugter H.

5. Verbindung nach einem der Ansprüche 1 bis 4, wobei R₅ und jedes R₆ H sind.

6. Verbindung nach einem der Ansprüche 1 bis 5, wobei wenigstens eine der folgenden Merkmale erfüllt ist:
n ist 1; und/oder
m ist 0; und/oder
m' ist 1; und/oder
R₁ stellt eine Aryl-Gruppe dar; und/oder
R₂ ist H; und/oder
R₃ stellt dar:
• einen Rest ausgewählt aus H, einem Aryl (z.B. Phenyl), einem (C₁-C₆)Alkyl (z.B. Ethyl), einem (C₂-C₆)Alkenyl (z.B. Isobutenyl), einem Cycloalkyl (z.B. Cyclopropyl, Cyclopentyl, Cyclohexyl), einem Heterocycloalkyl (z.B. Tetrahydropyranyl), einem Heteroaryl (z.B. Pyridyl, Imidazolyl), (C₁-C₆)Alkoxy (z.B. Methoxy), Aryl-(C₁-C₆)Alkyl (z.B. Benzyl),
wobei dieser Rest gegebenenfalls durch ein oder zwei (C₁-C₆)Alkyl, OH, Halogen (z.B. Fluor, Chlor), Aryl (z.B. Phenyl), Heteroaryl, Cycloalkyl, Heterocycloalkyl, (C₁-C₆)Alkoxy, -C(O)₂R, -N(R)C(O)R' substituiert ist; R und R' unabhängig voneinander H, (C₁-C₆)Alkyl, Cycloalkyl, Aryl (so wie ein Phenyl gegebenenfalls substitutiert durch ein Halogen (vorzugsweise ein Fluor)), Heterocycloalkyl, Heteroaryl, (C₁-C₆)Alkylcycloalkyl, (C₁-C₆)Alkylaryl, (C₁-C₆)Alkylheterocycloalkyl) oder (C₁-C₆)Alkylheteroaryl sind; oder
einen Rest ausgewählt aus der Gruppe bestehend aus -CHOHR, -C(O)R, -C(O)₂R; wobei R H, (C₁-C₆)Alkyl, Cycloalkyl, Aryl, Heterocycloalkyl, Heteroaryl, (C₁-C₆)-Alkylcycloalkyl, (C₁-C₆)-Alkylaryl, (C₁-C₆)-Alkylheterocycloalkyl) oder (C₁-C₆)Alkylheteroaryl ist; und/oder
R₅ H ist; und/oder
jedes R₆ H ist.

7. Verbindung nach einem der Ansprüche 1 bis 6, wobei die Aryl-Gruppe eine PhenylGruppe gegebenenfalls substituiert durch wenigstens einen Substituenten, wobei dieser wenigstens eine Substituent ausgewählt ist aus:
• einem Halogen, vorzugsweise einem Fluor oder einem Chlor;
• einem Alkyl gegenenenfalls substitutiert durch wenigstens ein Halogen, vorzugsweise ein Fluor, vorzugsweise -CF₃;
• -OH; und
• -OR, wobei R ein Alkyl, Cycloalkyl, Heterocycloalkyl, Aryl oder Heteroaryl ist, vorzugsweise ein Alkyl, bevorzugter ein Methyl.

8. Verbindung nach einem der Ansprüche 1 bis 7, wobei sie ausgewählt ist aus der Gruppe bestehend aus:
2-(1-Benzylpiperidin-4-yl)-4-phenylpyridazin-3(2H)-on Hydrochlorid;
2-(1-Benzylpiperidin-4-yl)-5-phenylpyridazin-3(2H)-on Hydrochlorid;
4-Phenyl-2-[1-(2-phenylethyl)piperidin-4-yl]-2,3-dihydropyridazin-3-on Hydrochlorid;
2-[1-(Cyclopropylmethyl)piperidin-4-yl]-4-phenyl-2,3-dihydropyridazin-3-on Hydrochlorid;
2-[1-(Cyclopentylmethyl)piperidin-4-yl]-4-phenyl-2,3-dihydropyridazin-3-on Hydrochlorid;
2-(1-Benzylazepan-4-yl)-4-phenyl-2,3-dihydropyridazin-3-on Hydrochlorid;
2-((1-Benzylpiperidin-4-yl)methyl)-6-methyl-4-phenylpyridazin-3(2H)-on;
2-((1-Benzylpiperidin-4-yl)methyl)-5-phenylpyridazin-3(2H)-on Hydrochlorid;
2-[(1-Benzylpiperidin-3-yl) methyl]-4-phenyl-2,3-dihydropyridazin-3-on Hydrochlorid;
2-((1-Phenethylpiperidin-4-yl)methyl)-4-phenylpyridazin-3(2H)-on Hydrochlorid;
4-Phenyl-2-((1-propylpiperidin-4-yl)methyl)pyridazin-3(2H)-on Hydrochlorid;
2-((1-(4-Chlorbenzyl)piperidin-4-yl)methyl)-4-phenylpyridazin-3(2H)-on Hydrochlorid;
2-((1-(Cyclohexylmethyl)piperidin-4-yl)methyl)-4-phenylpyridazin-3(2H)-on Hydrochlorid;
4-Phenyl-2-((1-(pyridin-4-ylmethyl)piperidin-4-yl)methyl)pyridazin-3(2H)-on Hydrochlorid;
4-Phenyl-2-((1-((tetrahydro-2H-pyran-4-yl)methyl)piperidin-4-yl)methyl)pyridazin-3(2H)-on, Hydrochlorid;
2-{[1-(1H-Imidazol-5-ylmethyl)piperidin-4-yl]methyl}-4-phenyl-2,3-dihydropyridazin-3-on, Hydrochlorid;
4-Phenyl-2-((1-((tetrahydro-2H-pyran-3-yl)methyl)piperidin-4-yl)methyl)pyridazin-3(2H)-on Hydrochlorid;
2-((1-(4-Hydroxybenzyl)piperidin-4-yl)methyl)-4-phenylpyridazin-3(2H)-on Hydrochlorid;
2-((1-(2-Methoxyethyl)piperidin-4-yl)methyl)-4-phenylpyridazin-3(2H)-on Hydrochlorid;
2-((1-(2-Hydroxyethyl)piperidin-4-yl)methyl)-4-phenylpyridazin-3(2H)-on Hydrochlorid;
2-((1-(2-Methoxy-1-phenylethyl)piperidin-4-yl)methyl)-4-phenylpyridazin-3(2H)-on Hydrochlorid;
N-(2-{4-[(6-Oxo-5-phenyl-1,6-dihydropyridazin-1-yl)methyl]piperidin-1-yl}ethyl)acetamid Hydrochlorid;
2-((1-(2-(4-Fluorphenyl)-2-hydroxyethyl)piperidin-4-yl)methyl)-4-phenylpyridazin-3(2H)-on Hydrochlorid;
2-((1-(2-(4-Fluorphenyl)-2-oxoethyl)piperidin-4-yl)methyl)-4-phenylpyridazin-3(2H)-on Hydrochlorid;
2-((1-(2-Hydroxy-2-phenylethyl)piperidin-4-yl)methyl)-4-phenylpyridazin-3(2H)-on Hydrochlorid;
2-(2-(1-Benzylpiperidin-4-yl)ethyl)-4-phenylpyridazin-3(2H)-on Hydrochlorid;
2-(2-(1-Benzylpiperidin-4-yl)ethyl)-5-phenylpyridazin-3(2H)-on Hydrochlorid;
2-(1-(1-Benzylpiperidin-4-yl)ethyl)-4-phenylpyridazin-3(2H)-on Hydrochlorid;
2-((1-Benzyl-4-hydroxypiperidin-4-yl)methyl)-4-phenylpyridazin-3(2H)-on Hydrochlorid;
2-(1-Benzylpiperidin-4-yl)-4-(4-methoxyphenyl)pyridazin-3(2H)-on Hydrochlorid;
2-(1-Benzylpiperidin-4-yl)-4-[4-(trifluormethyl)phenyl]-2,3-dihydropyridazin-3-on Hydrochlorid;
2-(1-Benzylpiperidin-4-yl)-4-[4-(chlorphenyl]-2,3-dihydropyridazin-3-on Hydrochlorid;
2-[(1-Benzylpiperidin-4-yl)methyl]-4-(2-chlorphenyl)-2,3-dihydropyridazin-3-on Hydrochlorid;
2-[(1-Benzylpiperidin-4-yl)methyl]-4-(4-hydroxyphenyl)-2,3-dihydropyridazin-3-on Hydrochlorid;
2-((1-Benzylpiperidin-4-yl)methyl)-4-(4-fluorphenyl)pyridazin-3(2H)-on Hydrochlorid;
2-((1-Benzylpiperidin-4-yl)methyl)-4-phenethylpyridazin-3(2H)-on,
2-(1-Benzylpiperidin-4-yl)-4-(2-phenylethyl)-2,3-dihydropyridazin-3-on Hydrochlorid;
2-(1-Benzylpiperidin-4-yl)-4-(piperidin-1-yl)-2,3-dihydropyridazin-3-on Hydrochlorid;
2-[(1-Benzylpiperidin-4-yl)methyl]-4-(piperidin-1-yl)-2,3-dihydropyridazin-3-on Hydrochlorid;
2-((1-Benzylpiperidin-4-yl)methyl)-4-morpholinopyridazin-3(2H)-on Hydrochlorid;
2-((1-Benzylpiperidin-4-yl)methyl)-4-(4-phenylpiperazin-1-yl)pyridazin-3(2H)-on Hydrochlorid;
2-((1-Benzylpiperidin-4-yl)methyl)-4-(3,4-dihydroisoquinolin-2(1H)-yl)pyridazin-3(2H)-on Hydrochlorid;
3-(1-Benzylpiperidin-4-yl)-5-phenylpyrimidin-4(3H)-on Hydrochlorid;
1-(1-Benzylpiperidin-4-yl)-3-phenyl-1,2-dihydropyridin-2-on Hydrochlorid;
3-((1-Benzylpiperidin-4-yl)methyl)-5-phenylpyrimidin-4(3H)-on; und
1-[(1-Benzylpiperidin-4-yl)methyl]-3-phenyl-1,2-dihydropyridin-2-on Hydrochlorid.

9. Verbindung nach einem der Ansprüche 1 bis 8, wobei sie ausgewählt ist in der Gruppe bestehend aus:
2-(1-Benzylpiperidin-4-yl)-4-phenylpyridazin-3(2H)-on Hydrochlorid;
3-(1-Benzylpiperidin-4-yl)-5-phenylpyrimidin-4(3H)-on Hydrochlorid;
2-((1-Benzylpiperidin-4-yl)methyl)-4-phenethylpyridazin-3(2H)-on;
2-[(1-Benzylpiperidin-4-yl)methyl]-4-(piperidin-1-yl)-2,3-dihydropyridazin-3-on Hydrochlorid;
2-((1-Benzyl-4-hydroxypiperidin-4-yl)methyl)-4-phenylpyridazin-3(2H)-on Hydrochlorid;
4-Phenyl-2-((1-((tetrahydro-2H-pyran-4-yl)methyl)piperidin-4-yl)methyl)pyridazin-3(2H)-on Hydrochlorid;
2-(1-(1-Benzylpiperidin-4-yl)ethyl)-4-phenylpyridazin-3(2H)-on Hydrochlorid; und
2-((1-(2-Hydroxyethyl)piperidin-4-yl)methyl)-4-phenylpyridazin-3(2H)-on Hydrochlorid.

10. Verbindung nach einem der Ansprüche 1 bis 9, zur Verwendung als Arzneimittel.

11. Pharmazeutische Zusammensetzung enthaltend eine Verbindung nach einem der Ansprüche 1 bis 9 und ein pharmazeutisch verträgliches Hilfsmittel.

12. Verbindung oder pharmazeutische Zusammensetzung nach einem der vorangehenden Ansprüche zur Verwendung bei der Behandlung von Erkrankung moduliert durch Sigma-1 Rezeptor.

13. Verbindung oder pharmazeutische Zusammensetzung zur Verwendung nach Anspruch 12, wobei die Erkrankung aus der Gruppe bestehend aus neurodegenerativen Erkrankungen wie Alzheimer-Krankheit, Parkinson-Krankheit, Huntington-Krankheit, Amyotropher Lateralsklerose, Multipler Sklerose; kognitiver und Gedächtnisveränderungen wie pathologischem Altern, ischämischer Amnesie, schizophreniebedingter kognitiver Defizite und Depression; entwicklungsbedingter kognitiver Störungen wie Autismus-bedingter Störungen und geistiger Behinderung-bedingter Störungen; und genetischen Krankheiten, die mit MAM-Funktionsstörungen verbunden sind, ausgewählt ist.

## Revendications

1. Composé de formule (I) suivante : 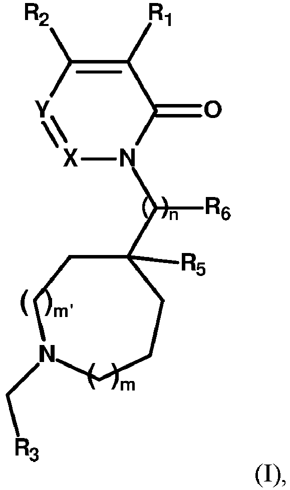 dans laquelle :
R₁ représente :
• H ;
• un groupe aryl-alkyle en Ci à C₆ ;
• un groupe aryle ;
• un groupe cycloalkyle ;
• un groupe hétérocyclique ; ou
• SR, R étant un alkyle, aryle ou aralkyle ;
R₂ représente :
• H ;
• un groupe aryl-alkyle en Ci à C₆ ;
• un groupe aryle ;
• un groupe cycloalkyle ; ou
• SR, R étant un alkyle, aryle ou aralkyle ;
où l'un de R₁ et R₂ est H, et l'autre de R₁ et R₂ est différent de H ;
X et Y sont respectivement :
• soit CH et N,
• soit N et CR₄,
• soit CH et CR₄,
où R₄ représente H ou un alkyle en Ci à C₄ ;
n vaut 0, 1 ou 2 ; m vaut 0 ou 1 ; m' vaut 0 ou 1 ;
R₃ représente :
• un radical choisi dans l'ensemble constitué par H, alkyle en Ci à C₆, alcényle en C₂ à C₆, aryle, hétéroaryle, cycloalkyle, hétérocycloalkyle, alcoxy en Ci à C₆, et aryl-alkyle en Ci à C₆, ledit radical étant éventuellement substitué par au moins un groupe choisi parmi alkyle en Ci à C₆, OH, halogène, aryle, hétéroaryle, cycloalkyle, hétérocycloalkyle, alcoxy en Ci à C₆, -C(O)R, -CHOHR, C(O)₂R, C(O)NRR', -CONHOR, -CONHSO₂R, NRR', -N(R)C(O)R', -N(R)NR'R", -N(R)C(O)₂R', -N(R)C(O)NR'R", -N(R)S(O)₂R', SR, -S(O)R, -S(O)₂R, -S(O)NRR', et -S(O)₂NRR' ; R, R' et R" étant indépendamment H, alkyle en Ci à C₆, cycloalkyle, aryle, hétérocycloalkyle, hétéroaryle, (alkyle en Ci à C₆)cycloalkyle, (alkyle en Ci à C₆)aryle, (alkyle en Ci à C₆)hétérocycloalkyle, ou (alkyle en Ci à C₆)hétéroaryle ; ou
• un radical choisi dans l'ensemble constitué par OH, -C(O)R, -CHOHR, C(O)₂R, C(O)NRR', -CONHOR, -CONHSO₂R, -NRR', -N(R)C(O)R', -N(R)NR'R", -N(R)C(O)₂R', -N(R)C(O)NR'R", -N(R)S(O)₂R', -SR, -S(O)R, -S(O)₂R, -S(O)NRR', et -S(O)₂NRR' ; R, R' et R" étant indépendamment H, alkyle en Ci à C₆, cycloalkyle, aryle, hétérocycloalkyle, hétéroaryle, (alkyle en Ci à C₆)cycloalkyle, (alkyle en Ci à C₆)aryle, (alkyle en Ci à C₆)hétérocycloalkyle, ou (alkyle en Ci à C₆)hétéroaryle ;
R₅ représente H ou OH ; et
chaque R₆ représente indépendamment H ou un groupe alkyle en Ci à C₆ ;
un stéréoisomère, un solvate ou un quelconque sel pharmaceutique de celui-ci.

2. Composé selon la revendication 1, dans lequel :
R₁ représente :
• un groupe aryl-alkyle en Ci à C₆ ;
• un groupe aryle ;
• un groupe cycloalkyle ;
• un groupe hétérocyclique ; ou
• SR, R étant un alkyle, aryle ou aralkyle ;
et R₂ est H.

3. Composé selon la revendication 1 ou 2, dans lequel :
R₁ représente :
• un groupe aryl-alkyle en Ci à C₆ ;
• un groupe aryle ; ou
• un groupe hétérocyclique ;
de préférence R₁ représente un groupe aryle ; et
R₂ représente H.

4. Composé selon l'une quelconque des revendications 1 à 3, dans lequel X et Y sont N et CR₄, respectivement, où R₄ est H ou un alkyle en Ci à C₄, de préférence H ou méthyle, mieux encore H.

5. Composé selon l'une quelconque des revendications 1 à 4, dans lequel R₅ et chaque R₆ sont H.

6. Composé selon l'une quelconque des revendications 1 à 5, dans lequel au moins l'une des caractéristiques suivantes est remplie :
n vaut 1 ; et/ou
m vaut 0 ; et/ou
m' vaut 1 ; et/ou
R₁ représente un groupe aryle ; et/ou
R₂ est H ; et/ou
R₃ représente :
• un radical choisi parmi H, un aryle (par exemple phényle), un alkyle en Ci à C₆ (par exemple éthyle), un alcényle en C₂ à C₆ (par exemple isobutényle), un cycloalkyle (par exemple cyclopropyle, cyclopentyle, cyclohexyle), un hétérocycloalkyle (par exemple tétrahydropyranyle), un hétéroaryle (par exemple pyridyle, imidazolyle), un alcoxy en Ci à C₆ (par exemple méthoxy), un aryl-alkyle en Ci à C₆ (par exemple benzyle)
ledit radical étant éventuellement substitué par un ou deux alkyles en Ci à C₆, OH, halogènes (par exemple fluor, chlore), aryles (par exemple phényle), hétéroaryles, cycloalkyles, hétérocycloalkyles, alcoxys en Ci à C₆, -C(O)₂R, -N(R)C(O)R' ; où R et R" sont indépendamment H, alkyle en Ci à C₆, cycloalkyle, aryle (tel qu'un phényle éventuellement substitué par un halogène (de préférence un fluor)), hétérocycloalkyle, hétéroaryle, (alkyle en Ci à C₆)cycloalkyle, (alkyle en Ci à C₆)aryle, (alkyle en Ci à C₆)hétérocycloalkyle, ou (alkyle en Ci à C₆)hétéroaryle ; ou
un radical choisi dans l'ensemble constitué par -CHOHR, -C(O)R, -C(O)₂R ; où R est H, alkyle en Ci à C₆, cycloalkyle, aryle, hétérocycloalkyle, hétéroaryle, (alkyle en Ci à C₆)cycloalkyle, (alkyle en Ci à C₆)aryle, (alkyle en Ci à C₆)hétérocycloalkyle, ou (alkyle en Ci à C₆)hétéroaryle ; et/ou
R₅ est H ; et/ou
chaque R₆ est H.

7. Composé selon l'une quelconque des revendications 1 à 6, dans lequel le groupe aryle est un groupe phényle éventuellement substitué par au moins un substituant, ledit au moins un substituant étant choisi parmi :
• un halogène, de préférence un fluor ou un chlore ;
• un alkyle éventuellement substitué par au moins un halogène, de préférence un fluor, de préférence -CF₃ ;
• -OH ; et
• -OR où R est un alkyle, cycloalkyle, hétérocycloalkyle, aryle ou hétéroaryle, de préférence un alkyle, mieux encore un méthyle.

8. Composé selon l'une quelconque des revendications 1 à 7, qui est choisi dans l'ensemble constitué par :
chlorhydrate de 2-(1-benzylpipéridin-4-yl)-4-phénylpyridazin-3(2H)-one ;
chlorhydrate de 2-(1-benzylpipéridin-4-yl)-5-phénylpyridazin-3(2H)-one ;
chlorhydrate de 4-phényl-2-[1-(2-phényléthyl)pipéridin-4-yl]-2,3-dihydropyridazin-3-one ;
chlorhydrate de 2-[1-(cyclopropylméthyl)pipéridin-4-yl]-4-phényl-2,3-dihydropyridazin-3-one ;
chlorhydrate de 2-[1-(cyclopentylméthyl)pipéridin-4-yl]-4-phényl-2,3-dihydropyridazin-3-one ;
chlorhydrate de 2-(1-benzylazépan-4-yl)-4-phényl-2,3-dihydropyridazin-3-one ;
2-((1-benzylpipéridin-4-yl)méthyl)-6-méthyl-4-phénylpyridazin-3(2H)-one ;
chlorhydrate de 2-((1-benzylpipéridin-4-yl)méthyl)-5-phénylpyridazin-3(2H)-one ;
chlorhydrate de 2-[(1-benzylpipéridin-3-yl)méthyl]-4-phényl-2,3-dihydropyridazin-3-one ;
chlorhydrate de 2-((1-phénéthylpipéridin-4-yl)méthyl)-4-phénylpyridazin-3(2H)-one ;
chlorhydrate de 4-phényl-2-((1-propylpipéridin-4-yl)méthyl)pyridazin-3(2H)-one ;
chlorhydrate de 2-((1-(4-chlorobenzyl)pipéridin-4-yl)méthyl)-4-phénylpyridazin-3(2H)-one ;
chlorhydrate de 2-((1-(cyclohexylméthyl)pipéridin-4-yl)méthyl)-4-phénylpyridazin-3(2H)-one ;
chlorhydrate de 4-phényl-2-((1-(pyridin-4-ylméthyl)pipéridin-4-yl)méthyl)pyridazin-3(2H)-one ;
chlorhydrate de 4-phényl-2-((1-((tétrahydro-2H-pyran-4-yl)méthyl)pipéridin-4-yl)méthyl)pyridazin-3(2H)-one ;
chlorhydrate de 2-{[1-(1H-imidazol-5-ylméthyl)pipéridin-4-yl]méthyl}-4-phényl-2,3-dihydropyridazin-3-one ;
chlorhydrate de 4-phényl-2-((1-((tétrahydro-2H-pyran-3-yl)méthyl)pipéridin-4-yl)méthyl)pyridazin-3(2H)-one ;
chlorhydrate de 2-((1-(4-hydroxybenzyl)pipéridin-4-yl)méthyl)-4-phénylpyridazin-3(2H)-one ;
chlorhydrate de 2-((1-(2-méthoxyéthyl)pipéridin-4-yl)méthyl)-4-phénylpyridazin-3(2H)-one ;
chlorhydrate de 2-((1-(2-hydroxyéthyl)pipéridin-4-yl)méthyl)-4-phénylpyridazin-3(2H)-one ;
chlorhydrate de 2-((1-(2 méthoxy-1-phényléthyl)pipéridin-4-yl)méthyl)-4-phénylpyridazin-3(2H)-one ;
chlorhydrate de N-(2-{4-[(6-oxo-5-phényl-1,6-dihydropyridazin-1-yl)méthyl]pipéridin-1-yl}éthyl)acétamide ;
chlorhydrate de 2-((1-(2-(4-fluorophényl)-2-hydroxyéthyl)pipéridin-4-yl)méthyl)-4-phénylpyridazin-3(2H)-one ;
chlorhydrate de 2-((1-(2-(4-fluorophényl)-2-oxoéthyl)pipéridin-4-yl)méthyl)-4-phénylpyridazin-3(2H)-one ;
chlorhydrate de 2-((1-(2-hydroxy-2-phényléthyl)pipéridin-4-yl)méthyl)-4-phénylpyridazin-3(2H)-one ;
chlorhydrate de 2-(2-(1-benzylpipéridin-4-yl)éthyl)-4-phénylpyridazin-3(2H)-one ;
chlorhydrate de 2-(2-(1-benzylpipéridin-4-yl)éthyl)-5-phénylpyridazin-3(2H)-one ;
chlorhydrate de 2-(1-(1-benzylpipéridin-4-yl)éthyl)-4-phénylpyridazin-3(2H)-one ;
chlorhydrate de 2-((1-benzyl-4-hydroxypipéridin-4-yl)méthyl)-4-phénylpyridazin-3(2H)-one ;
chlorhydrate de 2-(1-benzylpipéridin-4-yl)-4-(4-méthoxyphényl)pyridazin-3(2H)-one ;
chlorhydrate de 2-(1-benzylpipéridin-4-yl)-4-[4-(trifluorométhyl)phényl]-2,3-dihydropyridazin-3-one ;
chlorhydrate de 2-(1-benzylpipéridin-4-yl)-4-[4-(chlorophényl]-2,3-dihydropyridazin-3-one ;
chlorhydrate de 2-[(1-benzylpipéridin-4-yl)méthyl]-4-(2-chlorophényl)-2,3-dihydropyridazin-3-one ;
chlorhydrate de 2-[(1-benzylpipéridin-4-yl)méthyl]-4-(4-hydroxyphényl)-2,3-dihydropyridazin-3-one ;
chlorhydrate de 2-((1-benzylpipéridin-4-yl)méthyl)-4-(4-fluorophényl)pyridazin-3(2H)-one ;
2-((1-benzylpipéridin-4-yl)méthyl)-4-phénéthylpyridazin-3(2H)-one ;
chlorhydrate de 2-(1-benzylpipéridin-4-yl)-4-(2-phényléthyl)-2,3-dihydropyridazin-3-one ;
chlorhydrate de 2-(1-benzylpipéridin-4-yl)-4-(pipéridin-1-yl)-2,3-dihydropyridazin-3-one ;
chlorhydrate de 2-[(1-benzylpipéridin-4-yl)méthyl]-4-(pipéridin-1-yl)-2,3-dihydropyridazin-3-one ;
chlorhydrate de 2-((1-benzylpipéridin-4-yl)méthyl)-4-morpholinopyridazin-3(2H)-one ;
chlorhydrate de 2-((1-benzylpipéridin-4-yl)méthyl)-4-(4-phénylpipérazin-1-yl)pyridazin-3(2H)-one ;
chlorhydrate de 2-((1-benzylpipéridin-4-yl)méthyl)-4-(3,4-dihydroisoquinolin-2(lH)-yl)pyridazin-3(2H)-one ;
chlorhydrate de 3-(1-benzylpipéridin-4-yl)-5-phénylpyrimidin-4(3H)-one ;
chlorhydrate de 1-(1-benzylpipéridin-4-yl)-3 phényl-1,2-dihydropyridin-2-one ;
3-((1-benzylpipéridin-4-yl)méthyl)-5-phénylpyrimidin-4(3H)-one ; et
chlorhydrate de 1-[(1-benzylpipéridin-4-yl)méthyl]-3-phényl-1,2-dihydropyridin-2-one.

9. Composé selon l'une quelconque des revendications 1 à 8, qui est choisi dans l'ensemble constitué par :
chlorhydrate de 2-(1-benzylpipéridin-4-yl)-4-phénylpyridazin-3(2H)-one ;
chlorhydrate de 3-(1-benzylpipéridin-4-yl)-5-phénylpyrimidin-4(3H)-one ;
2-((1-benzylpipéridin-4-yl)méthyl)-4-phénéthylpyridazin-3(2H)-one;
chlorhydrate de 2-[(1-benzylpipéridin-4-yl)méthyl]-4-(pipéridin-1-yl)-2,3-dihydropyridazin-3-one ;
chlorhydrate de 2-((1-benzyl-4-hydroxypipéridin-4-yl)méthyl)-4-phénylpyridazin-3(2H)-one ;
chlorhydrate de 4-phényl-2-((1-((tétrahydro-2H-pyran-4-yl)méthyl)pipéridin-4-yl)méthyl)pyridazin-3(2H)-one ;
chlorhydrate de 2-(1-(1-benzylpipéridin-4-yl)éthyl)-4-phénylpyridazin-3(2H)-one ; et
chlorhydrate de 2-((1-(2-hydroxyéthyl)pipéridin-4-yl)méthyl)-4-phénylpyridazin-3(2H)-one.

10. Composé selon l'une quelconque des revendications 1 à 9, pour une utilisation en tant que médicament.

11. Composition pharmaceutique comprenant un composé selon l'une quelconque des revendications 1 à 9, et un support pharmaceutiquement acceptable.

12. Composé ou composition pharmaceutique selon l'une quelconque des revendications précédentes, pour une utilisation dans le traitement d'un trouble modulé par le récepteur sigma-1.

13. Composé ou composition pharmaceutique pour une utilisation selon la revendication 12, dans lequel ledit trouble est choisi dans l'ensemble constitué par les maladies neurodégénératives telles que la maladie d'Alzheimer, la maladie de Parkinson, la maladie de Huntington, la sclérose latérale amyotrophique, la sclérose en plaques ; les altérations cognitives et mémorielles telles qu'un vieillissement pathologique, une amnésie ischémique, des déficits cognitifs associés à une schizophrénie et une dépression ; des troubles cognitifs développementaux tels que des troubles associés à l'autisme et des troubles associés à un retard mental ; et les maladies génétiques associées à des dysfonctionnements de MAM.
